# EUROPEAN PATENT APPLICATION

(11) **EP 3 731 290 A1**
(43) Date of publication of application: **28.10.2020**
(21) Application number: 18890018.7
(22) Date of filing: 19.12.2018
(51) Int. Cl.: H01L 51/50, C07C 211/54, C07C 211/61, C09D 5/24, C09D 7/63

(54) **CHARGE-TRANSPORTING VARNISH**

(30) Priority: 20.12.2017 JP 2017243550
(71) Applicant: Nissan Chemical Corporation, Tokyo 103-6119 (JP)
(72) Inventor: ENDO, Toshiyuki, Funabashi-shi, Chiba 274-0052 (JP)
(74) Representative: Mewburn Ellis LLP
(86) International application number: PCT/JP2018/046693
(87) International publication number: WO 2019/124415

(57) **Abstract**

Provided is a charge-transporting varnish that contains: (A) an aryl sulfonate ester compound; (B) a tertiary aryl amine compound having at least one nitrogen atom, and for which the nitrogen atom has a tertiary aryl amine structure; and (C) an organic solvent.

## Description

### TECHNICAL FIELD

The invention relates to a charge-transporting varnish.

### BACKGROUND ART

Charge-transporting thin films made of organic compounds are used as light-emitting layers and charge-injecting layers in organic EL devices. In particular, a hole-injecting layer is responsible for transferring charge between an anode and a hole-transporting layer or a light-emitting layer, and thus carries out an important function for achieving low-voltage driving and high brightness in organic EL devices.

Methods for forming hole-injecting layers are classified broadly into dry processes typified by vapor deposition methods and wet processes typified by spin coating methods. Comparison of wet processes with dry processes shows that wet processes enable more efficient production of thin films with high flatness over a large area. Hence, as the areas of organic EL displays are being increased, hole-injecting layers that can be formed by wet processes are desired.

In view of such circumstances, the present inventor has developed charge-transporting materials which are applicable to various wet processes and which give thin films that enable achievement of excellent properties when applied to hole-injecting layers of organic EL elements, compounds having good solubility in organic solvents to be used for the charge-transporting materials, and charge-transporting varnishes (see, for example, Patent Documents 1 to 5). As charge-transporting materials soluble in organic solvents, particularly arylamine compounds have been widely researched and developed, and further, tertiary aryl amine compounds have been actively used as hole-injecting materials in dry processes (see, for example, Non-Patent Document 1), wet processes (see, for example, Patent Documents 5 and 6).

As a method for developing electrical conductivity of the tertiary aryl amine compound, a method is generally known in which the tertiary aryl amine compound is combined with a metal oxide or a cyano compound (see, for example, Patent Documents 5 and 6 and Non-Patent Document 1). When the tertiary aryl amine compound is cationized and made to coexist with an anionized sulfonic acid compound, the electrical conductivity can be exhibited (see, for example, Patent Document 6). On the other hand, it is not known that electrical conductivity is developed by combining a neutral tertiary aryl amine compound with a neutral sulfonic acid compound. This is mainly because a solvent species in which the neutral tertiary aryl amine compound is soluble (generally a non-polar solvent) is different from a solvent species in which the neutral sulfonic acid compound is soluble (polar solvent).

### PRIOR ART DOCUMENTS

### PATENT DOCUMENTS

Patent Document 1: WO 2008/032616
Patent Document 2: WO 2008/129947
Patent Document 3: WO 2006/025342
Patent Document 4: WO 2010/058777
Patent Document 5: WO 2015/050253
Patent Document 6: JP No. 5994213
Patent Document 7: JP No. 5136795

### NON-PATENT DOCUMENT

Non-Patent Literature 1: Japanese Journal of Applied Physics, Vol. 45, No. 12, pp. 9219-9223 (2006)

### SUMMARY OF INVENTION

### TECHNICAL PROBLEM

The present inventor has reported that by esterifying a sulfonic acid compound, the sulfonic acid compound is made soluble not only in a polar solvent but also in a non-polar solvent (Patent Document 7). This report has revealed that a secondary arylamine compound develops electrical conductivity when combined with a sulfonic acid ester compound. On the other hand, whether a tertiary aryl amine compound develops electrical conductivity with the aid of a sulfonic acid ester compound has been unknown.

The invention has been made in view of the above-described circumstances, and an object of the invention is to provide a charge-transporting varnish containing an aryl sulfonate ester compound and a tertiary aryl amine compound and which exhibits electrical conductivity when a thin film is formed.

### SOLUTION TO PROBLEM

The present inventor has conducted intensive studies for achieving the object, and resultantly found that a thin film obtained using a charge-transporting varnish containing an aryl sulfonate ester compound and a tertiary aryl amine compound having at least one nitrogen atom with all the nitrogen atoms forming a tertiary aryl amine structure develops electrical conductivity. Consequently, the present inventor has completed the invention.

Accordingly, the invention provides the following charge-transporting varnish.
1. A charge-transporting varnish comprising: (A) an aryl sulfonate ester compound; (B) a tertiary aryl amine compound having at least one nitrogen atom with all the nitrogen atoms forming a tertiary aryl amine structure; and (C) an organic solvent.
2. The charge-transporting varnish of 1, wherein the aryl sulfonate ester compound is a fluorine atom-containing aryl sulfonate ester compound.
3. The charge-transporting varnish of 1, wherein the aryl sulfonate ester compound is a compound of the following formula (1) or (1'): 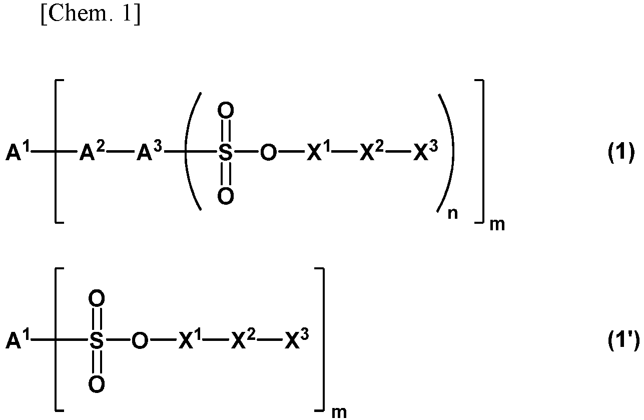 wherein A¹ is an m-valent hydrocarbon group of 6 to 20 carbon atoms which optionally has a substituent and which contains one or more aromatic rings, or an m-valent group derived from the following formula (2) or (3): 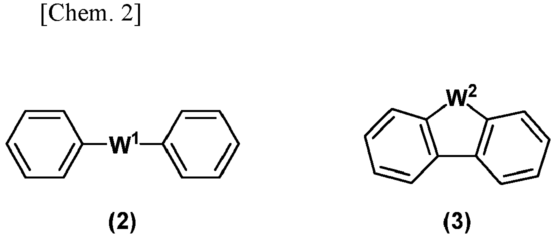
   wherein W¹ and W² are each independently -O-, -S-, -S (O)- or -S(O₂)-, or -N-, Si-, -P- or -P (O)- which optionally has a substituent;
   A² is -O-, -S- or -NH-;
   A³ is an (n+1)-valent aromatic group of 6 to 20 carbon atoms;
   X¹ is an alkylene group of 2 to 5 carbon atoms, the alkylene group optionally having -O-, -S- or a carbonyl group interposed between carbon atoms, the alkylene group being optionally substituted with alkyl groups of 1 to 20 carbon atoms at some or all of hydrogen atoms;
   X² is a single bond, -O-, -S- or -NR-, where R is a hydrogen atom or a monovalent hydrocarbon group of 1 to 10 carbon atoms;
   X³ is a monovalent hydrocarbon group of 1 to 20 carbon atoms which optionally has a substituent;
   m is an integer that satisfies the condition 1 ≤ m ≤ 4; and
   n is an integer that satisfies the condition 1 ≤ n ≤ 4.
4. The charge-transporting varnish of 3, wherein A¹ is an m-valent hydrocarbon group of 6 to 20 carbon atoms which is substituted with a fluorine atom and contains one or more aromatic rings, or an m-valent group derived from a compound of formula (2) or (3).
5. The charge-transporting varnish of any one of 1 to 4, wherein the aryl sulfonate ester compound is a compound of any of the following formulae (1-1) to (1-3): 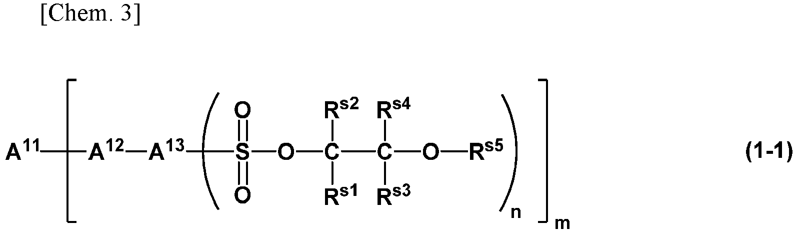
   wherein R^{s1} to R^{s4} are each independently a hydrogen atom or a linear or branched alkyl group of 1 to 6 carbon atoms, and R^{s5} is a monovalent hydrocarbon group of 2 to 20 carbon atoms which optionally has a substituent;
   A¹¹ is an m-valent group derived from perfluorobiphenyl, A¹² is -O- or -S-, and A¹³ is an (n+1)-valent group derived from naphthalene or anthracene; and
   m and n are the same as described above; 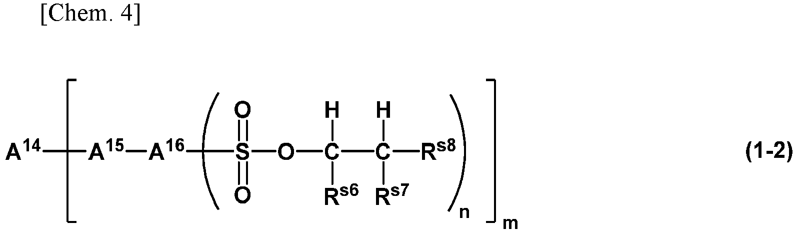
   wherein R^{s6} to R^{s7} are each independently a hydrogen atom or a linear or branched monovalent aliphatic hydrocarbon group, and R^{s8} is a linear of branched monovalent aliphatic hydrocarbon group, provided that the total number of carbon atoms of R^{s6}, R^{s7} and R^{s8} is 6 or more;
   A¹⁴ is an m-valent hydrocarbon group which optionally has a substituent and which contains one or more aromatic rings, A¹⁵ is -O- or -S-, and A¹⁶ is an (n+1)-valent aromatic group; and
   m and n are the same as described above; and 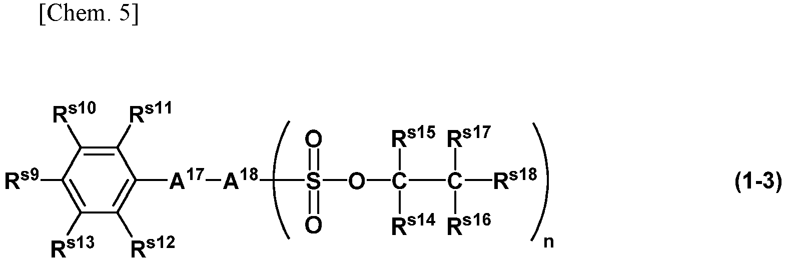
   wherein R^{s9} to R^{s13} are each independently a hydrogen atom, a nitro group, a cyano group, a halogen atom, an alkyl group of 1 to 10 carbon atoms, a halogenated alkyl group of 1 to 10 carbon atoms, or a halogenated alkenyl group of 2 to 10 carbon atoms;
   R^{s14} to R^{s17} are each independently a hydrogen atom, or a linear or branched monovalent aliphatic hydrocarbon group of 1 to 20 carbon atoms;
   R^{s18} is a linear or branched monovalent aliphatic hydrocarbon group of 1 to 20 carbon atoms, or -OR^{s19}, where R^{s19} is an optionally substituted monovalent hydrocarbon group of 2 to 20 carbon atoms;
   A¹⁷ is -O-, -S- or -NH-;
   A¹⁸ is an (n+1)-valent aromatic group; and
   n is the same as described above.
6. The charge-transporting varnish of any one of 1 to 5, wherein the tertiary aryl amine compound has at least two nitrogen atoms, with all the nitrogen atoms forming a tertiary aryl amine structure.
7. The charge-transporting varnish of any one of 1 to 6, wherein the organic solvent is a low-polarity organic solvent.
8. A charge-transporting thin film obtained using the charge-transporting varnish of any one of 1 to 7.
9. An organic EL device comprising the charge-transporting thin film of 8.

### ADVANTAGEOUS EFFECTS OF INVENTION

The sulfonic acid ester compound in the charge-transporting varnish of the invention enables development of electrical conductivity by acting on a wide range of tertiary aryl amine compounds including low-molecular-weight and high-molecular-weight tertiary aryl amine compounds. In the varnish, both the sulfonic acid compound and the conductive material are neutral, and the varnish has an ink electrical conductivity of 0 unlike varnishes containing anions or cations. Accordingly, it is possible to provide a hole-injecting varnish which enables achievement of excellent device properties when applied to an organic EL device and which is excellent in stability in air.

Also, because thin films obtained from the charge-transporting varnish of the invention have a high charge transportability, when such a film is used as a hole-injecting layer or a hole-transporting layer, the driving voltage of the organic EL device can be lowered. By taking advantage of the high flatness and high charge transportability of these thin films, it is also possible to employ the thin films as hole-transporting layers in solar cells, as fuel cell electrodes, as protective films for capacitor electrodes, and as antistatic films.

### DESCRIPTION OF EMBODIMENTS

### [Charge-Transporting Varnish]

A charge-transporting varnish of the invention includes (A) an aryl sulfonate ester compound; (B) a tertiary aryl amine compound having at least one nitrogen atom with all the nitrogen atoms forming a tertiary aryl amine structure; and (C) an organic solvent. In the invention, "charge-transportability" is synonymous with electrical conductivity, and also synonymous with hole-transportability. Also, "charge-transporting varnish" of the invention may refer to a varnish which itself has charge transportability or to a varnish which imparts charge-transportability to a solid film obtained using the varnish.

### [(A) Aryl Sulfonate Ester Compound]

The aryl sulfonate ester compound as component (A) functions as an electron-accepting substance precursor. In the invention, the electron-accepting substance is used for improving electron transporting ability and uniformity of film formation. The aryl sulfonate ester compound is not particularly limited, provided that a sulfonic acid ester group is bonded to an aromatic ring.

In a preferred embodiment of the invention, the molecular weight of the aryl sulfonate ester compound is preferably 100 or more, more preferably 200 or more, and preferably 5,000 or less, more preferably 4,000 or less, even more preferably 3,000 or less, still more preferably 2,000 or less. In a preferred embodiment of the invention, the number of sulfonic acid ester groups of the aryl sulfonate ester compound is preferably 2 or more, more preferably 3 or more, and preferably 6 or less, more preferably 5 or less. In a preferred embodiment of the invention, the aryl sulfonate ester compound contains an aromatic ring preferably substituted with fluorine.

The aryl sulfonate ester compound is preferably a compound of the following formula (1) or (1').

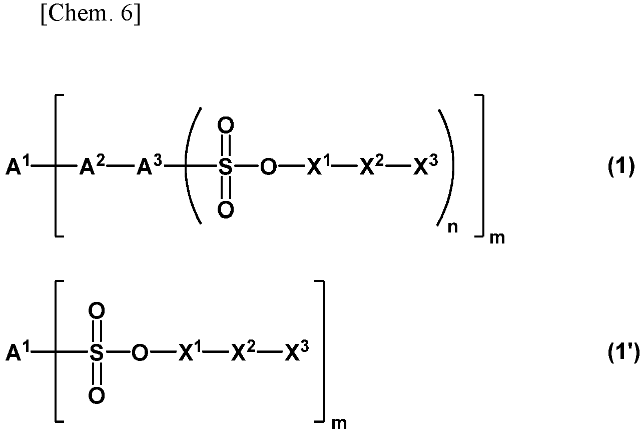

In formulae (1) and (1'), A¹ is an m-valent hydrocarbon group of 6 to 20 carbon atoms which optionally has a substituent and which contains one or more aromatic rings, or an m-valent group derived from a compound of formula (2) or (3) (i.e. a group obtained by removing m hydrogen atoms on an aromatic ring of a compound of the following formula (2) or (3)). 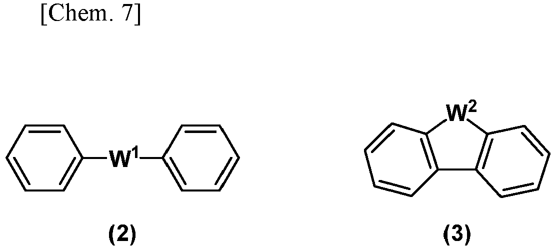 Herein, W¹ and W² are each independently -O-, -S-, -S(O)-, or -S(O₂)-, or -N-, -Si-, -P- or -P(O)- which optionally has a substituent.

The m-valent hydrocarbon group of 6 to 20 carbon atoms which containing one or more aromatic rings is a group obtained by removing m hydrogen atoms from a hydrocarbon of 6 to 20 carbon atoms which contains one or more aromatic rings. Examples of the hydrocarbon containing one or more aromatic rings include benzene, toluene, xylene, biphenyl, naphthalene, anthracene and pyrene. Of these, groups derived from benzene, biphenyl and the like are preferred as the hydrocarbon group.

The hydrocarbon group is optionally substituted with substituents at some or all of hydrogen atoms thereof. Examples of the substituent include a fluorine atom, a chlorine atom, a bromine atom, an iodine atom, a nitro group, a cyano group, a hydroxy group, an amino group, a silanol group, a thiol group, a carboxy group, a sulfonic acid ester group, a phosphoric acid group, and a phosphoric acid ester group, an ester group, a thioester group, an amide group, a monovalent hydrocarbon group, an organooxy group, an organoamino group, an organosilyl group, an organothio group, an acyl group and a sulfo group.

Here, examples of the monovalent aliphatic hydrocarbon group include alkyl groups of 1 to 10 carbon atoms such as a methyl group, an ethyl group, an n-propyl group, an isopropyl group, an n-butyl group, an isobutyl group, a sec-butyl group, a tert-butyl group, an n-pentyl group, a cyclopentyl group, an n-hexyl group, a cyclohexyl group, an n-heptyl group, an n-octyl group, an n-nonyl group and an n-decyl group; alkenyl groups of 2 to 10 carbon atoms such as a vinyl group, a 1-propenyl group, a 2-propenyl group, an isopropenyl group, a 1-methyl-2-propenyl group, a 1-butenyl group, a 2-butenyl group, a 3-butenyl group and a hexenyl group; aryl groups of 6 to 20 carbon atoms such as a phenyl group, a xylyl group, a tolyl group, a 1-naphthyl group and a 2-naphthyl group; and aralkyl groups of 7 to 20 carbon atoms such as a benzyl group and a phenylethyl group.

Examples of the organooxy group include alkoxy groups, alkenyloxy groups and aryloxy groups. Examples of the alkyl group, alkenyl group and aryl group contained therein include the same as those mentioned above.

Examples of the organoamino group include alkylamino groups of 1 to 12 carbon atoms such as a methylamino group, an ethylamino group, a propylamino group, a butylamino group, a pentylamino group, a hexylamino group, a cyclohexylamino group, a heptylamino group, an octylamino group, a nonylamino group, a decylamino group and a dodecylamino; dialkylamino groups in which each alkyl group is an alkyl group of 1 to 12 carbon atoms, such as a dimethylamino group, a diethylamino group, a dipropylamino group, a dibutylamino group, a dipentylamino group, a dihexylamino group, a dicyclohexylamino group, a diheptylamino group, a dioctylamino group, a dinonylamino group and a didecylamino group; and a morpholino group.

Examples of the organosilyl group include trialkylsilyl groups in which each alkyl group is an alkyl group of 1 to 10 carbon atoms, such as a trimethylsilyl group, a triethylsilyl group, a tripropylsilyl group, a tributylsilyl group, a tripentylsilyl group, a trihexylsilyl group, a pentyldimethylsilyl group, a hexyldimethylsilyl group, an octyldimethylsilyl group, and a decyldimethyl group.

Examples of the organothio group include alkylthio groups of 1 to 12 carbon atoms such as a methylthio group, an ethylthio group, a propylthio group, a butylthio group, a pentylthio group, a hexylthio group, a heptylthio group, an octylthio group, a nonylthio group, a decylthio group, and a dodecylthio group.

Examples of the acyl group include acyl groups of 1 to 10 carbon atoms such as a formyl group, an acetyl group, a propionyl group, a butyryl group, an isobutyryl group, a valeryl group, an isovaleryl group and a benzoyl group.

The number of carbon atoms of each of the monovalent hydrocarbon group, the organooxy group, the organoamino group, the organoamino group, the organosilyl group, the organothio group and the acyl group is preferably 1 to 8.

Of these substituents, a fluorine atom, a sulfonic acid group, an alkyl group, an organooxy group, and an organosilyl group are more preferred.

In formula (1), A² is -O-, -S- or -NH-. Of these, -O- is preferred because of easy synthesis.

In formula (1), A³ is an (n+1)-valent aromatic group of 6 to 20 carbon atoms. The aromatic group is a group obtained by removing n+1 hydrogen atoms from an aromatic ring of an aromatic compound of 6 to 20 carbon atoms. In the invention, the aromatic compound means an aromatic hydrocarbon and an aromatic heterocyclic compound. Examples of the aromatic compound include benzene, toluene, xylene, biphenyl, naphthalene, anthracene and pyrene. Of these, groups derived from naphthalene or anthracene are preferred as the aromatic group represented by A³.

In formulae (1) and (1'), X¹ is an alkylene group of 2 to 5 carbon atoms, the alkylene group optionally having -O-, -S- or a carbonyl group interposed between carbon atoms, the alkylene group being optionally substituted with alkyl groups of 1 to 20 carbon atoms at some or all of hydrogen atoms. X¹ is preferably an ethylene group, a trimethylene group, a methyleneoxymethylene group, a methylenethiomethylene group or the like, and such a group is optionally substituted with an alkyl group of 1 to 20 carbon atoms at some or all thereof. Examples of the alkyl group include a methyl group, an ethyl group, an n-propyl group, an isopropyl group, an n-butyl group, an isobutyl group, a sec-butyl group, a tert-butyl group, an n-pentyl group, a cyclopentyl group, an n-hexyl group, a cyclohexyl group, an n-heptyl group, an n-octyl group, an n-nonyl group, an n-decyl group, an n-undecyl group, an n-dodecyl group and a bicyclohexyl group.

In formulae (1) and (1'), X² is a single bond, -O-, -S-, or -NR-. R is a hydrogen atom or a monovalent hydrocarbon group of 1 to 10 carbon atoms. The monovalent hydrocarbon group is preferably an alkyl group such as a methyl group, an ethyl group or an n-propyl group. X² is preferably a single bond, -O- or -S-, more preferably a single bond or -O-.

In formulae (1) and (1'), X³ is an optionally substituted monovalent hydrocarbon group of 1 to 20 carbon atoms. Examples of the monovalent aliphatic hydrocarbon group include alkyl groups of 1 to 20 carbon atoms such as a methyl group, an ethyl group, an n-propyl group, an isopropyl group, an n-butyl group, an isobutyl group, a sec-butyl group, a tert-butyl group, an n-pentyl group, a cyclopentyl group, an n-hexyl group, a cyclohexyl group, an n-heptyl group, an n-octyl group, an n-nonyl group, an n-decyl group an n-undecyl group, an n-dodecyl group and a bicyclohexyl group; alkenyl groups of 2 to 20 carbon atoms such as a vinyl group, a 1-propenyl group, a 2-propenyl group, an isopropenyl group, a 1-methyl-2-propenyl group, a 1-butenyl group, a 2-butenyl group, a 3-butenyl group and a hexenyl group; aryl groups of 6 to 20 carbon atoms such as a phenyl group, a xylyl group, a tolyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 2-biphenyl group, a 3-biphenyl group and a 4-biphenyl group; and aralkyl groups of 7 to 20 carbon atoms such as a benzyl group, a phenylethyl group and a phenylcyclohexyl group. The monovalent hydrocarbon group is optionally substituted with substituents at some or all of hydrogen atoms thereof. Examples of the substituent include the same as those mentioned above for A¹. X³ is preferably an alkyl group of 1 to 20 carbon atoms or an aryl group of 6 to 20 carbon atoms.

In formulae (1) and (1'), m is an integer which satisfies the condition 1 ≤ m ≤ 4, and m is preferably 2. n is an integer which satisfies the condition 1 ≤ n ≤ 4, and n is preferably 2.

Because the aryl sulfonate ester compound of formula (1) or (1') exhibits a high solubility in a broad range of solvents including low-polarity solvents, the physical properties of the solution can be adjusted using a variety of solvents, and the solution has a high coatability. Therefore, it is preferable for application to be carried out while the solution is in the state of a sulfonic acid ester, and for sulfonic acid to be generated when the applied film is dried or fired. Because it is desirable for the sulfonic acid ester to be stable at room temperature and at or below the firing temperature, the temperature at which sulfonic acid is generated from the sulfonic acid ester is typically from 40 to 260°C. Taking into account the high stability of the sulfonic acid ester within the varnish and the ease of dissociation during firing, the temperature is preferably from 80 to 230°C, and more preferably from 120 to 180°C.

The aryl sulfonate ester compound of formula (1) is preferably a compound of the following formula (1-1) or (1-3). 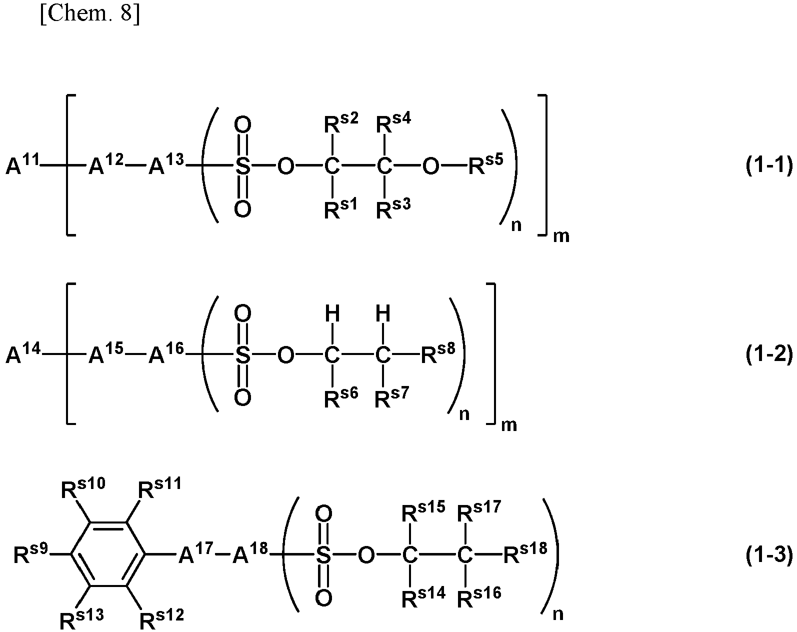 Herein, m and n are the same as described above.

In formula (1-1), A¹¹ is an m-valent group derived from perfluorobiphenyl (i.e. a group obtained by removing m fluorine atoms from perfluorobiphenyl). A¹² is -O- or -S-, preferably -O-. A¹³ is an (n+1)-valent group derived from naphthalene or anthracene, preferably a group derived from naphthalene.

In the formula (1-1), R^{s1} to R^{s4} are each independently a hydrogen atom or a linear or branched alkyl group of 1 to 6 carbon atoms, and R^{s5} is an optionally substituted monovalent hydrocarbon group of 2 to 20 carbon atoms.

Examples of the linear or branched alkyl group include, but are not particularly limited to, a methyl group, an ethyl group, an n-propyl group, an isopropyl group, an n-butyl group, an isobutyl group, a tert-butyl group and an n-hexyl group. Of these, alkyl groups of 1 to 3 carbon atoms is preferred.

Examples of the monovalent hydrocarbon group of 2 to 20 carbon atoms include alkyl groups such as an ethyl group, an n-propyl group, an isopropyl group, an n-butyl group, an isobutyl group and a tert-butyl group, and aryl groups such as a phenyl group, a naphthyl group and a phenanthryl.

It is preferable that among R^{s1} to R^{s4}, R^{s1} or R^{s3} is preferably a linear alkyl group of 1 to 3 carbon atoms, and the remainder is a hydrogen atom. Further, it is preferable that R^{s1} is a linear alkyl group of 1 to 3 carbon atoms, and R^{s2} to R^{s4} are hydrogen atoms. The linear alkyl group of 1 to 3 carbon atoms is preferably a methyl group. R^{s5} is preferably a linear alkyl group of 2 to 4 carbon atoms or a phenyl group.

In formula (1-2), A¹⁴ is an optionally substituted m-valent hydrocarbon group of 6 to 20 carbon atoms which contains one or more aromatic rings. The hydrocarbon group is a group obtained by removing m hydrogen atoms from a hydrocarbon of 6 to 20 carbon atoms which contains one or more aromatic rings. Examples of the hydrocarbon include benzene, toluene, xylene, ethylbenzene, biphenyl, naphthalene, anthracene and phenanthrene. The hydrocarbon group is optionally substituted with substituents at some or all of hydrogen atoms thereof. Examples of the substituents include the same as those mentioned above for A¹. Preferred examples of A¹⁴ include the same as those mentioned above as preferred examples of A¹.

In formula (1-2), A¹⁵ is -O- or -S-, preferably -O-.

In formula (1-2), A¹⁶ is an (n+1)-valent aromatic group of 6 to 20 carbon atoms. The aromatic group is a group obtained by removing n+1 hydrogen atoms from an aromatic ring of an aromatic compound of 6 to 20 carbon atoms. Examples of the aromatic compound include benzene, toluene, xylene, biphenyl, naphthalene, anthracene and pyrene. Of these, A¹⁶ is preferably a group derived from naphthalene or anthracene, more preferably a group derived from naphthalene.

In formula (1-2), R^{s6} to R^{s7} are each independently a hydrogen atom, or a linear or branched monovalent aliphatic hydrocarbon group. R^{s8} is a linear or branched monovalent aliphatic hydrocarbon group. However, the total number of carbon atoms of R^{s6}, R^{s7} and R^{s8} is 6 or more. The upper limit of the total number of carbon atoms of R^{s6}, R^{s7} and R^{s8} is not particularly limited, but is preferably 20 or less, more preferably 10 or less.

Examples of the monovalent aliphatic hydrocarbon group include, but are not particularly limited to, alkyl groups of 1 to 20 carbon atoms such as a methyl group, an ethyl group, an n-propyl group, an isopropyl group, an n-butyl group, an isobutyl group, a tert-butyl group, an n-hexyl group, an n-octyl group, a 2-ethylhexyl group and an n-decyl group; and alkenyl groups of 2 to 20 carbon atoms such as a vinyl group, a 1-propenyl group, a 2-propenyl group, an isopropenyl group, a 1-methyl-2-propenyl group, a 1-butenyl group, a 2-butenyl group, a 3-butenyl group and a hexenyl group.

R^{s6} is preferably a hydrogen atom, and R^{s7} and R^{s8} are each preferably an alkyl group of 1 to 6 carbon atoms. Here, R^{s7} and R^{s8} may be identical to or different from each other.

In formula (1-2), m is an integer which satisfies the condition 1 ≤ m ≤ 4, and m is preferably 2. n is an integer which satisfies the condition 1 ≤ n ≤ 4, and n is preferably 2.

In formula (1-3), R^{s9} to R^{s13} are each independently a hydrogen atom, a nitro group, a cyano group, a halogen atom, an alkyl group of 1 to 10 carbon atoms, a halogenated alkyl group of 1 to 10 carbon atoms, or a halogenated alkenyl group of 2 to 10 carbon atoms.

The alkyl group of 1 to 10 carbon atoms may be linear, branched or cyclic, and specific examples thereof include a methyl group, an ethyl group, an n-propyl group, an isopropyl group, an n-butyl group, an isobutyl group, a sec-butyl group, a tert-butyl group, an n-pentyl group, a cyclopentyl group, an n-hexyl group, a cyclohexyl group, an n-heptyl group, an n-octyl group, an n-nonyl group, and an n-decyl group.

The alkyl halide group of 1 to 10 carbon atoms is not particularly limited as long as some or all of the hydrogen atoms of the alkyl group of 1 to 10 carbon atoms are substituted with halogen atoms. The alkyl halide group may be linear, branched or cyclic, and specific examples thereof include a trifluoromethyl group, a 2,2,2-trifluoroethyl group, a 1,1,2,2,2-pentafluoroethyl group, a 3,3,3-trifluoropropyl group, a 2,2,3,3,3-pentafluoropropyl group, a 1,1,2,2,3,3,3-heptafluoropropyl group, a 4,4,4-trifluorobutyl group, a 3,3,4,4,4-pentafluorobutyl group, a 2,2,3,3,4,4,4-heptafluorobutyl group and a 1,1,2,2,3,3,4,4,4-nonafluorobutyl group.

The alkenyl halide group of 2 to 10 carbon atoms is not particularly limited as long as some or all of the hydrogen atoms of the alkenyl group of 2 to 10 carbon atoms are substituted with halogen atoms. Specific examples thereof include a perfluorovinyl group, a perfluoro-1-propenyl group, a perfluoro-2-propenyl group, a perfluoro-1-butenyl group, a perfluoro-2-butenyl group and a perfluoro-3-butenyl group.

Of these, R^{s9} is preferably a nitro group, a cyano group, a halogenated alkyl group of 1 to 10 carbon atoms, a halogenated alkenyl group of 2 to 10 carbon atoms or the like, more preferably a nitro group, a cyano group, a halogenated alkyl group of 1 to 4 carbon atoms, a halogenated alkenyl group of 2 to 4 carbon atoms, or the like, still more a nitro group, a cyano group, a trifluoromethyl group, a perfluoropropenyl group or the like. R^{s10} to R^{s13} are each preferably a halogen atom, more preferably a fluorine atom.

In the formula (1-3), A¹⁷ is -O-, -S- or -NH-, preferably -O-.

In formula (1-3), A¹⁸ is an (n+1)-valent aromatic group of 6 to 20 carbon atoms. The aromatic group is a group obtained by removing n+1 hydrogen atoms from an aromatic ring of an aromatic compound of 6 to 20 carbon atoms. Examples of the aromatic compound include benzene, toluene, xylene, biphenyl, naphthalene, anthracene and pyrene. Of these, A¹⁸ is preferably a group derived from naphthalene or anthracene, more preferably a group derived from naphthalene.

In the formula (1-3), R^{s14} to R^{s17} are each independently a hydrogen atom or a linear or branched monovalent aliphatic hydrocarbon group of 1 to 20 carbon atoms.

Examples of the monovalent aliphatic hydrocarbon group include alkyl groups of 1 to 20 carbon atoms such as a methyl group, an ethyl group, an n-propyl group, an isopropyl group, an n-butyl group, an isobutyl group, a sec-butyl group, a tert-butyl group, an n-pentyl group, a cyclohexyl group, an n-hexyl group, a cyclohexyl group, an n-heptyl group, an n-octyl group, an n-nonyl group, an n-decyl group, an n-undecyl group, and an n-dodecyl group; and alkenyl groups of 2 to 20 carbon atoms such as a vinyl group, a 1-propenyl group, a 2-propenyl group, an isopropenyl group, a 1-methyl-2-propenyl group, a 1-butenyl group, a 2-butenyl group, a 3-butenyl group and a hexenyl group. Of these, alkyl groups of 1 to 20 carbon atoms are preferred, alkyl groups of 1 to 10 carbon atoms are more preferred, and alkyl groups of 1 to 8 carbon atoms are still more preferred.

In formula (1-3), R^{s18} is a linear or branched monovalent aliphatic hydrocarbon group of 1 to 20 carbon atoms, or -OR^{s19}. R^{s19} is an optionally substituted monovalent hydrocarbon group of 2 to 20 carbon atoms.

Examples of the linear or branched monovalent aliphatic hydrocarbon group of 1 to 20 carbon atoms, which is represented by R^{s18}, include the same as those mentioned above. When R^{s18} is a monovalent aliphatic hydrocarbon group, R^{s18} is preferably an alkyl group of 1 to 20 carbon atoms, more preferably an alkyl group of 1 to 10 carbon atoms, still more preferably an alkyl group of 1 to 8 carbon atoms.

Examples of the monovalent hydrocarbon group of 2 to 20 carbon atoms, which is represented by R^{s19}, include the above-mentioned monovalent aliphatic hydrocarbon groups except for a methyl group, and aryl groups such as a phenyl group, a naphthyl group and a phenanthryl group. Of these, R^{s19} is preferably a linear alkyl group of 2 to 4 carbon atoms or a phenyl group. Examples of the substituent optionally present in the monovalent hydrocarbon group include a fluoro group, an alkoxy group of 1 to 4 carbon atoms, a nitro group and a cyano group.

In formula (1-3), n is an integer which satisfies the condition 1 ≤ n ≤ 4, and n is preferably 2.

The aryl sulfonate ester compound of formula (1-3) is particularly preferably a compound of the following formula (1-3-1) or (1-3-2).

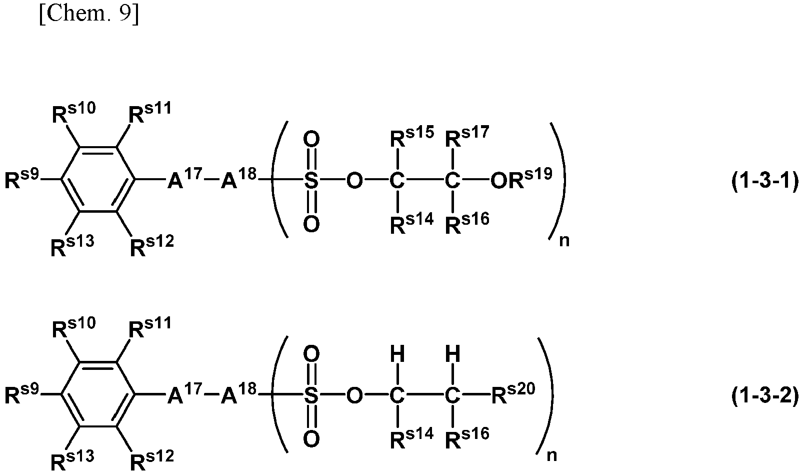

In the formula, A¹⁷, A¹⁸, R^{s9} to R^{s17}, R^{s19} and n are the same as described above. R^{s20} is a linear or branched monovalent aliphatic hydrocarbon group of 1 to 20 carbon atoms, and specific examples thereof include the same as those mentioned above for R^{s18}.

In the aryl sulfonate ester compound of formula (1-3-1), it is preferable that among R^{s14} to R^{s17}, R^{s14} or R^{s16} is a linear alkyl group of 1 to 3 carbon atoms, and the remainder is a hydrogen atom. Further, it is preferable that R^{s14} is a linear alkyl group of 1 to 3 carbon atoms, and R^{s15} to R^{s17} are hydrogen atoms. The linear alkyl group of 1 to 3 carbon atoms is preferably a methyl group. R^{s19} is preferably a linear alkyl group of 2 to 4 carbon atoms or a phenyl group.

In the aryl sulfonate ester compound of formula (1-3-2), the total number of carbon atoms of R^{s14}, R^{s16} and R^{s20} is preferably 6 or more. The upper limit of the total number of carbon atoms of R^{s14}, R^{s16} and R^{s20} is preferably 20 or less, and more preferably 10 or less. Here, R^{s14} is preferably a hydrogen atom, and R^{s16} to R^{s20} are each preferably an alkyl group of 1 to 6 carbon atoms. R^{s16} and R^{s20} may be identical to or different from each other.

The aryl sulfonate ester compounds of formula (1) may be used singly, or in combination of two or more thereof.

The aryl sulfonate ester compound of formula (1) can be synthesized by, for example, as shown in Scheme A below, reacting a sulfonic acid salt compound of formula (1A) with a halogenating agent so as to synthesize a sulfonyl halide compound of formula (1B) below (referred to below as "Step 1"), and then reacting this sulfonyl halide compound with a compound of formula (1C) (referred to below as "Step 2"). 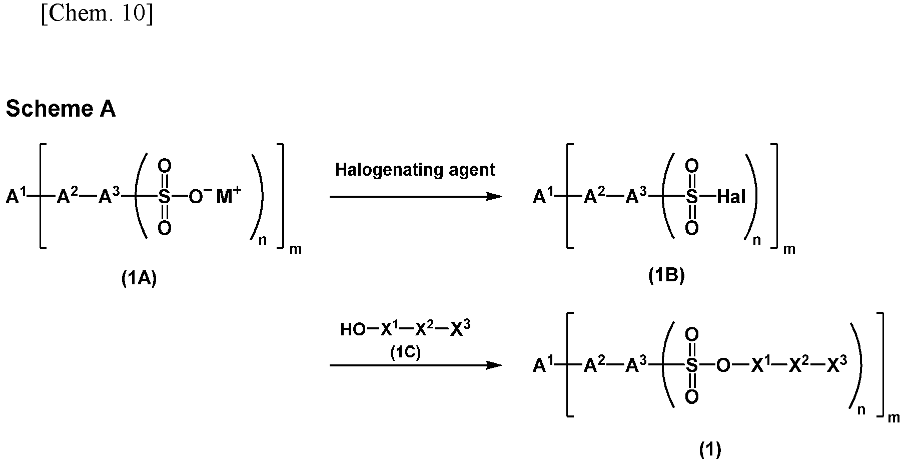 Herein, A¹ to A³, X¹ to X³, m and n are the same as described above; M⁺ is a monovalent cation such as a sodium ion, a potassium ion, a pyridinium ion or a quaternary ammonium ion; and Hal is a halogen atom such as a chlorine atom and a bromine atom.

The sulfonic acid salt compound of formula (1A) can be synthesized by a known method.

Examples of the halogenating agent used in Step 1 include thionyl chloride, oxalyl chloride, phosphorus oxychloride and phosphorus(V) chloride; thionyl chloride is preferred. The amount of halogenating agent used is not limited, so long as it is at least one mole per mole of the sulfonic acid salt compound, although use in an amount that, expressed as a weight ratio, is from 2 to 10 times the amount of the sulfonic acid salt compound is preferred.

The reaction solvent used in Step 1 is preferably a solvent that does not react with the halogenating agent, examples of which include chloroform, dichloroethane, carbon tetrachloride, hexane and heptane. The reaction can be carried out without a solvent, and here, the halogenating agent is preferably used in at least the amount at which the system becomes a uniform solution at the time of reaction completion. Further, a catalyst such as N,N-dimethylformamide may be used for accelerating the reaction. The reaction temperature may be set to from about 0 to about 150°C, although the reaction temperature is preferably from 20 to 100°C and at or below the boiling point of the halogenating agent used. Following reaction completion, the crude product obtained by vacuum concentration or the like is generally used in the next step.

Examples of the compounds of formula (1C) include glycol ethers such as propylene glycol monoethyl ether, propylene glycol monopropyl ether, propylene glycol monobutyl ether, propylene glycol monophenyl ether, ethylene glycol monobutyl ether and ethylene glycol monohexyl ether; and alcohols such as 2-ethyl-1-hexanol, 2-butyl-1-octanol, 1-octanol and 3-nonanol.

In Step 2, a base may be concomitantly used. Examples of bases that may be used include sodium hydride, pyridine, triethylamine and diisopropylethylamine. Sodium hydride, pyridine and triethylamine are preferred. The base is preferably used in an amount that ranges from one mole per mole of the sulfonyl halide compound up to the amount of solvent.

Various organic solvents may be used as the reaction solvent in Step 2, although tetrahydrofuran, dichloroethane, chloroform and pyridine are preferred. The reaction temperature, although not particularly limited, is preferably from 0 to 80°C. Following reaction completion, pure aryl sulfonate ester compound can be obtained by work-up and purification using customary methods such as vacuum concentration, liquid/liquid extraction, water rinsing, reprecipitation, recrystallization and chromatography. The pure aryl sulfonate ester compound thus obtained can be rendered into a high-purity sulfonic acid compound by being subjected to heat treatment or the like.

Alternatively, as shown in Scheme B below, the aryl sulfonate ester compound of formula (1) can be synthesized from a sulfonic acid compound of formula (1D). In the Scheme B below, the halogenating agent, compound of formula (1C), reaction solvent and other ingredients used in the first-stage and second-stage reactions may be the same as those used in Steps 1 and 2 of Reaction Scheme A. 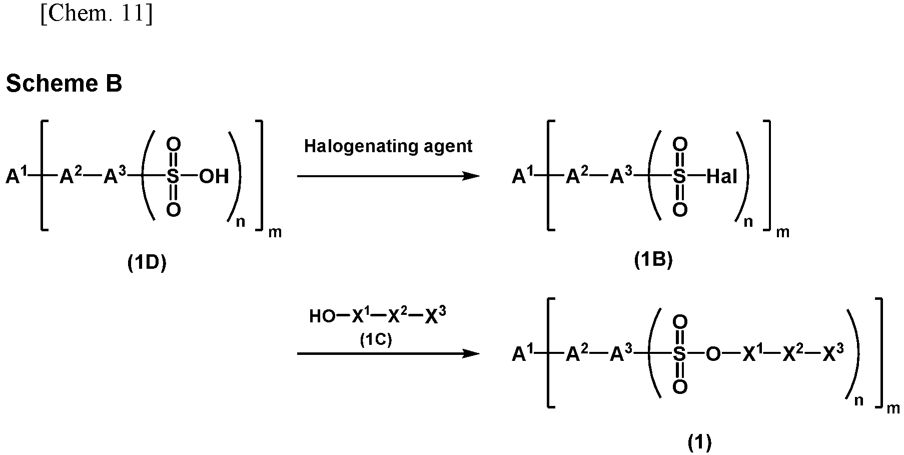 Herein, A¹ to A³, X¹ to X³, Hal, m and n are the same as described above.

The sulfonic acid compound of formula (1D) can be synthesized by a known method.

The aryl sulfonate ester compound of formula (1') can be synthesized according to a conventionally known method, for example, the method described in JP No. 5136795.

### [(B) Tertiary Aryl Amine Compound]

The tertiary aryl amine compound as component (B) has at least one nitrogen atom, with all the nitrogen atoms forming a tertiary aryl amine structure. In other words, the tertiary aryl amine compound has a structure in which at least one nitrogen atom is present and three aromatic groups are bonded to all the nitrogen atoms. It is preferable that the tertiary aryl amine compound two or more nitrogen atoms. The tertiary aryl amine compound as component (B) functions as a charge-transporting substance.

Preferred examples of the tertiary aryl amine compound include compounds of the following formula (A1) or (A2).

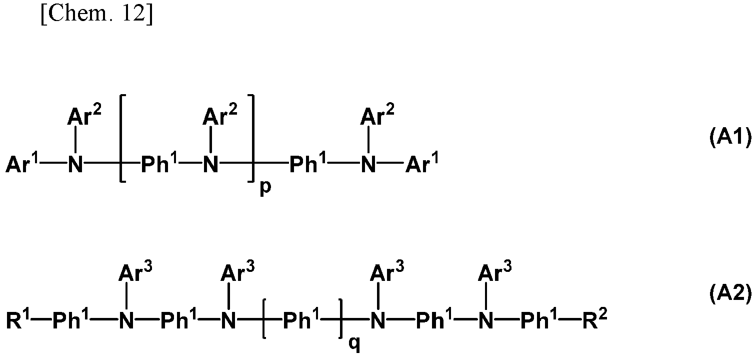

In the formula (A2), R¹ and R² are each independently a hydrogen atom, a halogen atom, a nitro group, or a cyano group, or an alkyl group of 1 to 20 carbon atoms, alkenyl group of 2 to 20 carbon atoms, alkynyl group of 2 to 20 carbon atoms, aryl group of 6 to 20 carbon atoms or heteroaryl group of 2 to 20 carbon atoms which is optionally substituted with a halogen atom.

Examples of the halogen atom include fluorine, chlorine, bromine and iodine atoms.

The alkyl group of 1 to 20 carbon atoms may be linear, branched or cyclic. Specific examples thereof include linear or branched alkyl groups of 1 to 20 carbon atoms such as methyl, ethyl, n-propyl, isopropyl, n-butyl, isobutyl, sec-butyl, tert-butyl, n-pentyl, n-hexyl, n-heptyl, n-octyl, n-nonyl and n-decyl groups; and cyclic alkyl groups of 3 to 20 carbon atoms such as cyclopropyl, cyclobutyl, cyclopentyl, cyclohexyl, cycloheptyl, cyclooctyl, cyclononyl, cyclodecyl, bicyclobutyl, bicyclopentyl, bicyclohexyl, bicycloheptyl, bicyclooctyl, bicyclononyl and bicyclodecyl groups.

The alkenyl group of 2 to 20 carbon atoms may be linear, branched or cyclic. Specific examples thereof include vinyl, n-1-propenyl, n-2-propenyl, 1-methylvinyl, n-1-butenyl, n-2-butenyl, n-3-butenyl, 2-methyl-1-propenyl, 2-methyl-2-propenyl, 1-ethylvinyl, 1 methyl-1-propenyl, 1-methyl-2-propenyl, n-1-pentenyl, n-1-decenyl and n-1-eicosenyl groups.

The alkynyl group of 2 to 20 carbon atoms may be linear, branched or cyclic. Specific examples thereof include ethynyl, n-1-propynyl, n-2-propynyl, n-1-butynyl, n-2-butynyl, n-3-butynyl, 1-methyl-2-propynyl, n-1-pentynyl, n-2-pentynyl, n-3-pentynyl, n-4-pentynyl, 1-methyl-n-butynyl, 2-methyl-n-butynyl, 3-methyl-n-butynyl, 1,1-dimethyl-n-propynyl, n-1-hexynyl, n-1-decynyl, n-1-pentadecynyl and n-1-eicosynyl groups.

Examples of the aryl group of 6 to 20 carbon atoms include phenyl, 1-naphthyl, 2-naphthyl, 1-anthryl, 2-anthryl, 9-anthryl, 1-phenanthryl, 2-phenanthryl, 3-phenanthryl, 4-phenanthryl and 9-phenanthryl groups.

Examples of the heteroaryl group of 2 to 20 carbon atoms include 2-thienyl, 3-thienyl, 2-furanyl, 3-furanyl, 2-oxazolyl, 4-oxazolyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl, 2-imidazolyl, 4-imidazolyl, 2-pyridyl, 3-pyridyl and 4-pyridyl groups.

Of these, R¹ and R² are preferably hydrogen atoms, fluorine atoms, cyano groups, alkyl groups of 1 to 20 carbon atoms which are optionally substituted with halogen atoms, aryl groups of 6 to 20 carbon atoms which are optionally substituted with halogen atoms, or heteroaryl groups of 2 to 20 carbon atoms which are optionally substituted with halogen atoms; more preferably hydrogen atoms, fluorine atoms, cyano groups, alkyl groups of 1 to 10 carbon atoms which are optionally substituted with halogen atoms, or phenyl groups which are optionally substituted with halogen atoms; even more preferably hydrogen atoms or fluorine atoms; and most preferably hydrogen atoms.

In the formulas (A1) and (A2), Ph¹ is a group of formula (PI).

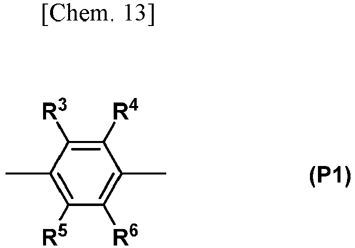

In the formula, R³ and R⁶ are each independently a hydrogen atom, a halogen atom, a nitro group, a cyano group, or an alkyl group of 1 to 20 carbon atoms, alkenyl group of 2 to 20 carbon atoms, alkynyl group of 2 to 20 carbon atoms, aryl group of 6 to 20 carbon atoms or heteroaryl group of 2 to 20 carbon atoms which is optionally substituted with a halogen atom. Specific examples thereof include the same as those mentioned above for R¹ and R².

In particular, R³ to R⁶ are preferably hydrogen atoms, fluorine atoms, cyano groups, alkyl groups of 1 to 20 carbon atoms which are optionally substituted with halogen atoms, aryl groups of 6 to 20 carbon atoms which are optionally substituted with halogen atoms, or heteroaryl groups of 2 to 20 carbon atoms which are optionally substituted with halogen atoms; more preferably hydrogen atoms, fluorine atoms, cyano groups, alkyl groups of 1 to 10 carbon atoms which are optionally substituted with halogen atoms, or phenyl groups which are optionally substituted with halogen atoms; even more preferably hydrogen atoms or fluorine atoms; and most preferably hydrogen atoms.

Examples of suitable groups for Ph¹ include, but are not limited to, a 1,4-phenylene group.

Each Ar¹ in formula (A1) is independently a group of any of formulas (B1) to (B 11), and more preferably a group of any of formulas (B1') to (B11').

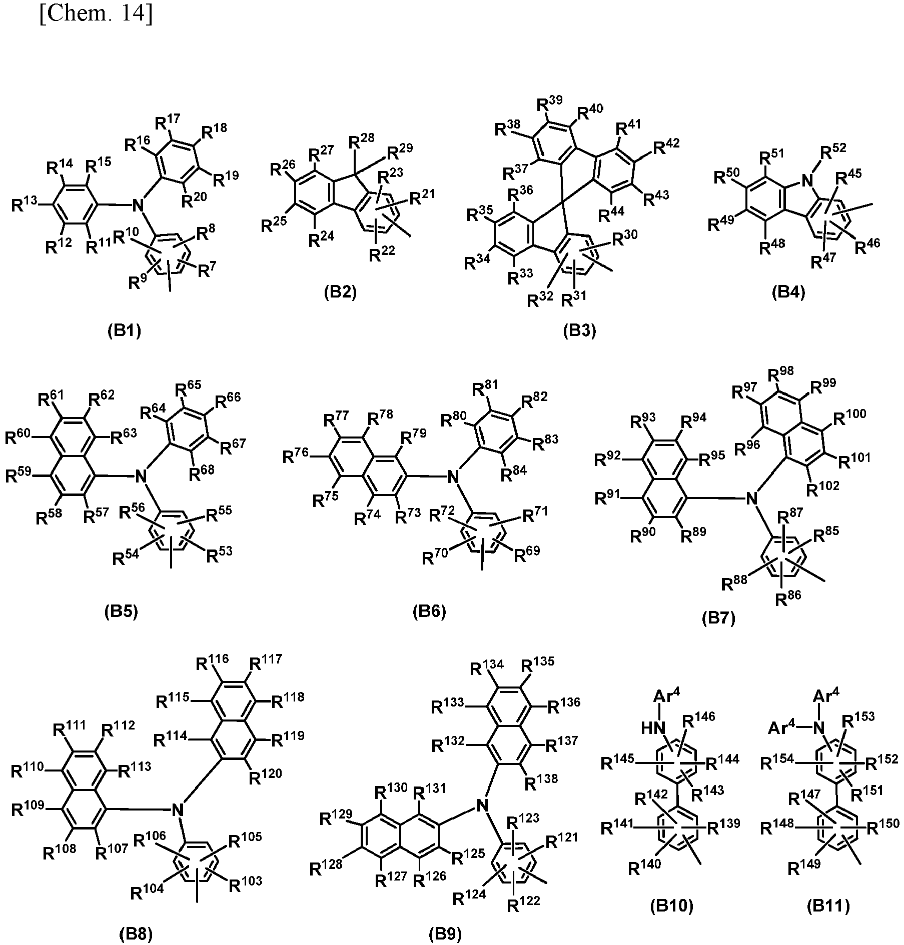

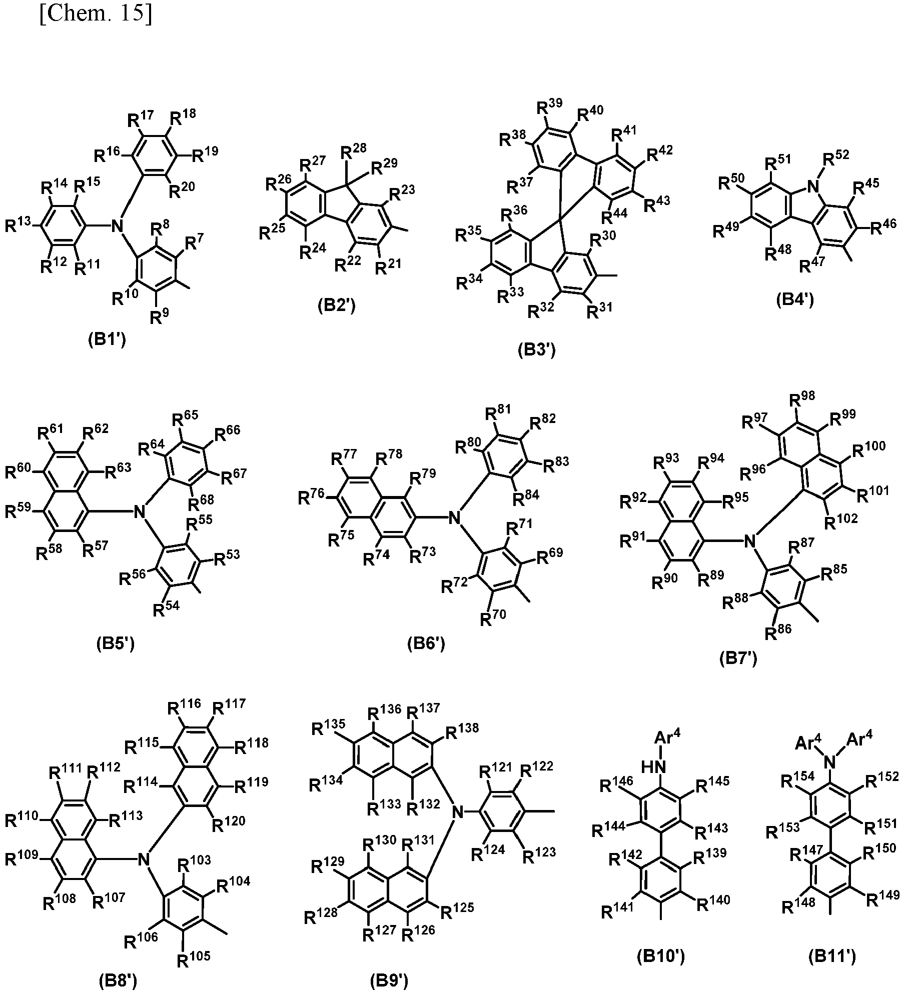

In formulas (B1) to (B11) and (B1') to (B11'), R⁷ to R²⁷, R³⁰ to R⁵¹ and R⁵³ to R¹⁵⁴ are each independently a hydrogen atom, a halogen atom, a nitro group, a cyano group, or a diphenylamino group, an alkyl group of 1 to 20 carbon atoms, alkenyl group of 2 to 20 carbon atoms, alkynyl group of 2 to 20 carbon atoms, aryl group of 6 to 20 carbon atoms or heteroaryl group of 2 to 20 carbon atoms which is optionally substituted with a halogen atom. R²⁸ and R²⁹ are each independently an aryl group of 6 to 20 carbon atoms or a heteroaryl group of 2 to 20 carbon atoms which is optionally substituted with Z¹. R⁵² is an aryl group of 6 to 20 carbon atoms or a heteroaryl group of 2 to 20 carbon atoms which is optionally substituted with Z¹.

Z¹ is a halogen atom, a nitro group or a cyano group, or an alkyl group of 1 to 20 carbon atoms, an alkenyl group of 2 to 20 carbon atoms, or an alkynyl group of 2 to 20 carbon atoms which is optionally substituted with Z². Z² is a halogen atom, a nitro group or a cyano group, or an aryl group of 6 to 20 carbon atoms or a heteroaryl group of 2 to 20 carbon atoms which is substituted with Z³. Z³ is a halogen atom, a nitro group or a cyano group.

In particular, R⁷ to R²⁷, R³⁰ to R⁵¹ and R⁵³ to R¹⁵⁴ are preferably hydrogen atoms, fluorine atoms, cyano groups, diphenylamino groups optionally substituted with halogen atoms, alkyl groups of 1 to 20 carbon atoms which are optionally substituted with halogen atoms, aryl groups of 6 to 20 carbon atoms which are optionally substituted with halogen atoms, or heteroaryl groups of 2 to 20 carbon atoms which are optionally substituted with halogen atoms; more preferably hydrogen atoms, fluorine atoms, cyano groups, alkyl groups of 1 to 10 carbon atoms which are optionally substituted with halogen atoms, or phenyl groups which are optionally substituted with halogen atoms; even more preferably hydrogen atoms or fluorine atoms; and most preferably hydrogen atoms.

R²⁸ and R²⁹ are preferably aryl groups of 6 to 14 carbon atoms which are optionally substituted with halogen atoms or heteroaryl groups of 2 to 14 carbon atoms which are optionally substituted with halogen atoms; more preferably phenyl groups optionally substituted with halogen atoms or naphthyl groups optionally substituted with halogen atoms; even more preferably phenyl groups optionally substituted with halogen atoms; and still more preferably phenyl groups.

R⁵² is preferably a hydrogen atom or an aryl group of 6 to 20 carbon atoms which is optionally substituted with Z¹; more preferably a hydrogen atom, a phenyl group optionally substituted with Z¹, or a naphthyl group optionally substituted with Z¹; even more preferably a phenyl group optionally substituted with Z¹; and still more preferably a phenyl group.

Each Ar⁴ in formulae (B10), (B11), (B10') and (B11') is independently an aryl group of 6 to 20 carbon atoms, which is optionally substituted with a diarylamino group in which each aryl group is an aryl group of 6 to 20 carbon atoms. Specific examples of the aryl group of 6 to 20 carbon atoms include the same as those mentioned above for R¹ and R². Specific examples of the diarylamino group include diphenylamino, 1-naphthylphenylamino, di(1-naphthyl)amino, 1-naphthyl-2-naphthylamino and di(2-naphthyl)amino groups.

Ar⁴ is preferably a phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, p-(diphenylamino)phenyl, p-(1-naphthylphenylamino)phenyl, p-(di(1-naphthyl)amino)phenyl, p-(1-naphthyl-2-naphthylamino)phenyl or p-(di(2-naphthyl)amino)phenyl group; and more preferably a p-(diphenylamino)phenyl group.

Each Ar² in formula (A1) is independently a group of any of formulas (C1) to (C18), and particularly preferably a group of any of formulas (C1'-1) to (C18'-2). In the following formula, Ar⁴ is the same as described above, and DPA is a diphenylamino group.

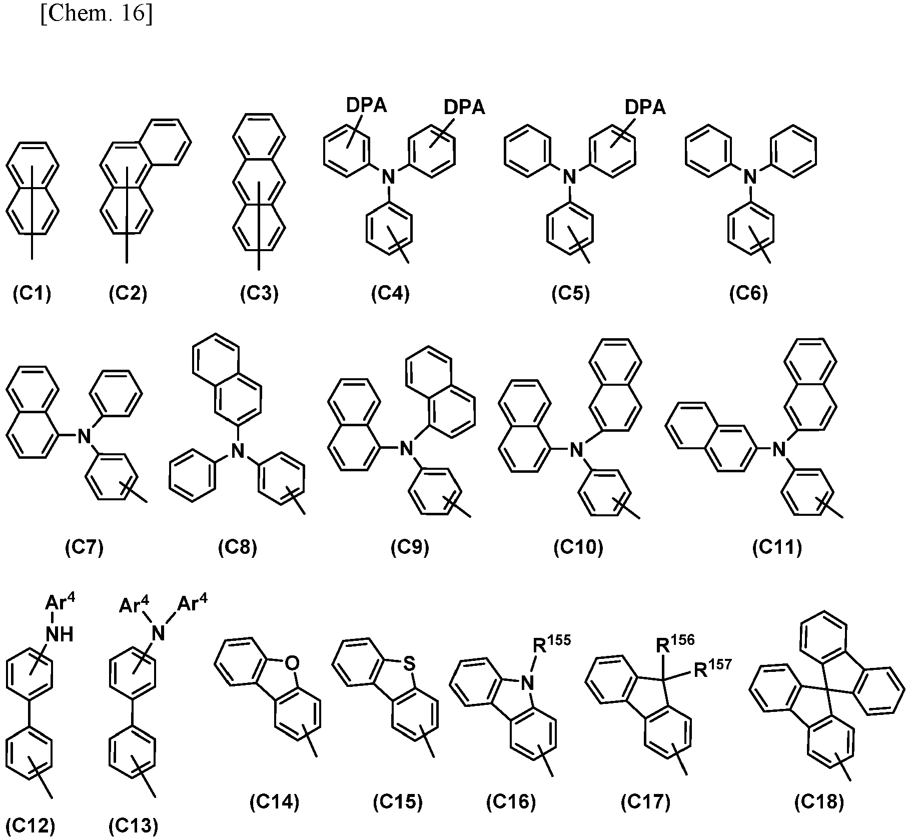

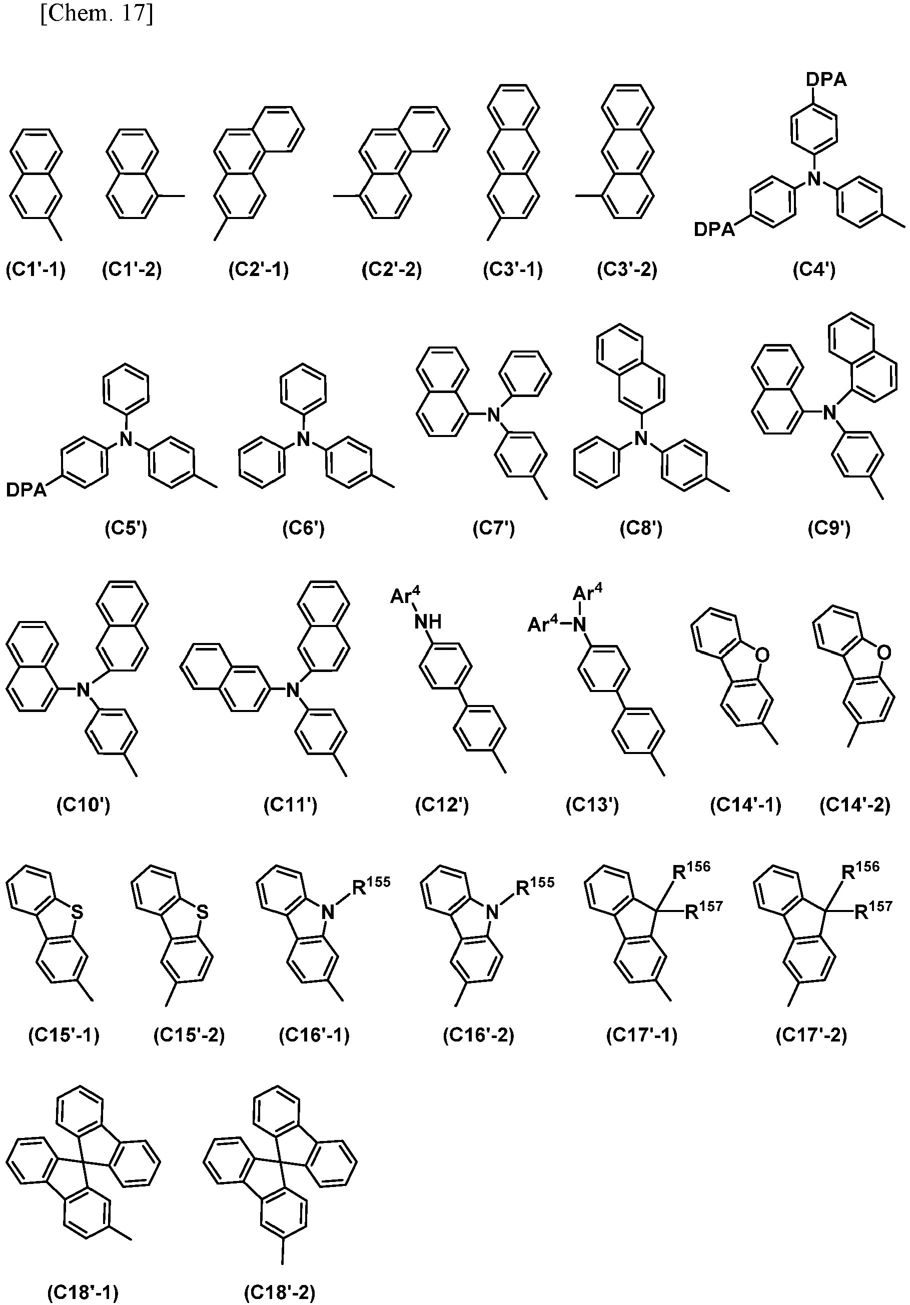

In the formulae (C16), (C16'-1) and (C16'-2), R¹⁵⁵ is a hydrogen atom, an aryl group of 6 to 14 carbon atoms which is optionally substituted with Z¹, or a heteroaryl group of 2 to 14 carbon atoms which is optionally substituted with Z¹. Examples of the aryl group and the heteroaryl group include the same as those mentioned above for R¹ and R². Of these, R¹⁵⁵ is preferably a hydrogen atom, a phenyl group optionally substituted with Z¹, a 1-naphthyl group optionally substituted with Z¹, a 2-naphthyl group optionally substituted with Z¹, a 2-pyridyl group optionally substituted with Z¹, a 3-pyridyl group optionally substituted with a phenyl group optionally substituted with Z¹, or a 4-pyridyl group optionally substituted with Z¹; even more preferably a phenyl group optionally substituted with Z¹; and even more preferably a phenyl group or a (2,3,5,6-tetrafluoro-4-(trifluoromethyl)phenyl) group.

In formulae (C17), (C17'-1) and (C17'-2), R¹⁵⁶ and R¹⁵⁷ are aryl groups of 6 to 14 carbon atoms which are optionally substituted with phenyl groups optionally substituted with Z¹, or heteroaryl groups of 2 to 14 carbon atoms which are optionally substituted with phenyl groups optionally substituted with Z¹. Examples of the aryl group and the heteroaryl group include the same as those mentioned above for R¹ and R². Of these, R¹⁵⁶ and R¹⁵⁷ are preferably aryl groups of 6 to 14 carbon atoms which are optionally substituted with phenyl groups optionally substituted with Z¹; and more preferably phenyl groups optionally substituted with phenyl groups optionally substituted with Z¹, 1-naphthyl groups optionally substituted with phenyl groups optionally substituted with Z¹, or 2-naphthyl groups optionally substituted with Z¹.

In the formula (A2), Ar³ is a group of any of formulae (D1) to (D8), and particularly preferably a group of any of (D1') to (D8').

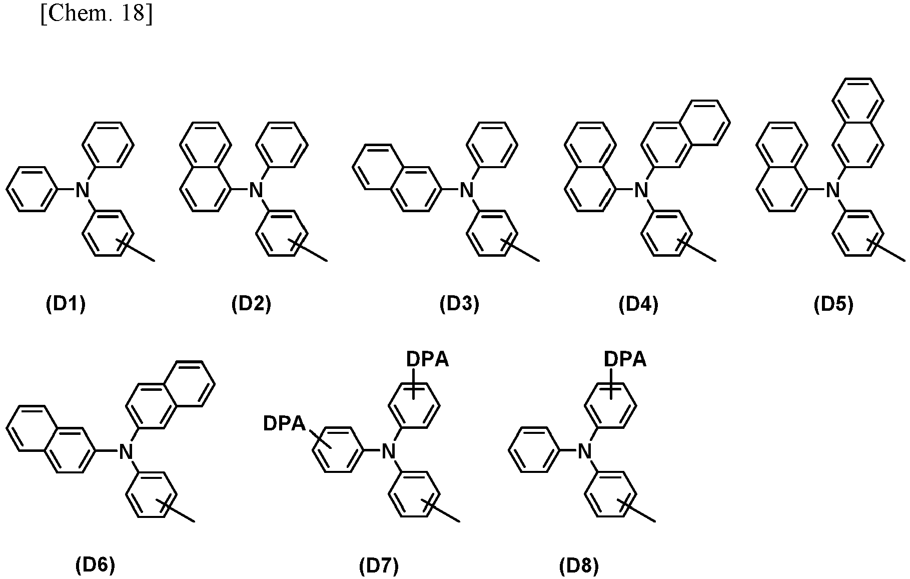

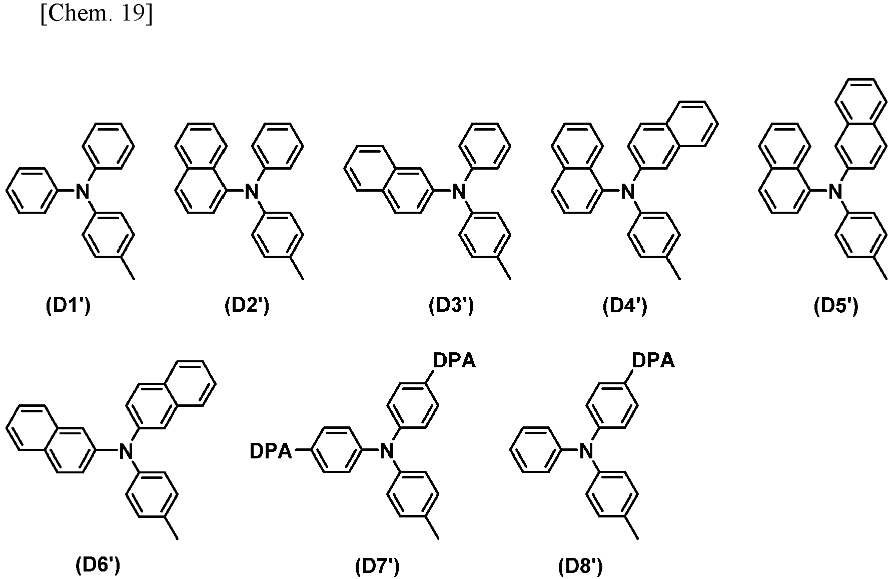

In formula (A1), the subscript is an integer from 1 to 10. From the standpoint of increasing the solubility of the compound in organic solvents, p is preferably from 1 to 5, more preferably from 1 to 3, even more preferably 1 or 2, and most preferably 1. In formula (A2), q is 1 or 2.

The aniline derivative of formula (A1) and the aniline derivative of formula (A2) can be produced according to, for example, the method described in WO 2015/050253.

Other preferred examples of the tertiary aryl amine compound include compounds of the following formula (A3).

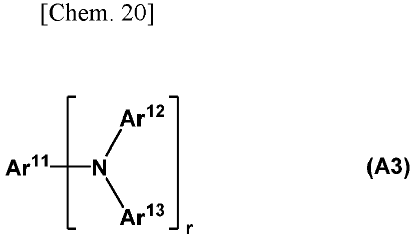

In formula (A3), r is an integer of 2 to 4. Ar¹¹ is an optionally substituted r-valent aromatic group of 6 to 20 carbon atoms. The aromatic group is a group obtained by removing r hydrogen atoms from an aromatic ring of an aromatic compound of 6 to 20 carbon atoms. The aromatic group is particularly preferably a group derived from a compound of any of the following formulae (A3-1) to (A3-8).

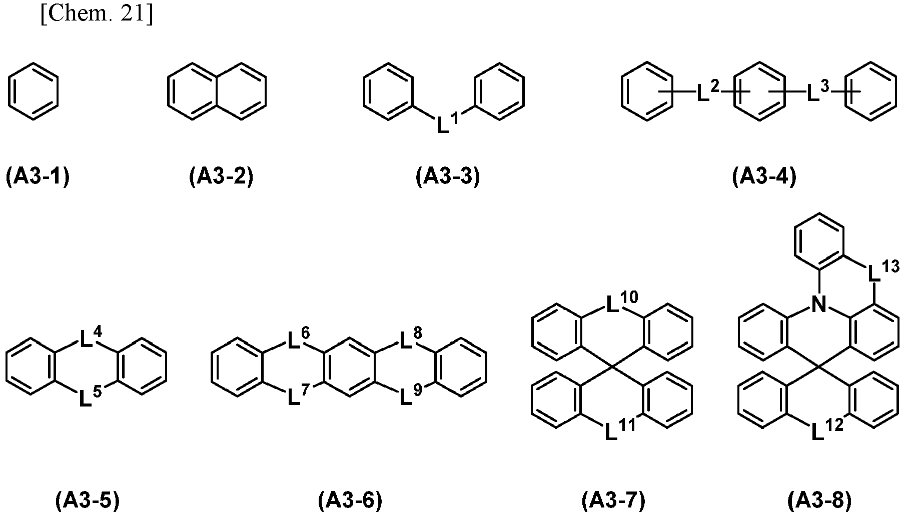

In the formula, L¹ to L³ are each independently a single bond, -(CR²⁰¹R²⁰²)ₛ-, -C(O)-, -O-, -S-, -S(O)-, -S(O₂)- or -NR²⁰³-. s is an integer of 1 to 6. L⁴ to L¹³ are each independently a single bond, -CR²⁰¹R²⁰²-, -C(O)-, -O-, -S-, -S(O)-, -S(O₂)- or -NR²⁰³-. R²⁰¹ and R²⁰² are each independently a hydrogen atom or a monovalent hydrocarbon group of 1 to 20 carbon atoms. R²⁰¹ and R²⁰² may be bonded to each other to form a ring with carbon atoms to which these groups are bonded. In (CR²⁰¹R²⁰²)ₛ-, R²⁰¹ and R²⁰² may be identical to or different from each other when s is 2 or more. R²⁰³ is a hydrogen atom or a monovalent hydrocarbon group of 1 to 20 carbon atoms.

The aromatic group is optionally substituted with substituents at some or all of hydrogen atoms thereof. Examples of the substituent include the same as those mentioned above for A¹ in formula (1), and the substituent is preferably a halogen atom, a nitro group, a cyano group or a monovalent hydrocarbon group of 1 to 20 carbon atoms.

Ar¹¹ is preferably an optionally substituted 1,4-phenylene group, a fluorene-2,7-diyl group, a 9,9-dimethylfluorene-2,7-diyl group or the like, more preferably an optionally substituted 1,4-phenylene group or a biphenyl-4,4'-diyl group.

In formula (A3), Ar¹² and Ar¹³ are each independently a monovalent aromatic group of 6 to 20 carbon atoms which is optionally substituted with Z¹¹. Ar¹² and Ar¹³ may be bonded to each other to form a ring with nitrogen atoms to which these groups are bonded. Ar¹² and Ar¹³ may be identical to or different from each other. Z¹¹ is a halogen atom, a nitro group, a cyano group, or a monovalent aliphatic hydrocarbon group of 1 to 20 carbon atoms or a monovalent aromatic group which is optionally substituted with a halogen atom, or a polymerizable group.

Examples of the monovalent aromatic group include aryl groups such as a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 2-biphenyl group, a 3-biphenyl group and a 4-biphenyl group.

The monovalent aliphatic hydrocarbon may be linear, branched or cyclic, and specific examples thereof include alkyl groups of 1 to 20 carbon atoms such as a methyl group, an ethyl group, a n-propyl group, an isopropyl group, an n-butyl group, an isobutyl group, a sec-butyl group, a tert-butyl group, an n-pentyl group, a cyclohexyl group, an n-hexyl group, an n-cyclohexyl group, an n-heptyl group, an n-octyl group, an n-nonyl group, an n-decyl group, an n-undecyl group, and an n-dodecyl group; and alkenyl groups of 2 to 20 carbon atoms such as a vinyl group, a 1-propenyl group, a 2-propenyl group, an isopropenyl group, a 1-methyl-2-propenyl group, a 1-butenyl group, a 2-butenyl group, a 3-butenyl group and a hexenyl group.

Examples of the polymerizable group include, but are not limited to, those of the following formula.

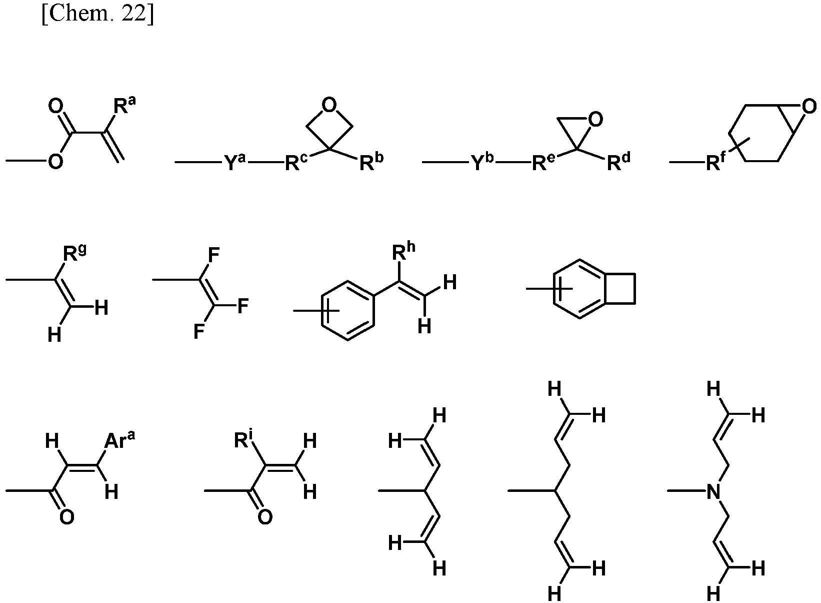

In the formula, R^{a} is a hydrogen atom or a methyl group. R^{b} and R^{d} are each independently a hydrogen atom or an alkyl group of 1 to 6 carbon atoms, and preferably a methyl group or an ethyl group. R^{c}, R^{e} and R^{f} are each independently a single bond or an alkylene group of 1 to 8 carbon atoms which optionally contains an oxygen atom, a sulfur atom or a nitrogen atom. R^{g}, R^{h} and Rⁱ are each independently a hydrogen atom or an alkyl group of 1 to 10 carbon atoms such as a methyl group, an ethyl group or an n-propyl group.

Y^{a} and Y^{b} are each independently a single bond or a divalent aromatic group of 6 to 20 carbon atoms. Examples of the divalent aromatic group include a 1,3-phenylene group, a 1,4-phenylene group, a 1,5-naphthylene group, a 1,6-naphthylene group, a 1,7-naphthylene group, a 2,6-naphthylene group and a 4,4'-biphenylylene group. Of these, a 1,3-phenylene group or a 1,4-phenylene group is preferred.

Ar^{a} is a monovalent aromatic group of 6 to 20 carbon atoms which optionally has a substituent. Examples of the monovalent aromatic group include the same as those mentioned above.

Z¹¹ is preferably a methyl group, an ethyl group, or a polymerizable group of the following formula.

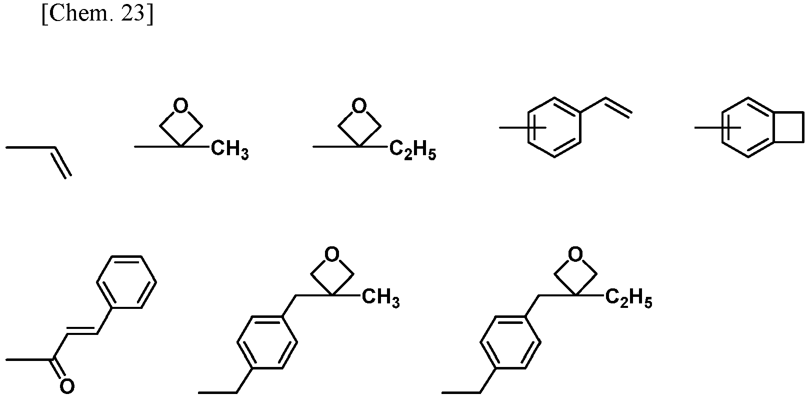

Ar¹² and Ar¹³ are each preferably a phenyl group, a 2-methylphenyl group, a 3-methylphenyl group, a 4-methylphenyl group, a 2-ethylphenyl group, a 3-ethylphenyl group, a 4-ethylphenyl group, a 2-vinylphenyl group, a 3-vinylphenyl group, a 4-vinylphenyl group, a 1-naphthyl group, a 2-naphthyl group or the like.

The compound of formula (A3) can be synthesized by a known method, and a commercially available product can also be used.

Other preferred examples of the tertiary aryl amine compound include those of the following formula (A4).

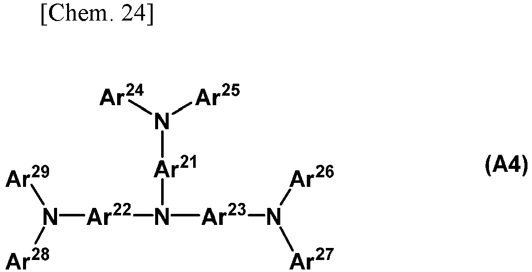

In formula (A4), Ar²¹ to Ar²³ are each independently a divalent aromatic group of 6 to 20 carbon atoms. The divalent aromatic group is furthermore preferably a divalent group derived from a compound of the above formula (A3-1), (A3-3) or (A3-4).

Of these, Ar²¹ to Ar²³ are each preferably a 1,4-phenylene group, a biphenyl-4,4'-diyl group, a terphenyl-4,4"-diyl group or the like, more preferably a 1,4-phenylene group or a biphenyl A -4,4'-diyl group.

In formula (A4), Ar²⁴ to Ar²⁹ each independently represent a monovalent aromatic group of 6 to 20 carbon atoms which is substituted with Z²¹. Examples of the monovalent aromatic group include aryl groups such as a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 2-biphenyl group, a 3-biphenyl group and a 4-biphenyl group.

Z²¹ is a halogen atom, a nitro group, a cyano group, a monovalent aliphatic hydrocarbon group of 1 to 20 carbon atoms which is optionally substituted with a halogen atom, a nitro group or a cyano group, -N(Ar³⁰)(Ar³¹), or a polymerizable group. The monovalent aliphatic hydrocarbon group of 1 to 20 carbon atoms may be linear, branched or cyclic, and specific examples thereof include alkyl groups of 1 to 20 carbon atoms such as a methyl group, an ethyl group, an n-propyl group, an isopropyl group, an n-butyl group, an isobutyl group, a sec-butyl group, a tert-butyl group, an n-pentyl group, a cyclohexyl group, an n-hexyl group, an n-cyclohexyl group, an n-heptyl group, an n-octyl group, an n-nonyl group, an n-decyl group, an n-undecyl group, and an n-dodecyl group; and alkenyl groups of 2 to 20 carbon atoms such as a vinyl group, a 1-propenyl group, a 2-propenyl group, an isopropenyl group, a 1-methyl-2-propenyl group, a 1-butenyl group, a 2-butenyl group, a 3-butenyl group and a hexenyl group. Examples of the polymerizable group include the same as those mentioned above.

Ar³⁰ and Ar³¹ are each independently an aryl group of 6 to 20 carbon atoms which is optionally substituted with Z²². Ar³⁰ and Ar³¹ may be bonded to each other to form a ring with nitrogen atoms to which these groups are bonded. Z²² is a halogen atom, a nitro group, a cyano group, or a monovalent aliphatic hydrocarbon group of 1 to 20 carbon atoms which is optionally substituted with a halogen atom, a nitro group or a cyano group.

Examples of the aryl group of 6 to 20 carbon atoms and the monovalent aliphatic hydrocarbon group of 1 to 20 carbon atoms include the same as those mentioned above.

Ar³⁰ and Ar³¹ are each preferably a phenyl group, a 1-naphthyl group, a 2-naphthyl group, or a 1-biphenylyl group, more preferably a phenyl group, a 1-biphenylyl group or the like.

In particular, -N(Ar³⁰)(Ar³¹) is preferably a diphenylamino group, a phenyl(4-biphenylyl)amino group, a bis(4-biphenylyl)amino group, an N-carbazolyl group or the like.

Z²¹ is preferably an alkyl group of 1 to 10 carbon atoms, -N(Ar³⁰)(Ar³¹) or the like.

Ar²⁴ to Ar²⁹ are each preferably a phenyl group, a 4-biphenylyl group, a 4-diphenylaminophenyl group, a 4-phenyl(4-biphenylyl)aminophenyl group, a bis(4-biphenylyl)aminophenyl group, a 4'-diphenylamino-4-biphenylyl group, a 4-phenyl(4-biphenylyl)amino-4-biphenylyl group, a 4'-bis(4-biphenylyl)amino-4-biphenylyl group, a N-carbazolylphenyl group, a 4'-N-carbazolyl- A 4-biphenylyl group or the like.

The compound of formula (A4) can be synthesized by a known method, and a commercially available product can also be used.

Other preferred examples of the tertiary aryl amine compound include polymers containing repeating units of the following formula (A5a) and repeating units of the following formula (A5b).

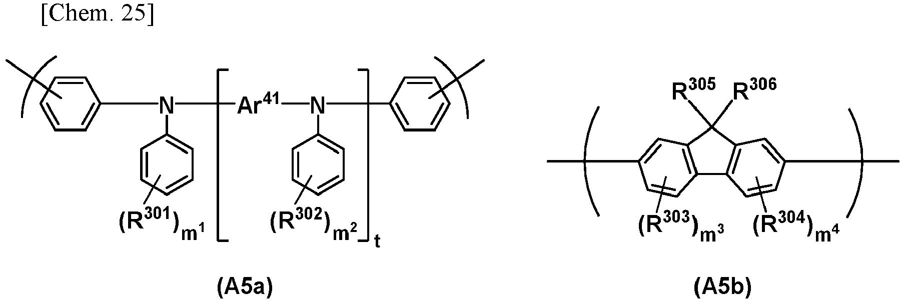

In formula (A5a), Ar⁴¹ is a divalent aromatic group of 6 to 20 carbon atoms. Examples of the divalent aromatic group include the same as those mentioned above for Ar²¹ to Ar²³ in formula (A4). Of these, a 1,4-phenylene group or a biphenyl-4,4'-diyl group is preferred.

In formulae (A5a) and (A5b), R³⁰¹ to R³⁰⁴ are each independently a monovalent hydrocarbon group of 1 to 20 carbon atoms, and are optionally substituted with -O-, -S-, -S(O)-, -S (O₂)-, -NR'-, a carbonyl group, an ester bond or a sulfonic acid ester bond at a part of -CH₂- which makes up the monovalent hydrocarbon group. R' is a hydrogen atom or a monovalent hydrocarbon group of 1 to 10 carbon atoms. The monovalent hydrocarbon group is preferably an alkyl group such as a methyl group, an ethyl group or an n-propyl group.

In formula (A5b), R³⁰⁵ and R³⁰⁶ are each independently a hydrogen atom or a monovalent hydrocarbon group of 1 to 20 carbon atoms. Examples of the monovalent hydrocarbon group include the same as those mentioned above for X³ in formula (1). Of these, alkyl groups of 1 to 10 carbon atoms are preferred, and alkyl groups of 1 to 6 carbon atoms are more preferred.

In formula (A5a), t is 0 or 1.

m¹ and m² are each independently an integer of 0 to 4, and preferably 1 or 2, more preferably 1. m³ and m⁴ are each independently an integer of 0 to 3, and preferably 0 or 1, more preferably 0.

The terminal of the polymer is optionally blocked with a polymerizable group. Examples of the polymerizable group include the same as those mentioned above.

The lower limit of the weight average molecular weight (Mw) of the polymer is preferably 1,000, more preferably 5,000, even more preferably 10,000, still more preferably 15,000, most preferably 20,000. On the other hand, the upper limit of the weight average molecular weight (Mw) of the polymer is preferably 1,000,000, more preferably 500,000, still more preferably 200,000. In the invention, the weight average molecular weight (Mw) is a value measured by gel permeation chromatography (GPC) with tetrahydrofuran as a solvent, and calculated as polystyrene.

The polymer can be synthesized by condensation polymerization of a triphenylamine compound which gives repeating units of formula (A5a) and a fluorene derivative which gives repeating units of formula (A5b), or a commercial products can also be used.

Other preferred examples of the tertiary aryl amine compound include those of the following formula (A6).

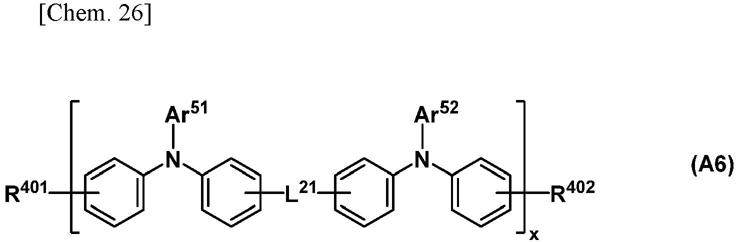

In the formula, Ar⁵¹ and Ar⁵² are each independently a phenyl group, a 1-naphthyl group or a 2-naphthyl group. R⁴⁰¹ and R⁴⁰² are each independently a hydrogen atom, a diarylaminophenyl group in which each aryl group is an aryl group of 6 to 20 carbon atoms, a chlorine atom, a bromine atom or an iodine atom. Examples of the aryl group include the same as those mentioned above for R¹ and R² in formula (A2). L²¹ is a divalent linking group containing a propane-2,2-diyl group or a 1,1,1,3,3,3-hexafluoropropane-2,2-diyl group. x is an integer of 1 or more.

The compound of formula (A6) can be synthesized by a known method, and a commercially available product can also be used.

Other preferred examples of the tertiary aryl amine compound include those of the following formula (A7).

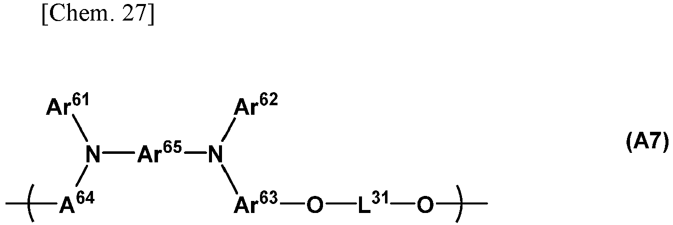

In formula (A7), Ar⁶¹ and Ar⁶² are each independently an optionally substituted monovalent aromatic group. Ar⁶³ to Ar⁶⁵ are each independently an optionally substituted divalent aromatic group. L³¹ is a linking group of any of the following formulae.

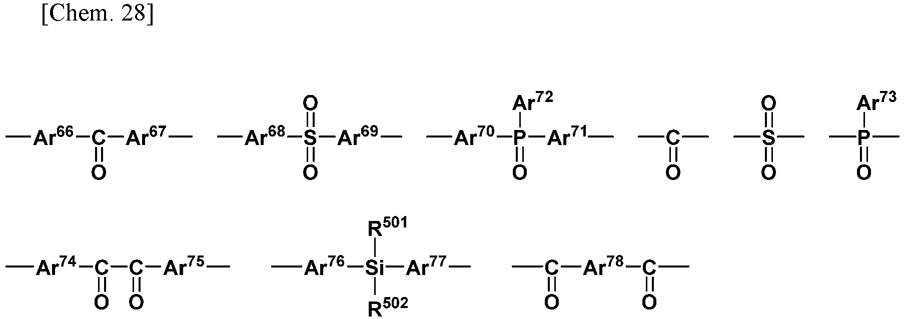

In the formula, Ar⁶⁶ to Ar⁷¹ and Ar⁷⁴ to Ar⁷⁸ are each independently an optionally substituted divalent aromatic group. Ar⁷² to Ar⁷³ are each independently an optionally substituted monovalent aromatic group. R⁵⁰¹ and R⁵⁰² are each independently a hydrogen atom or any substituent. The substituent is not particularly limited, provided that the effects of the invention are not impaired, and examples thereof include the same as the substituents mentioned above for A¹ in formula (1).

The compound of formula (A7) can be synthesized by a known method, and a commercially available product can also be used.

The tertiary aryl amine compound has at least one nitrogen atom is not limited to those described above, provided that all the nitrogen atoms form a tertiary aryl amine structure. Examples of other tertiary aryl amine compounds which can be used in the invention include arylamine compounds as described in WO 2005/094133, polymers as described in WO 2011/132702, paragraph [0180], aromatic tertiary amine polymer compounds as described in WO 2014/073683, fluorine atom-containing polymers as described in WO 2016/006674, polymerizable compounds having a triarylamine partial structure and a polymerizable group as described in JP No. 5287455, aromatic tertiary amine polymer compounds of formula (11) as described in JP No. 5381931, triarylamine compounds as described in JP No. 5602191, compounds as described in JP No. 6177771, paragraph [0054], and polymers containing these compounds as structural units.

Preferred examples of the tertiary aryl amine compounds include, but are not limited to, the following compounds. 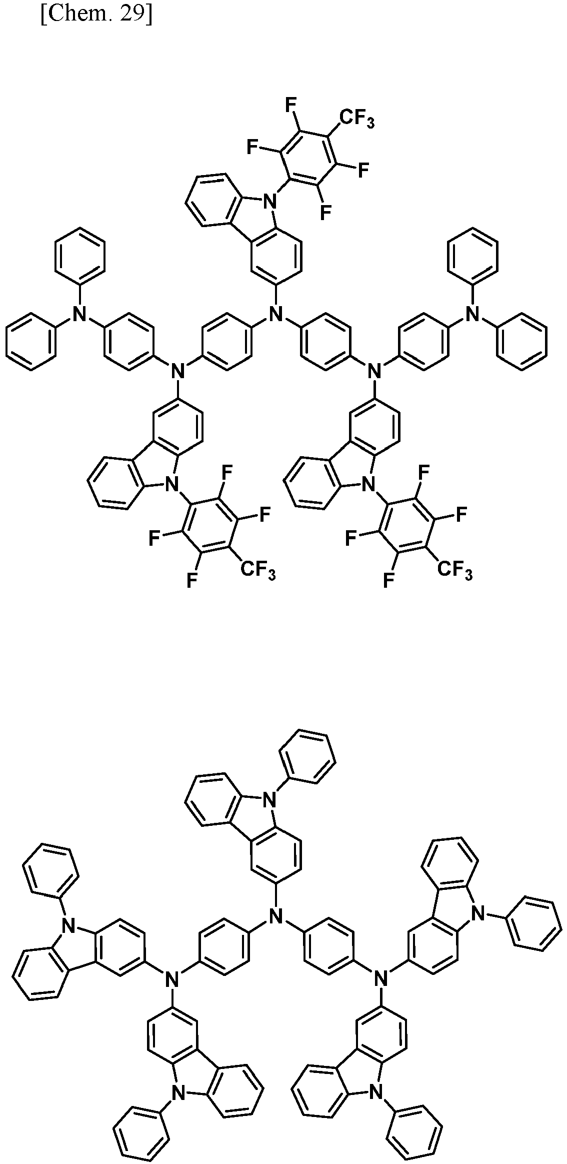 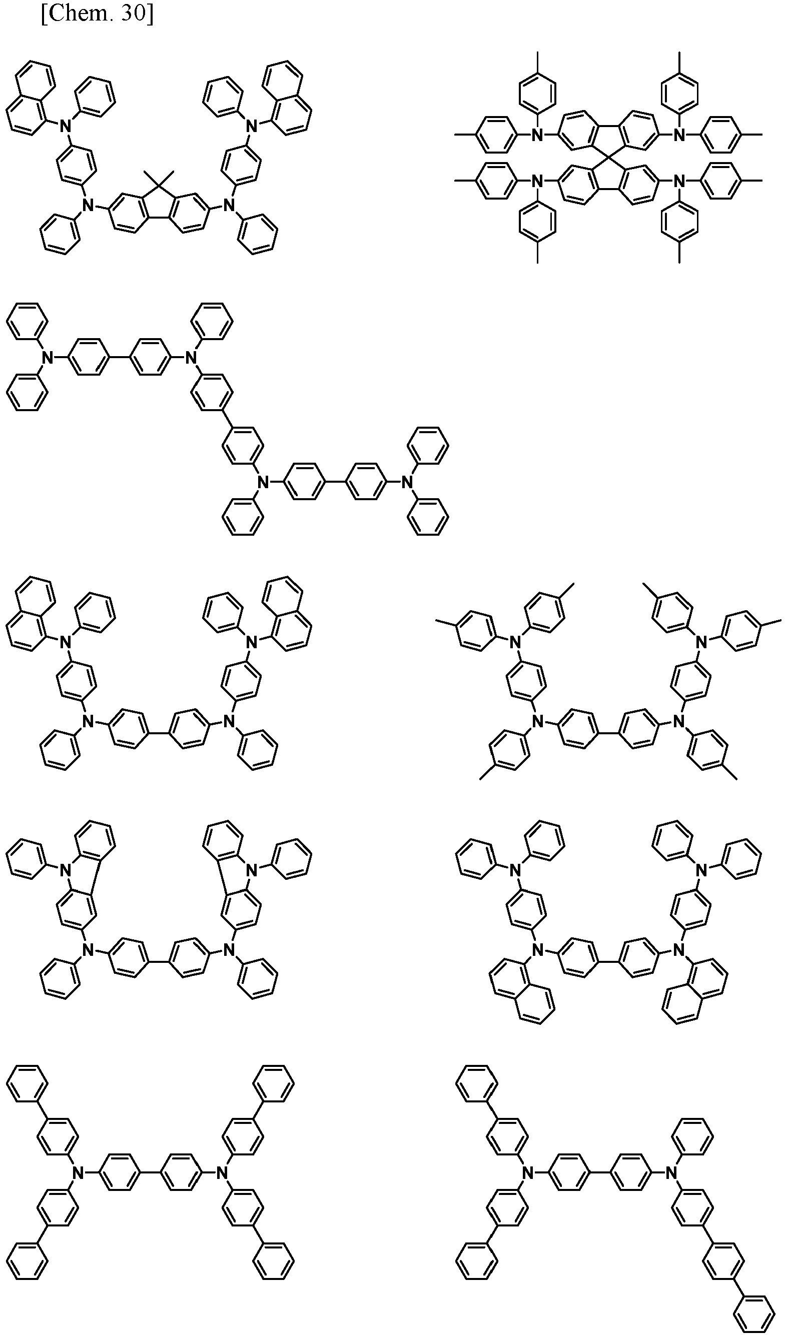 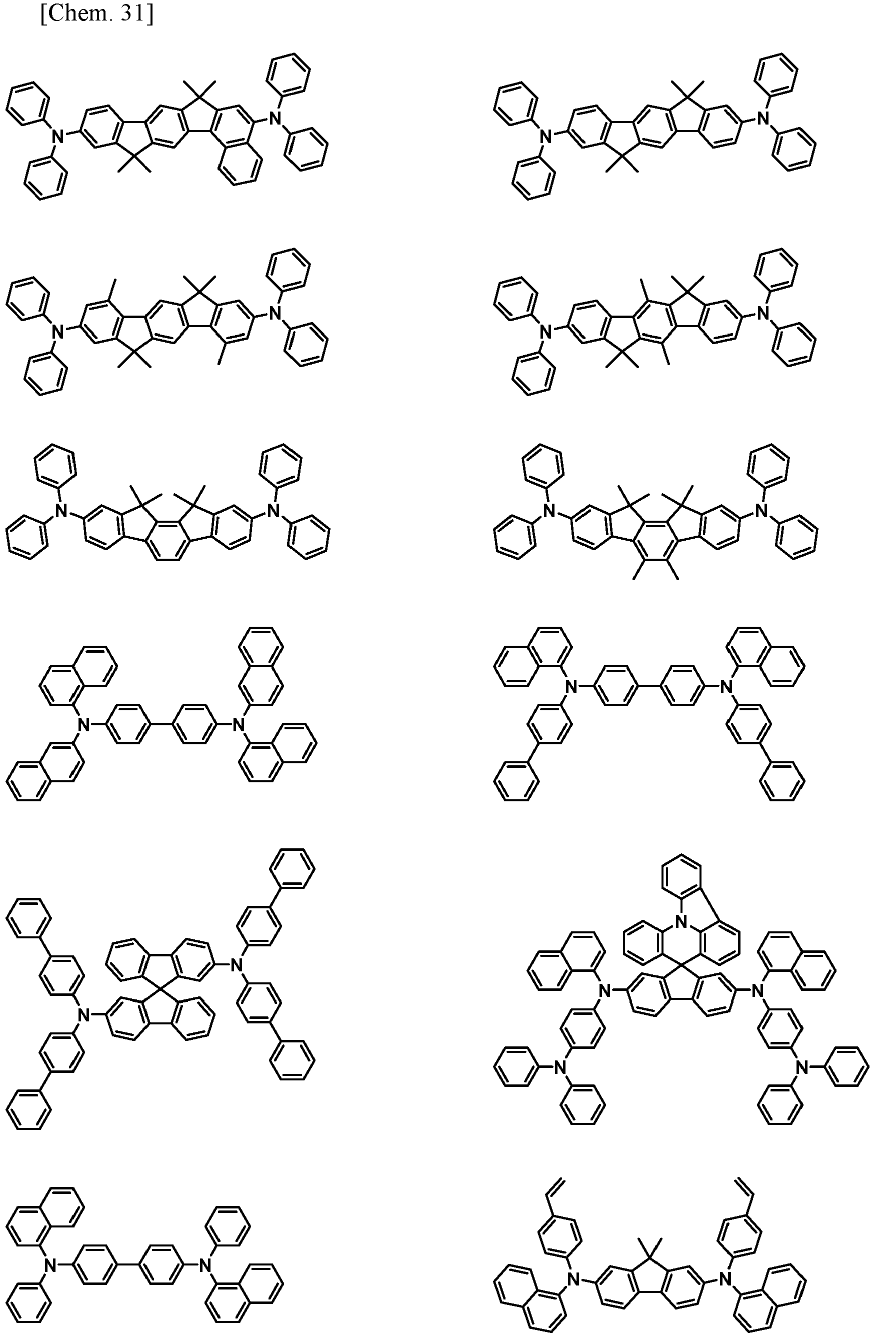 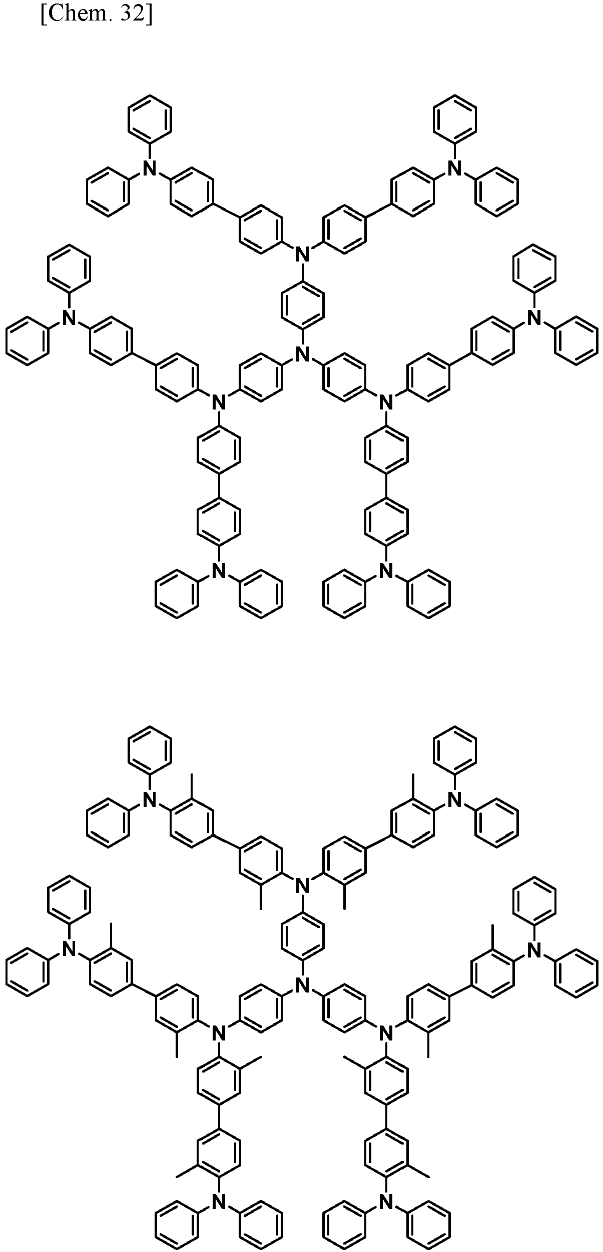 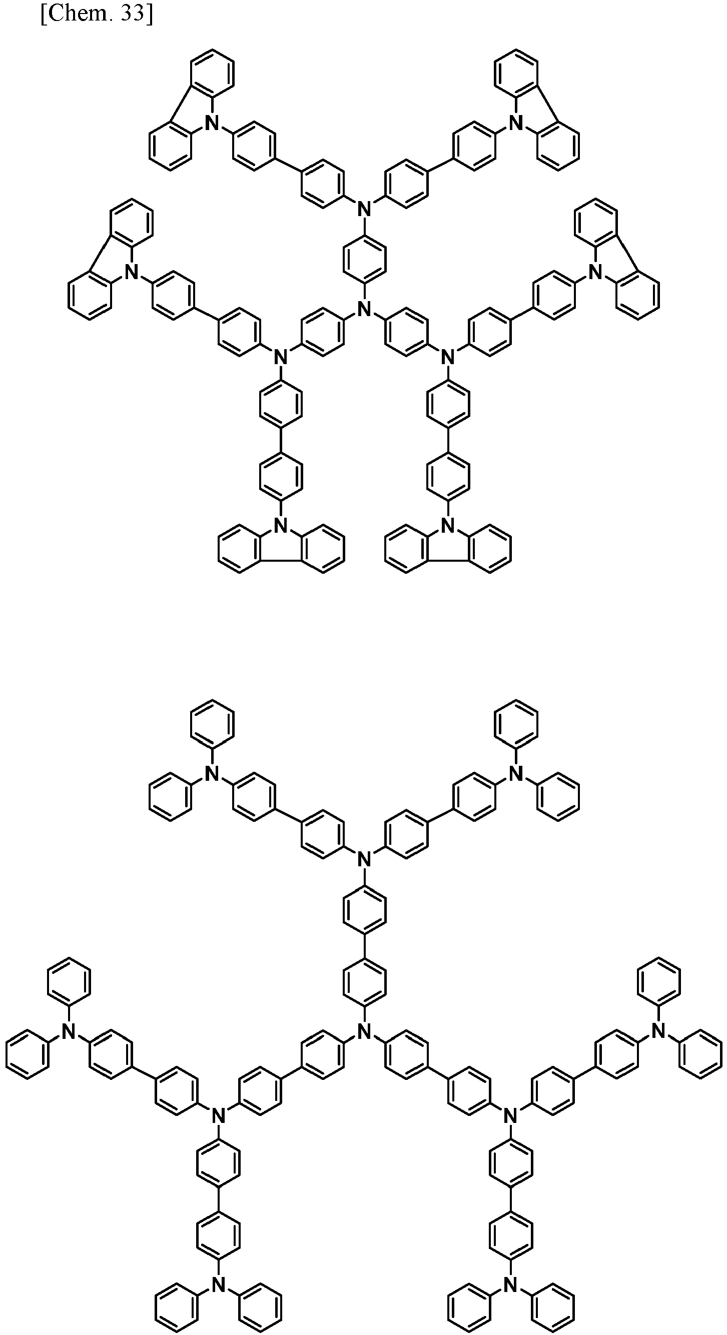 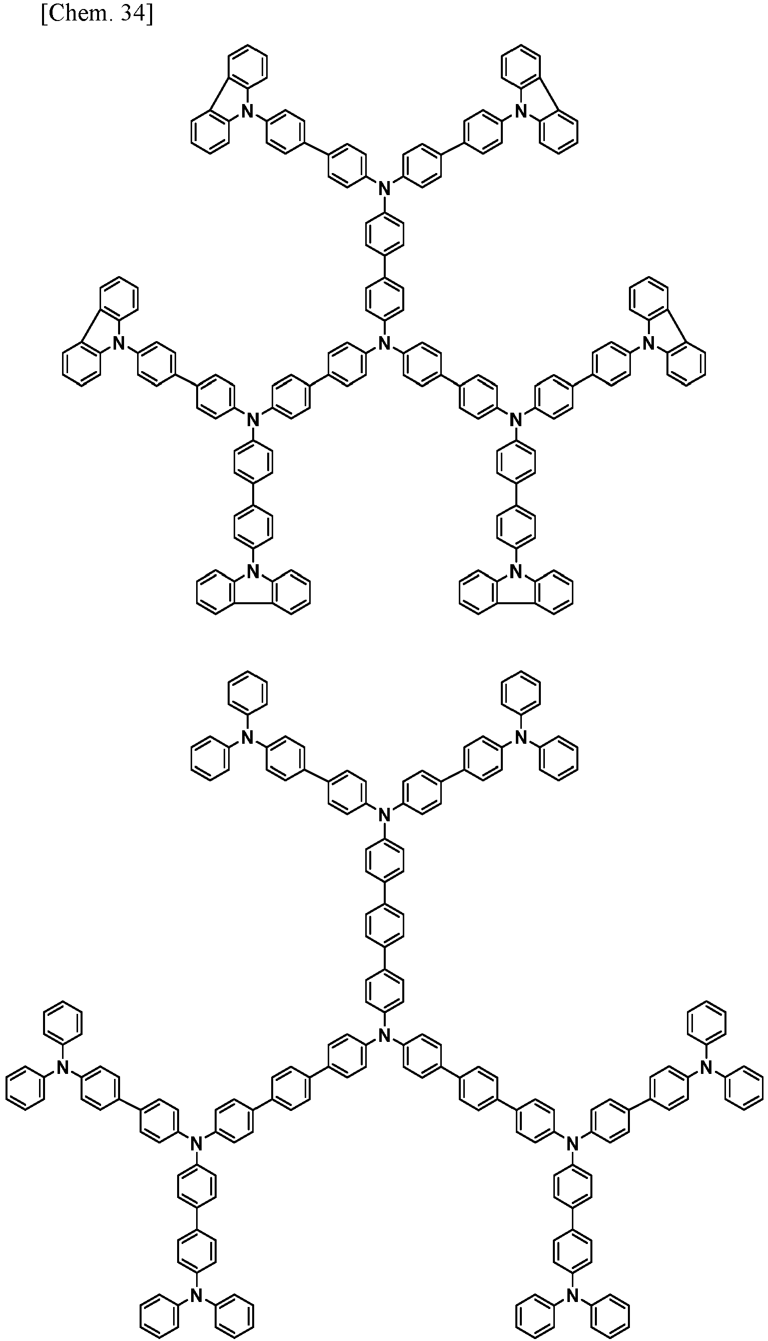 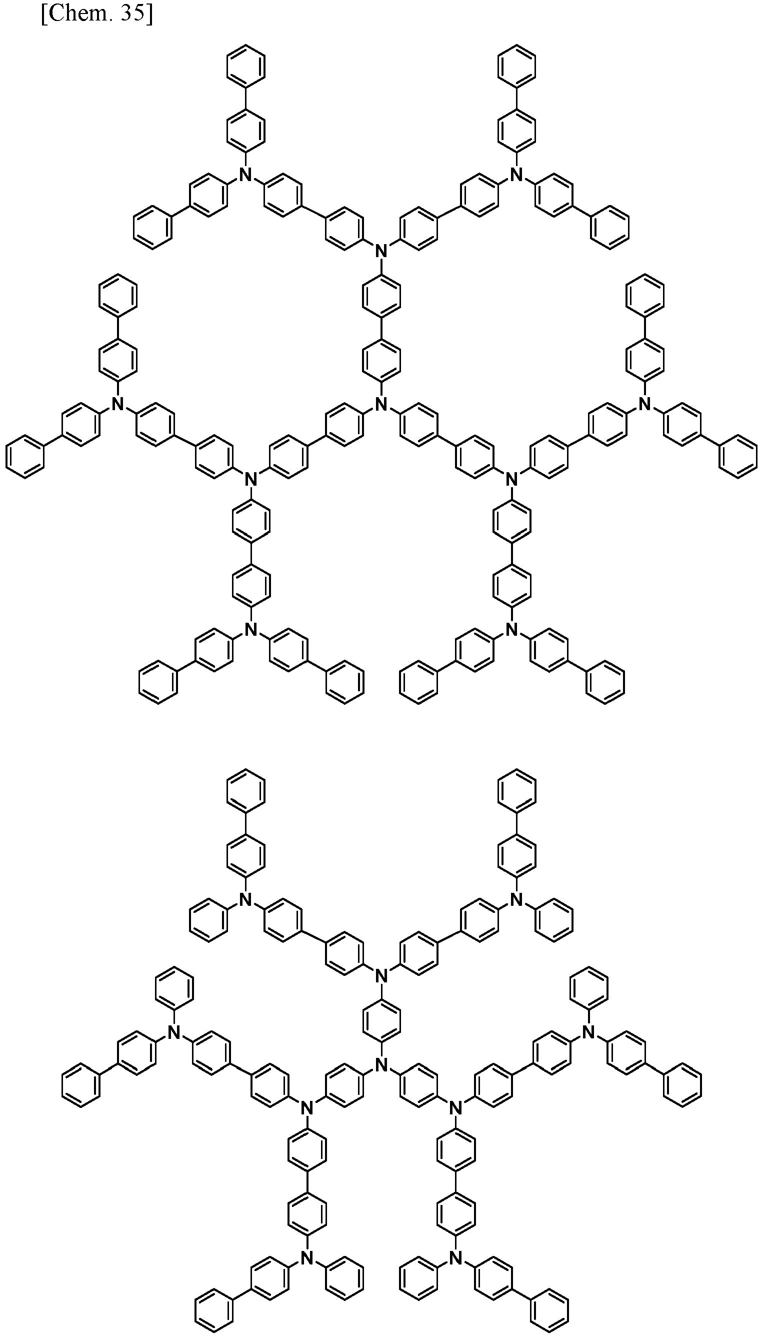 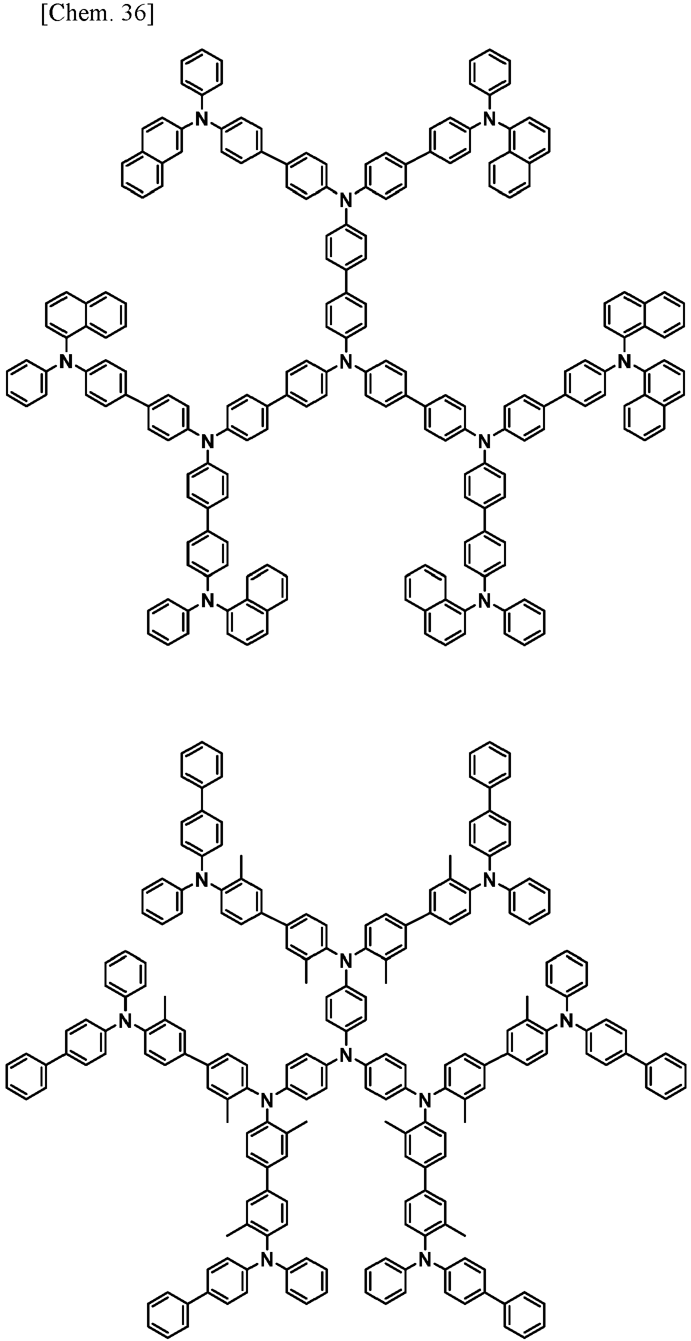 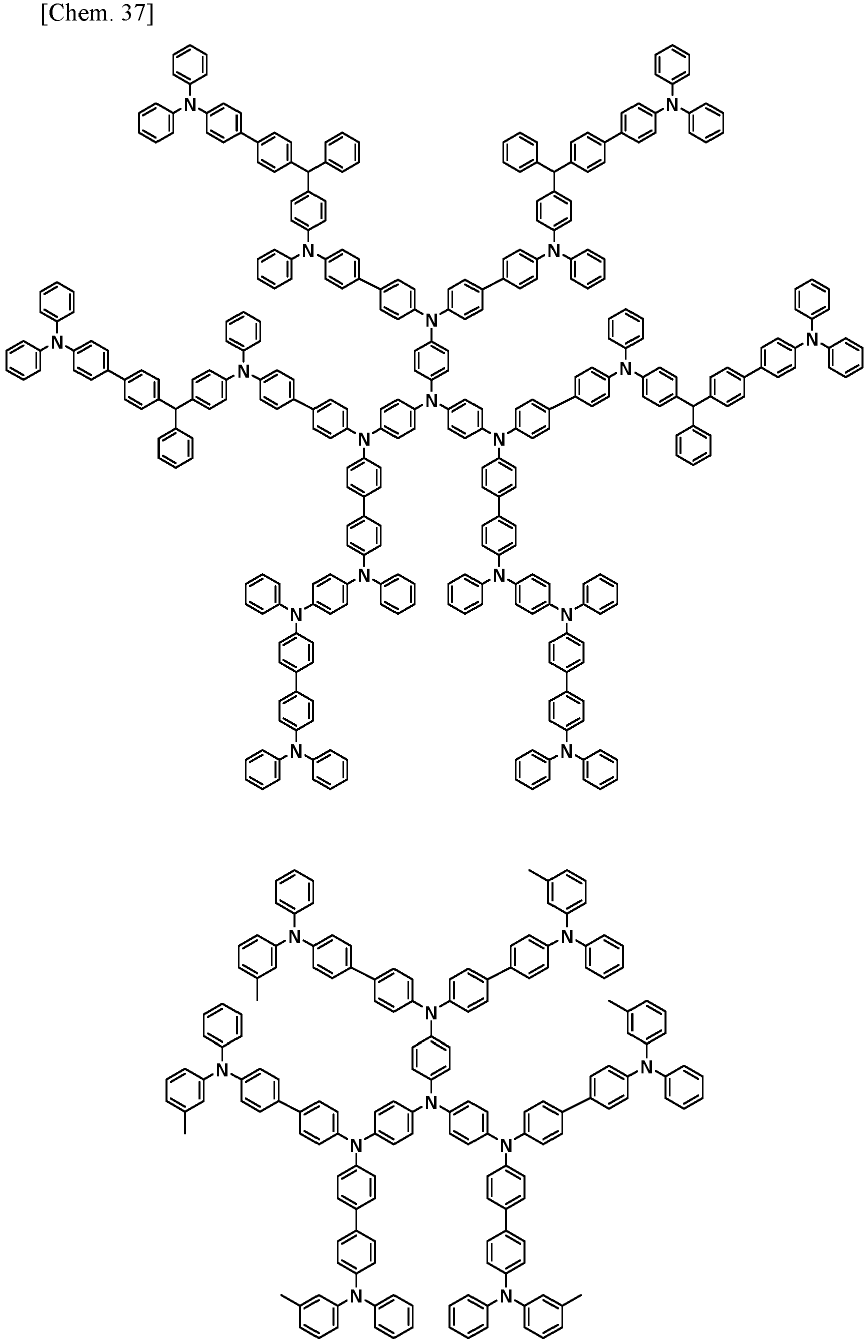 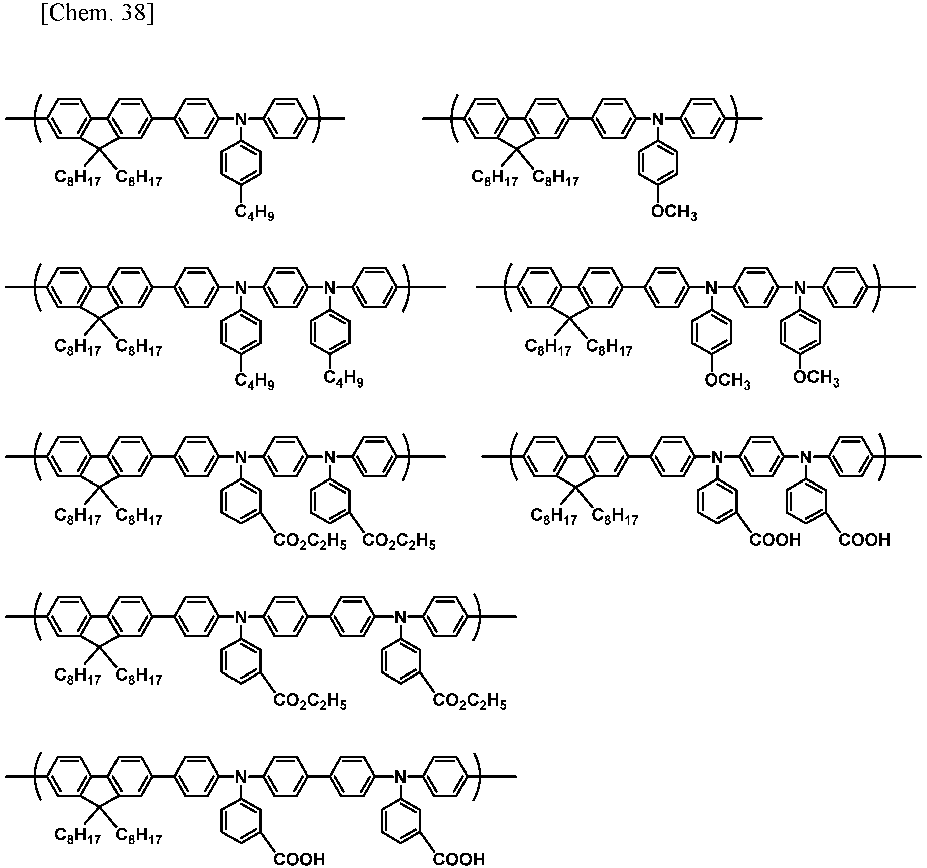 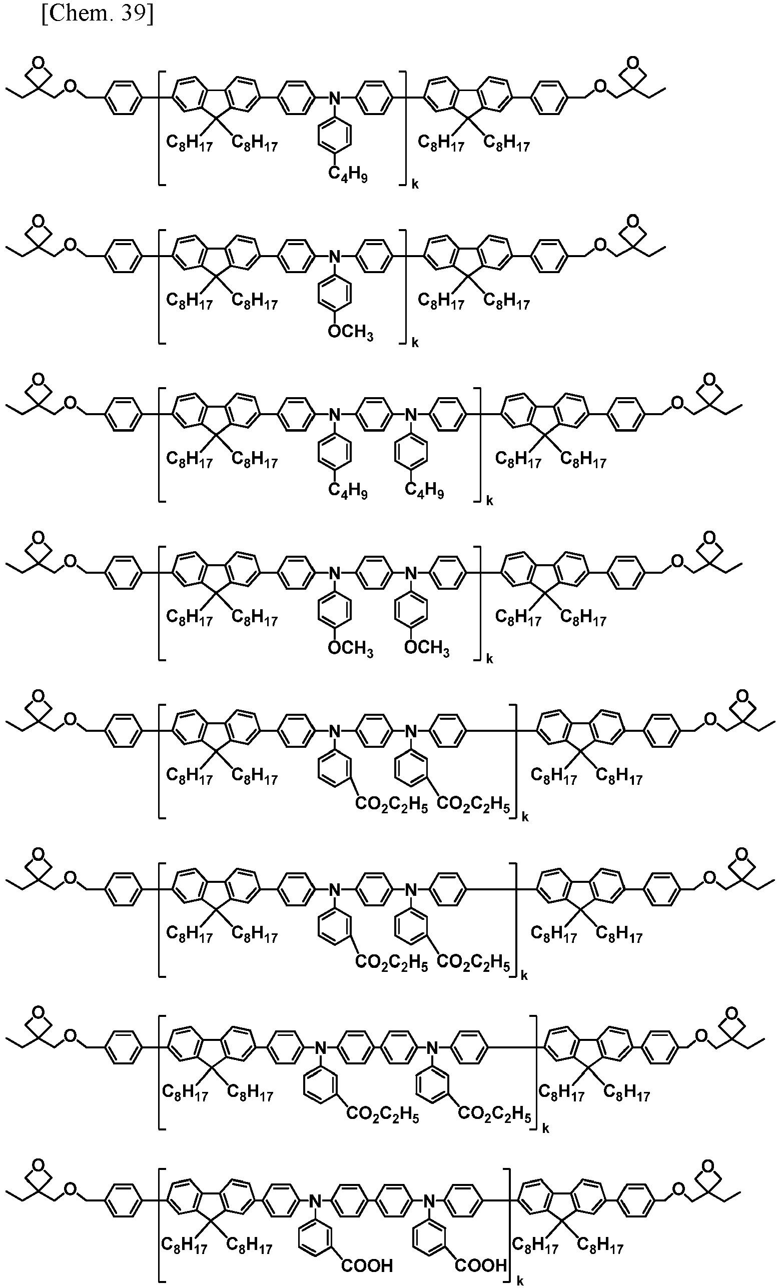 Herein, k is an integer of 1 or more.

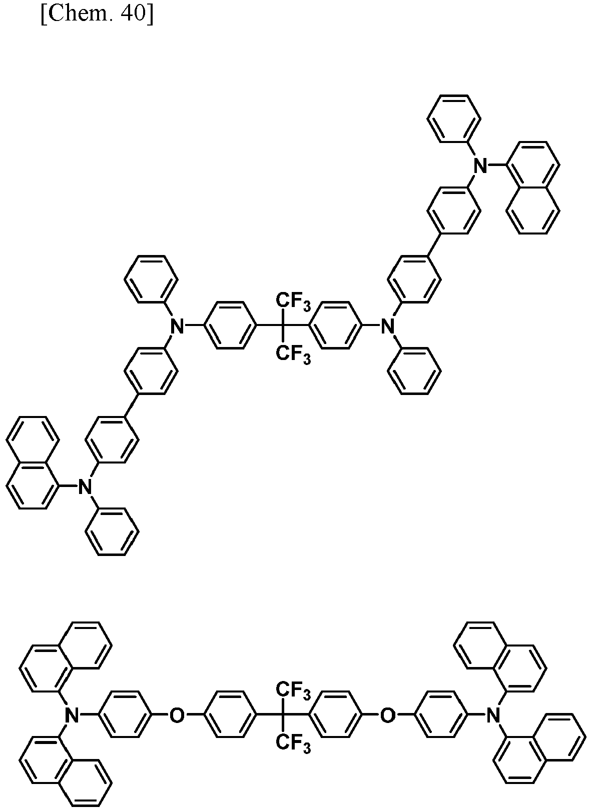

### [(C) Organic Solvent]

A high-solvency solvent capable of dissolving well the above aryl sulfonate ester compounds and the tertiary aryl amine compounds may be used as the organic solvent employed when preparing the charge-transporting varnish of the invention. It is preferable to use a low-polarity solvent for dissolving the tertiary aryl amine compound and obtaining an amorphous coating film. To dissolve an unesterified sulfonic acid compound, it is necessary that at least one highly polar solvent be included. By contrast, it is possible to dissolve the above aryl sulfonate ester compounds in a solvent regardless of the polarity of the solvent. In the invention, a low-polarity solvent is defined as a solvent having a dielectric constant at a frequency of 100 kHz that is less than 7, and a high-polarity solvent is defined as a solvent having a dielectric constant at a frequency of 100 kHz that is 7 or more.

Examples of low-polarity solvents include
chlorinated solvents such as chloroform and chlorobenzene;
aromatic hydrocarbon solvents such as toluene, xylene, tetralin, cyclohexylbenzene and decylbenzene;
aliphatic alcohol solvents such as 1-octanol, 1-nonanol and 1-decanol;
ether solvents such as tetrahydrofuran, dioxane, anisole, 4-methoxytoluene, 3-phenoxytoluene, dibenzyl ether, diethylene glycol dimethyl ether, diethylene glycol butyl methyl ether, triethylene glycol dimethyl ether and triethylene glycol butyl methyl ether; and
ester solvents such as methyl benzoate, ethyl benzoate, butyl benzoate, isoamyl benzoate, bis(2-ethylhexyl) phthalate, dibutyl maleate, dibutyl oxalate, hexyl acetate, diethylene glycol monoethyl ether acetate and diethylene glycol monobutyl ether acetate.

Examples of high-polarity solvents include
amide solvents such as N,N-dimethylformamide, N,N-dimethylacetamide, N,N-dimethylisobutyramide, N-methylpyrrolidone and 1,3-dimethyl-2-imidazolidinone;
ketone solvents such as ethyl methyl ketone, isophorone and cyclohexanone;
cyano solvents such as acetonitrile and 3-methoxypropionitrile;
polyhydric alcohol solvents such as ethylene glycol, diethylene glycol, triethylene glycol, dipropylene glycol, 1,3-butanediol and 2,3-butanediol;
monohydric alcohol solvents other than aliphatic alcohols, such as
diethylene glycol monomethyl ether, diethylene glycol monophenyl ether, triethylene glycol monomethyl ether, dipropylene glycol monomethyl ether, benzyl alcohol, 2-phenoxyethanol, 2-benzyloxyethanol, 3-phenoxybenzyl alcohol and tetrahydrofurfuryl alcohol; and
sulfoxide solvents such as dimethylsulfoxide.

Depending on the intended use, these solvents may be used singly, or in admixture of two or more thereof.

It is preferable for all the charge-transporting substances to be in a completely dissolved or uniformly dispersed state in the above solvent, and more preferable for them to be completely dissolved.

Examples of the method for preparing a charge-transporting varnish include, but are not limited to, a method in which component (A), component (B) and the like are added to a solvent in any order or at the same time. When there are a plurality of organic solvents, component (A), component (B) and the like may be dissolved in one solvent, followed by adding another solvent thereto, or component (A), component (B) and the like may be dissolved in a mixed solvent of a plurality of organic solvents in order or at the same time.

From the standpoint of reproducibly obtaining thin films having a higher flatness, it is desirable for component (A), component (B) and the like to be obtained by dissolving the charge-transporting varnish in the organic solvent and subsequently filtering the solution using a submicron-order filter or the like.

The solids concentration in the varnish of the invention, from the standpoint of ensuring a sufficient film thickness while minimizing deposition of the charge-transporting substance, is generally from about 0.1 to about 20% by weight, and preferably from 0.5 to 10% by weight. As used herein, the "solid" refers to the constituents which are contained in the varnish and which do not include solvents. The viscosity of the inventive varnish is generally from 1 to 50 mPa·s at 25°C.

The content of the electron-accepting substance precursor within these solids, expressed as a molar ratio with respect to unity (1) for the charge-transporting substance, is preferably from about 0.01 to about 20, and more preferably from about 0.05 to about 15.

The charge-transporting varnish of the present invention may further contain an organic silane compound. Examples of the organic silane compound include dialkoxysilane compounds, trialkoxysilane compounds and tetraalkoxysilane compounds. In particular, the organic silane compound is preferably a dialkoxysilane compound or a trialkoxysilane compound, and more preferably a trialkoxysilane compound. The organic silane compounds may be used singly, or in combination of two or more thereof.

The content of the organic silane compound is typically from about 0.1 to 50% by weight based on the total mass of the charge-transporting substance and the dopant. Taking into account the suppression of deterioration of charge transportability of the resulting thin film and the enhancement of the hole-injecting ability into layers laminated so as to contact the hole-injecting layer on a side opposite to the anode, such as a hole-transporting layer and a light-emitting layer, the content of the organic silane compound is preferably from about 0.5 to 40% by weight, more preferably from about 0.8 to 30% by weight, still more preferably from about 1 to 20% by weight.

### [Charge-Transporting Thin Film]

A charge-transporting thin film can be formed on a substrate by applying the charge-transporting varnish of the invention onto the substrate and drying the applied varnish.

Examples of methods for applying the varnish include, but are not limited to, dipping, spin coating, transfer printing, roll coating, brush coating, inkjet coating, spraying and slit coating. It is preferable for the viscosity and surface tension of the varnish to be adjusted according to the method of application.

When using the varnish of the invention, the liquid film drying conditions are not particularly limited; one example is heating and firing on a hot plate. A dry film can be obtained by heating and firing in a temperature range of generally from about 100 to about 260°C for a period of from about 1 minute to about 1 hour. The firing atmosphere is not particularly limited.

The thickness of the charge-transporting thin film is not particularly limited. However, when the thin film is to be used as a functional layer in an organic EL device, a film thickness of from 5 to 200 nm is preferred. Methods for changing the film thickness include, for example, changing the solids concentration in the varnish and changing the amount of solution on the substrate at the time of application.

### [Organic EL Device]

The organic EL device of the invention has a pair of electrodes and additionally has, between these electrodes, the above-described charge-transporting thin film of the invention.

Typical organic EL device configurations include, but are not limited to, configurations (a) to (f) below. In these configurations, where necessary, an electron-blocking layer or the like may be provided between the light-emitting layer and the anode, and a hole-blocking layer or the like may be provided between the light-emitting layer and the cathode. Alternatively, the hole-injecting layer, hole-transporting layer or hole-injecting-and-transporting layer may also have the function of an electron-blocking layer or the like; and the electron-injecting layer, electron-transporting layer or electron-injecting-and-transporting layer may also have the function of a hole-blocking layer or the like.
(a) anode/hole-injecting layer/hole-transporting layer/light-emitting layer/electron-transporting layer/electron-injecting layer/cathode
(b) anode/hole-injecting layer/hole-transporting layer/light-emitting layer/electron-injecting-and-transporting layer/cathode
(c) anode/hole-inj ecting-and-transporting layer/light-emitting layer/electron-transporting layer/electron-injecting layer/cathode
(d) anode/hole-inj ecting-and-transporting layer/light-emitting layer/electron-inj ecting-and-transporting layer/cathode
(e) anode/hole-injecting layer/hole-transporting layer/light-emitting layer/cathode
(f) anode/hole-injecting-and-transporting layer/light-emitting layer/cathode

As used herein, "hole-inj ecting layer," "hole-transporting layer" and "hole injecting-and-transporting layer" refer to layers which are formed between the light emitting layer and the anode and which have the function of transporting holes from the anode to the light-emitting layer. When only one layer of hole-transporting material is provided between the light-emitting layer and the anode, this is a "hole injecting and transporting layer"; when two or more layers of hole-transporting material are provided between the light-emitting layer and the anode, the layer that is closer to the anode is a "hole-injecting layer" and the other layer is a "hole-transporting layer." In particular, thin films having not only an excellent ability to accept holes from the anode but also an excellent ability to inject holes into, respectively, the hole-transporting layer and the light-emitting layer may be used as the hole-injecting layer and the hole injecting and-transporting layer.

The "electron-injecting layer," "electron-transporting layer" and "electron injecting-and-transporting layer" refer to layers which are formed between the light-emitting layer and the cathode and which have the function of transporting electrons from the cathode to the light-emitting layer. When only one layer of electron-transporting material is provided between the light-emitting layer and the cathode, this is an "electron injecting-and-transporting layer"; when two or more layers of electron transporting material are provided between the light-emitting layer and the cathode, the layer that is closer to the cathode is an "electron-injecting layer" and the other layer is an "electron-transporting layer."

The "light-emitting layer" is an organic layer having a light-emitting function. When a doping system is used, this layer includes a host material and a dopant material. The function of the host material is primarily to promote the recombination of electrons and holes and to confine the resulting excitons within the light-emitting layer. The function of the dopant material is to cause the excitons obtained by recombination to efficiently luminesce. In the case of phosphorescent devices, the host material functions primarily to confine within the light-emitting layer the excitons generated by the dopant.

The materials and method employed to produce an organic EL device using the charge-transporting varnish of the invention are exemplified by, but not limited to, those described below.

The electrode substrate to be used is preferably cleaned beforehand by liquid washing with, for example, a cleaning agent, alcohol or pure water. For example, when the electrode substrate is an anode substrate, it is preferably subjected to surface treatment such as UV/ozone treatment or oxygen-plasma treatment just prior to use. However, surface treatment need not be carried out in cases where the anode material contains an organic substance as a principal component.

An example is described below of a method for producing the organic EL device of the invention in which a thin-film obtained from the charge-transporting varnish of the invention serves as the hole-injecting layer.

Using the above-described method, a hole-injecting layer is formed on an electrode by applying the charge-transporting varnish of the invention onto an anode substrate and then firing the applied varnish. A hole-transporting layer, a light-emitting layer, an electron-transporting layer, an electron-injecting layer and a cathode are provided in this order on the hole-injecting layer. The hole-transporting layer, light-emitting layer, electron-transporting layer and electron-injecting layer may be formed by either a vapor deposition process or a coating process (wet process), depending on the properties of the material used.

Illustrative examples of anode materials include transparent electrodes such as indium-tin oxide (ITO) and indium-zinc oxide (IZO), and metal anodes made of a metal such as aluminum or an alloy of such a metal. An anode material on which planarizing treatment has been carried out is preferred. Use can also be made of polythiophene derivatives and polyaniline derivatives having a high charge transportability.

Examples of other metals that may make up the metal anode include, but are not limited to, scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, and alloys thereof.

Specific examples of hole-transporting layer-forming materials include the following hole-transporting low-molecular-weight materials: triarylamines such as (triphenylamine) dimer derivatives, [(triphenylamine) dimer] spirodimer, N,N'-bis(naphthalen-1-yl)-N,N'-bis(phenyl)benzidine (α-NPD), N,N'-bis(naphthalen-2-yl)-N,N'-bis(phenyl)benzidine, N,N'-bis(3-methylphenyl)-N,N'-bis(phenyl)benzidine, N,N'-bis(3-methylphenyl)-N,N'-bis(phenyl)-9,9-spirobifluorene, N,N'-bis(naphthalen-1-yl)-N,N'-bis(phenyl)-9,9-spirobifluorene, N,N'-bis(3-methylphenyl)-N,N'-bis(phenyl)-9,9-dimethylfluorene, N,N'-bis(naphthalen-1-yl)-N,N'-bis(phenyl)-9,9-dimethylfluorene, N,N'-bis(3-methylphenyl)-N,N'-bis(phenyl)-9,9-diphenylfluorene, N,N'-bis(naphthalen-1-yl)-N,N'-bis(phenyl)-9,9-diphenylfluorene, N,N'-bis(naphthalen-1-yl)-N,N'-bis(phenyl)-2,2'-dimethylbenzidine, 2,2',7,7'-tetrakis(N,N-diphenylamino)-9,9-spirobifluorene, 9,9-bis[4-(N,N-bis-biphenyl-4-ylamino)phenyl]-9H-fluorene, 9,9-bis[4-(N,N-bisnaphthalen-2-ylamino)phenyl]-9H-fluorene, 9,9-bis[4-(N-naphthalen-1-yl-N-phenylamino)phenyl]-9H-fluorene, 2,2',7,7'-tetrakis[N-naphthalenyl(phenyl)amino]-9,9-spirobifluorene, N,N'-bis(phenanthren-9-yl)-N,N'-bis(phenyl)benzidine, 2,2'-bis[N,N-bis(biphenyl-4-yl)amino]-9,9-spirobifluorene, 2,2'-bis(N,N-diphenylamino)-9,9-spirobifluorene, di[4-(N,N-di(p-tolyl)amino)phenyl]cyclohexane, 2,2',7,7' -tetra(N,N-di(p-tolyl))amino-9,9-spirobifluorene, N,N,N',N'-tetra-naphthalen-2-yl-benzidine, N,N,N',N'-tetra(3-methylphenyl)-3,3'-dimethylbenzidine, N,N'-di(naphthalenyl)-N,N'-di(naphthalen-2-yl)benzidine, N,N,N',N'-tetra(naphthalenyl)benzidine, N,N'-di(naphthalen-2-yl)-N,N'-diphenylbenzidine-1-4-diamine, N¹,N⁴-diphenyl-N¹,N⁴-di(m-tolyl)benzene-1,4-diamine, N²,N²,N⁶,N⁶-tetraphenylnaphthalene-2,6-diamine, tris(4-(quinolin-8-yl)phenyl)amine, 2,2'-bis(3-(N,N-di(p-tolyl)amino)phenyl)biphenyl, 4,4',4"-tris[3-methylphenyl(phenyl)amino]triphenylamine (m-MTDATA) and 4,4',4"-tris[1-naphthyl(phenyl)amino]triphenylamine (1-TNATA); and oligothiophenes such as 5,5"-bis-{4-[bis(4-methylphenyl)amino]phenyl}-2,2':5',2"-terthiophene (BMA-3T).

Specific examples of light-emitting layer-forming materials include tris(8-quinolinolate) aluminum(III) (Alq₃), bis(8-quinolinolate) zinc(II) (Znq₂), bis(2-methyl-8-quinolinolate)-4-(p-phenylphenolate) aluminum(III) (BAlq), 4,4'-bis(2,2-diphenylvinyl)biphenyl, 9,10-di(naphthalen-2-yl)anthracene, 2-tert-butyl-9,10-di(naphthalen-2-yl)anthracene, 2,7-bis[9,9-di(4-methylphenyl)-fluoren-2-yl]-9,9-di(4-methylphenyl)fluorene, 2-methyl-9,10-bis(naphthalen-2-yl)anthracene, 2-(9,9-spirobifluoren-2-yl)-9,9-spirobifluorene, 2,7-bis(9,9-spirobifluoren-2-yl)-9,9-spirobifluorene, 2-[9,9-di(4-methylphenyl)-fluoren-2-yl]-9,9-di(4-methylphenyl)fluorene, 2,2'-dipyrenyl-9,9-spirobifluorene, 1,3,5-tris(pyren-1-yl)benzene, 9,9-bis[4-(pyrenyl)phenyl]-9H-fluorene, 2,2'-bi(9,10-diphenylanthracene), 2,7-dipyrenyl-9,9-spirobifluorene, 1,4-di(pyren-1-yl)benzene, 1,3-di(pyren-1-yl)benzene, 6,13-di(biphenyl-4-yl)pentacene, 3,9-di(naphthalen-2-yl)perylene, 3,10-di(naphthalen-2-yl)perylene, tris[4-(pyrenyl)-phenyl]amine, 10,10' -di(biphenyl-4-yl)-9,9' -bianthracene, N,N'-di(naphthalen-1-yl)-N,N'-diphenyl-[1,1':4',1":4",1'"-quaterphenyl]-4,4"'-diamine, 4,4'-di[10-(naphthalen-1-yl)anthracen-9-yl]biphenyl, dibenzo{[f,f']-4,4',7,7'-tetraphenyl}diindeno[1,2,3-cd:1',2',3'-lm]perylene, 1-(7-(9,9'-bianthracen-10-yl)-9,9-dimethyl-9H-fluoren-2-yl)pyrene, 1-(7-(9,9'-bianthracen-10-yl)-9,9-dihexyl-9H-fluoren-2-yl)pyrene, 1,3-bis(carbazol-9-yl)benzene, 1,3,5-tris(carbazol-9-yl)benzene, 4,4',4"-tris(carbazol-9-yl)triphenylamine, 4,4'-bis(carbazol-9-yl)biphenyl (CBP), 4,4'-bis(carbazol-9-yl)-2,2'-dimethylbiphenyl, 2,7-bis(carbazol-9-yl)-9,9-dimethylfluorene, 2,2',7,7'-tetrakis(carbazol-9-yl)-9,9-spirobifluorene, 2,7-bis(carbazol-9-yl)-9,9-di(p-tolyl)fluorene, 9,9-bis[4-(carbazol-9-yl)-phenyl]fluorene, 2,7-bis(carbazol-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenylmethane, 2,7-bis(carbazol-9-yl)-9,9-dioctylfluorene, 4,4"-di(triphenylsilyl)-p-terphenyl, 4,4'-di(triphenylsilyl)biphenyl, 9-(4-tert-butylphenyl)-3,6-bis(triphenylsilyl)-9H-carbazole, 9-(4-tert-butylphenyl)-3,6-ditrityl-9H-carbazole, 9-(4-tert-butylphenyl)-3,6-bis(9-(4-methoxyphenyl)-9H-fluoren-9-yl)-9H-carbazole, 2,6-bis(3-(9H-carbazol-9-yl)phenyl)pyridine, triphenyl(4-(9-phenyl-9H-fluoren-9-yl)phenyl)silane, 9,9-dimethyl-N,N-diphenyl-7-(4-(1-phenyl-1H-benzo[d]imidazol-2-yl)phenyl-9H-fluoren-2-amine, 3,5-bis(3-(9H-carbazol-9-yl)phenyl)pyridine, 9,9-spirobifluoren-2-yl-diphenyl-phosphine oxide, 9,9'-(5-triphenylsilyl)-1,3-phenylene)bis(9H-carbazole), 3-(2,7-bis(diphenylphosphoryl)-9-phenyl-9H-fluoren-9-yl)-9-phenyl-9H-carbazole, 4,4,8,8,12,12-hexa(p-tolyl)-4H-8H-12H-12C-azadibenzo[cd,mn]pyrene, 4,7-di(9H-carbazol-9-yl)-1,10-phenanthroline, 2,2'-bis(4-(carbazol-9-yl)phenyl)biphenyl, 2,8-bis(diphenylphosphoryl)dibenzo[b,d]thiophene, bis(2-methylphenyl)diphenylsilane, bis[3,5-di(9H-carbazol-9-yl)phenyl]diphenylsilane, 3,6-bis(carbazol-9-yl)-9-(2-ethylhexyl)-9H-carbazole, 3-(diphenylphosphoryl)-9-(4-(diphenylphosphoryl)phenyl)-9H-carbazole and 3,6-bis[(3,5-diphenyl)phenyl]-9-phenylcarbazole. The light-emitting layer may be formed by the co-vapor deposition of these materials with a light-emitting dopant.

Specific examples of light-emitting dopants include 3-(2-benzothiazolyl)-7-(diethylamino)coumarin, 2,3,6,7-tetrahydro-1,1,7,7-tetramethyl-1H,5H,11H-10-(2-benzothiazolyl)quinolidino-[9,9a,1gh]coumarin, quinacridone, N,N'-dimethylquinacridone, tris(2-phenylpyridine)iridium(III) (Ir(ppy)₃), bis(2-phenylpyridine)(acetylacetonate) iridium(III) (Ir(ppy)₂(acac)), tris[2-(p-tolyl)pyridine]iridium(III) (Ir(mppy)₃), 9,10-bis[N,N-di(p-tolyl)amino]anthracene, 9,10-bis[phenyl(m-tolyl)amino]anthracene, bis[2-(2-hydroxyphenyl)benzothiazolate] zinc(II), N¹⁰,N¹⁰,N¹⁰,N¹⁰-tetra(p-tolyl)-9,9'-bianthracene-10,10'-diamine, N¹⁰,N¹⁰,N¹⁰,N¹⁰-tetraphenyl-9,9'-bianthracene-10,10'-diamine, N¹⁰,N¹⁰-diphenyl-N¹⁰,N¹⁰-dinaphthalenyl-9,9'-bianthracene-10,10'-diamine, 4,4'-bis(9-ethyl-3-carbazovinylene)-1,1'-biphenyl, perylene, 2,5,8,11-tetra-tert-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene, bis[3,5-difluoro-2-(2-pyridyl)phenyl-(2-carboxypyridyl)] iridium(III), 4,4'-bis[4-(diphenylamino)styryl]biphenyl, bis(2,4-difluorophenylpyridinato)tetrakis(1-pyrazolyl)borate iridium(III), N,N'-bis(naphthalen-2-yl)-N,N'-bis(phenyl)-tris(9,9-dimethylfluorenylene), 2,7-bis{2-[phenyl(m-tolyl)amino]-9,9-dimethylfluoren-7-yl}-9,9-dimethylfluorene, N-(4-((E)-2-(6((E)-4-(diphenylamino)styryl)naphthalen-2-yl)vinyl)phenyl)-N-phenylbenzenamine, fac-iridium(III) tris(1-phenyl-3-methylbenzimidazolin-2-ylidene-C,C²), mer-iridium(III) tris(1-phenyl-3-methylbenzimidazolin-2-ylidene-C,C²), 2,7-bis[4-(diphenylamino)styryl]-9,9-spirobifluorene, 6-methyl-2-(4-(9-(4-(6-methylbenzo[d]thiazol-2-yl)phenyl)anthracen-10-yl)phenyl)benzo-[d]thiazole, 1,4-di[4-(N,N-diphenyl)amino]styrylbenzene, 1,4-bis(4-(9H-carbazol-9-yl)styryl)benzene, (E)-6-(4-(diphenylamino)styryl)-N,N-diphenylnaphthalen-2-amine, bis(2,4-difluorophenylpyridinato)(5-(pyridin-2-yl)-1H-tetrazolate) iridium(III), bis(3-trifluoromethyl-5-(2-pyridyl)pyrazole)((2,4-difluorobenzyl)diphenylphosphinate) iridium(III), bis(3-trifluoromethyl-5-(2-pyridyl)pyrazolate)(benzyldiphenylphosphinate) iridium(III), bis(1-(2,4-difluorobenzyl)-3-methylbenzimidazolium)(3-(trifluoromethyl)-5-(2-pyridyl)-1,2,4-triazolate) iridium(III), bis(3-trifluoromethyl-5-(2-pyridyl)pyrazolate)(4',6'-difluorophenylpyridinate) iridium(III), bis(4',6'-difluorophenylpyridinato)(3,5-bis(trifluoromethyl)-2-(2'-pyridyl)pyrrolate) iridium(III), bis(4',6'-difluorophenylpyridinato)(3-(trifluoromethyl)-5-(2-pyridyl)-1,2,4-triazolate) iridium (III), (Z)-6-mesityl-N-(6-mesitylquinolin-2(1H)-ylidene)quinoline-2-amine-BF₂, (E)-2-(2-(4-(dimethylamino)styryl)-6-methyl-4H-pyran-4-ylidene)malononitrile, 4-(dicyanomethylene)-2-methyl-6-julolidyl-9-enyl-4H-pyran, 4-(dicyanomethylene)-2-methyl-6-(1,1,7,7-tetramethyljulolidyl-9-enyl)-4H-pyran, 4-(dicyanomethylene)-2-tert-butyl-6-(1,1,7,7-tetramethyljulolidin-4-ylvinyl)-4H-pyran, tris(dibenzoylmethane)phenanthroline europium(III), 5,6,11,12-tetraphenylnaphthacene, bis(2-benzo[b]thiophen-2-yl-pyridine)(acetylacetonate) iridium(III), tris(1-phenylisoquinoline) iridium(III), bis(1-phenylisoquinoline)(acetylacetonate) iridium(III), bis[1-(9,9-dimethyl-9H-fluoren-2-yl)isoquinoline](acetylacetonate) iridium(III), bis[2-(9,9-dimethyl-9H-fluoren-2-yl)quinoline](acetylacetonate) iridium(III), tris[4,4'-di-tert-butyl-(2,2')-bipyridine]ruthenium(III)·bis(hexafluorophosphate), tris(2-phenylquinoline) iridium(III), bis(2-phenylquinoline)(acetylacetonate) iridium(III), 2,8-di-tert-butyl-5,11-bis(4-tert-butylphenyl)-6,12-diphenyltetracene, bis(2-phenylbenzothiazolate)(acetylacetonate) iridium(III), platinum 5,10,15,20-tetraphenyltetrabenzoporphyrin, osmium(II) bis(3-trifluoromethyl-5-(2-pyridine)pyrazolate)dimethylphenylphosphine, osmium(II) bis(3-trifluoromethyl)-5-(4-tert-butylpyridyl)-1,2,4-triazolate)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolate)dimethylphenylphosphine, bis[2-(4-n-hexylphenyl)quinoline](acetylacetonate) iridium(III), tris[2-(4-n-hexylphenyl)quinoline]iridium(III), tris[2-phenyl-4-methylquinoline] iridium(III), bis(2-phenylquinoline)(2-(3-methylphenyl)pyridinate) iridium(III), bis(2-(9,9-diethylfluoren-2-yl)-1-phenyl-1H-benzo[d]imidazolato)(acetylacetonate) iridium(III), bis(2-phenylpyridine)(3-(pyridin-2-yl)-2H-chromen-2-onate) iridium(III), bis(2-phenylquinoline)(2,2,6,6-tetramethylheptane-3,5-dionate) iridium(III), bis(phenylisoquinoline)(2,2,6,6-tetramethylheptane-3,5-dionate) iridium(III), iridium(III) bis(4-phenylthieno[3,2-c]pyridinato-N,C²)acetylacetonate, (E)-2-(2-tert-butyl-6-(2-(2,6,6-trimethyl-2,4,5,6-tetrahydro-1H-pyrrolo[3,2,1-ij]quinolin-8-yl)vinyl)-4H-pyran-4-ylidene)malononitrile, bis(3-trifluoromethyl-5-(1-isoquinolyl)pyrazolate)(methyldiphenylphosphine) ruthenium, bis[(4-n-hexylphenyl)isoquinoline](acetylacetonate) iridium(III), platinum(II) octaethylporphin, bis(2-methyldibenzo[f,h]quinoxaline)(acetylacetonate) iridium(III) and tris[(4-n-hexylphenyl)isoquinoline] iridium(III).

Specific examples of electron-transporting layer-forming materials include lithium 8-hydroxyquinolinate, 2,2',2"-(1,3,5-benzinetriyl)-tris(1-phenyl-1-H-benzimidazole), 2-(4-biphenyl)-5-(4-tert-butylphenyl)-1,3,4-oxadiazole, 2,9-dimethyl-4,7-diphenyl-1,10-phenanthroline, 4,7-diphenyl-1,10-phenanthroline, bis(2-methyl-8-quinolinolate)-4-(phenylphenolato)aluminum, 1,3-bis[2-(2,2'-bipyridin-6-yl)-1,3,4-oxadiazo-5-yl]benzene, 6,6'-bis[5-(biphenyl-4-yl)-1,3,4-oxadiazo-2-yl]-2,2'-bipyridine, 3-(4-biphenyl)-4-phenyl-5-tert-butylphenyl-1,2,4-triazole, 4-(naphthalen-1-yl)-3,5-diphenyl-4H-1,2,4-triazole, 2,9-bis(naphthalen-2-yl)-4,7-diphenyl-1,10-phenanthroline, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, tris(2,4,6-trimethyl-3-(pyridin-3-yl)phenyl)borane, 1-methyl-2-(4-(naphthalen-2-yl)phenyl)-1H-imidazo[4,5f][1,10]phenanthroline, 2-(naphthalen-2-yl)-4,7-diphenyl-1,10-phenanthroline, phenyldipyrenylphosphine oxide, 3,3',5,5'-tetra[(m-pyridyl)-phen-3-yl]biphenyl, 1,3,5-tris[(3-pyridyl)-phen-3-yl]benzene, 4,4'-bis(4,6-diphenyl-1,3,5-triazin-2-yl)biphenyl, 1,3-bis[3,5-di(pyridin-3-yl)phenyl]benzene, bis(10-hydroxybenzo[h]quinolinato)beryllium, diphenylbis(4-(pyridin-3-yl)phenyl)silane and 3,5-di(pyren-1-yl)pyridine.

Examples of electron-injecting layer-forming materials include lithium oxide (Li₂O), magnesium oxide (MgO), alumina (Al₂O₃), lithium fluoride (LiF), sodium fluoride (NaF), magnesium fluoride (MgF₂), cesium fluoride (CsF), strontium fluoride (SrF₂), molybdenum trioxide (MoO₃), aluminum, lithium acetylacetonate (Li(acac)), lithium acetate and lithium benzoate.

Examples of cathode materials include aluminum, magnesium-silver alloys, aluminum-lithium alloys, lithium, sodium, potassium and cesium.

Another example is described below of a method for producing the organic EL device of the invention in which a thin-film obtained from the charge-transporting varnish of the invention serves as the hole-injecting layer.

An organic EL device having a charge-transporting thin film formed with the charge-transporting varnish of the invention can be produced by, in the organic EL device production method described above, successively forming a hole-transporting layer and a light-emitting layer instead of carrying out vacuum evaporation operations for a hole transporting layer, a light-emitting layer, an electron-transporting layer and an electron-injecting layer. Specifically, the charge-transporting varnish of the invention is applied onto an anode substrate, and a hole-injecting layer is formed by the above described method. A hole-transporting layer and a light-emitting layer are then successively formed thereon, following which a cathode material is vapor-deposited on top, thereby giving an organic EL device.

The cathode and anode materials used here may be similar to those described above, and similar cleaning treatment and surface treatment may be carried out.

The method of forming the hole-transporting layer and the light-emitting layer is exemplified by a film-forming method that involves adding a solvent to a hole-transporting polymer material or a light-emitting polymer material, or to the material obtained by adding a dopant to either of these, thereby dissolving or uniformly dispersing the material, and then applying the solution or dispersion onto the hole-injecting layer or the hole-transporting layer and subsequently firing.

Examples of hole-transporting polymer materials include poly[(9,9-dihexylfluorenyl-2,7-diyl)-co-(N,N'-bis{p-butylphenyl}-1,4-diaminophenylene)], poly[(9,9-dioctylfluorenyl-2,7-diyl)-co-(N,N'-bis{p-butylphenyl}-1,1'-biphenylene-4,4-diamine)], poly[(9,9-bis{1'-penten-5'-yl}fluorenyl-2,7-diyl)-co-(N,N'-bis{p-butylphenyl}-1,4-diaminophenylene)], poly[N,N'-bis(4-butylphenyl)-N,N'-bis(phenyl)-benzidine] end-capped with polysilsesquioxane and poly[(9,9-dioctylfluorenyl-2,7-diyl)-co-(4,4'-(N-(p-butylphenyl))diphenylamine)] (TFB).

Examples of light-emitting polymer materials include polyfluorene derivatives such as poly(9,9-dialkylfluorene) (PDAF), poly(phenylene vinylene) derivatives such as poly(2-methoxy-5-(2'-ethylhexoxy)-1,4-phenylene vinylene) (MEH-PPV), polythiophene derivatives such as poly(3-alkylthiophene) (PAT), and polyvinylcarbazole (PVCz).

Examples of solvents include toluene, xylene and chloroform. Examples of the method of dissolution or uniform dispersion include stirring, stirring under applied heat, and ultrasonic dispersion.

Examples of the method of application include, but are not particularly limited to, inkjet coating, spraying, dipping, spin coating, transfer printing, roll coating and brush coating. Application is preferably carried out in an inert gas atmosphere such as nitrogen or argon.

Examples of the firing method include methods that involve heating in an oven or on a hot plate, either within an inert gas atmosphere or in a vacuum.

An example is described below of a method for producing the organic EL device of the invention in cases where a thin film obtained from the charge-transporting varnish of the invention serves as a hole-injecting-and-transporting layer.

A hole-injecting-and-transporting layer is formed on an anode substrate. A light emitting layer, an electron-transporting layer, an electron-injecting layer and a cathode are provided in this order on the hole-injecting-and-transporting layer. Methods of forming the light-emitting layer, electron-transporting layer and electron-injecting layer, and specific examples thereof, include the same as those mentioned above.

The anode material, the light-emitting layer, the light-emitting dopant, the materials which form the electron-transporting layer and the electron-blocking layer, and the cathode material are exemplified in the same way as mentioned above.

A hole-blocking layer, an electron-blocking layer or the like may be optionally provided between the electrodes and any of the above layers. By way of illustration, an example of a material that forms an electron-blocking layer is tris(phenylpyrazole)iridium.

The materials which make up the anode, the cathode and the layers formed therebetween differ according to whether a device provided with a bottom emission structure or a top emission structure is to be fabricated, and so are suitably selected while taking this into account.

Typically, in a device having a bottom emission structure, a transparent anode is used on the substrate side and light is extracted from the substrate side, whereas in a device having a top emission structure, a reflective anode made of metal is used and light is extracted from the transparent electrode (cathode) side in the opposite direction from the substrate. Hence, for example, with regard to the anode material, when fabricating a device having a bottom emission structure, a transparent anode of ITO or the like is used, and when fabricating a device having a top emission structure, a reflective anode of Al/Nd or the like is used.

To prevent deterioration of the device characteristics, the organic EL device of the invention may be sealed in the usual manner with, if necessary, a desiccant or the like.

### EXAMPLES

Examples and Comparative Examples are given below to more concretely illustrate the invention, although the invention is not limited by these Examples. In the Examples, the following equipment was used for sample preparation and for analyzing physical properties.

| | |
|---|---|
| (1) ¹H-NMR, ¹⁹F-NMR: | Nuclear magnetic resonance apparatus AL-300, from JEOL Ltd. |
| (2) LC/MS: | ZQ 2000, from Waters Corporation |
| (3) Substrate Cleaning: | Substrate cleaning machine (reduced-pressure plasma system), from Choshu Industry Co., Ltd. |
| (4) Varnish Coating: | MS-A100 Spin Coater, from Mikasa Co., Ltd. |
| (5) Film Thickness Measurement: | Surfcorder ET-4000 microfigure measuring instrument, from Kosaka Laboratory, Ltd. |
| (6) EL Device Fabrication: | C-E2L1G1-N Multifunction Vapor Deposition System, from Choshu Industry Co., Ltd. |
| (7) Measurement of luminance of ELelement, etc.: | Multi-channel IVL measurement apparatus, from EHC Co., Ltd. |

### [1] Synthesis of Sulfonic Acid Ester Compound

### [Synthesis Example 1-1] Synthesis of NSO-2-PGEE-1

The aryl sulfonate ester compound NSO-2-PGEE was synthesized in accordance with the following scheme.

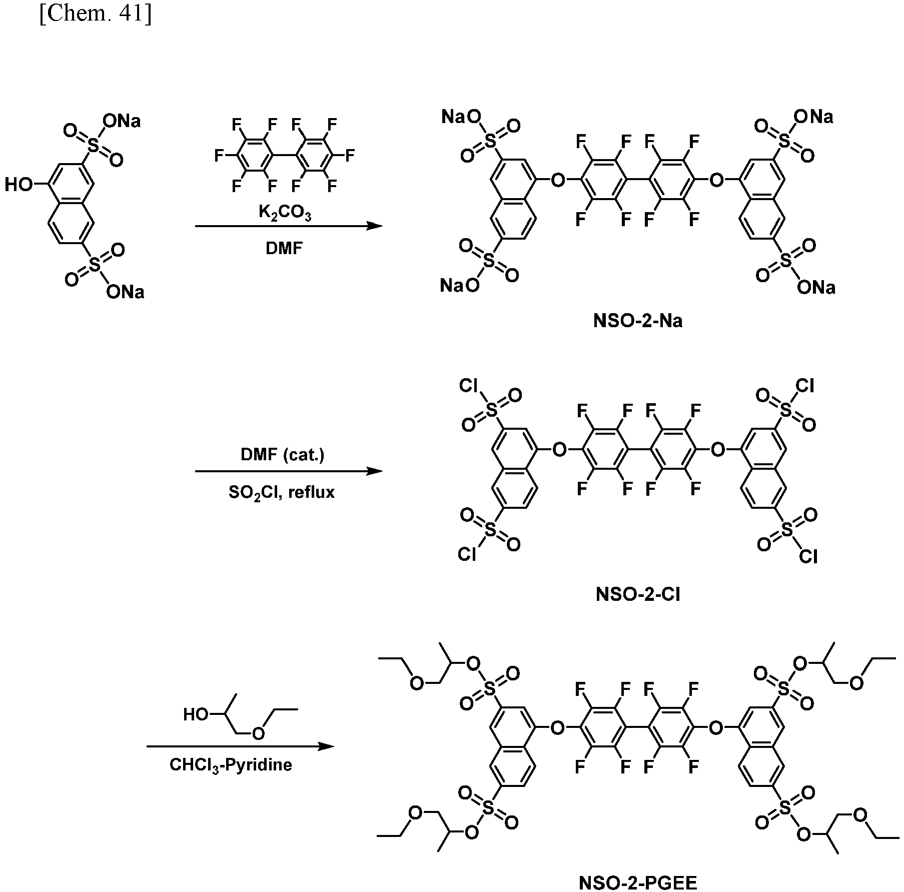

Under a nitrogen atmosphere, 4.8 g (14.36 mol) of perfluorobiphenyl, 4.2 g (30.15 mol) of potassium carbonate and 100 mL of N,N-dimethylformamide were successively added to 11 g (31.59 mmol) of sodium 1-naphthol-3,6-disulfonate, and the reaction system was flushed with nitrogen and subsequently stirred for 6 hours at an internal temperature of 100°C. The system was allowed to cool to room temperature, following which the potassium carbonate residue was removed by filtration and vacuum concentration was carried out. To remove the remaining impurities, 100 mL of methanol was added to the residue and stirring was carried out for 30 minutes at room temperature. The suspension was then filtered, giving 11.8 g (yield: 83%) of NSO-2-Na.

Thionyl chloride (8 mL) and N,N-dimethylformamide (DMF) (0.1 mL) as a catalyst were added to 2 g (2 mmol) of NSO-2-Na, and the system was refluxed under heating for 1 hour, following which the thionyl chloride was driven off, giving a solid containing NSO-2-Cl. This compound was used in the next step without further purification.

Chloroform (12 mL) and pyridine (8 mL) were added to the solid, and 2.50 g (24 mmol) of propylene glycol monoethyl ether (Junsei Chemical Co., Ltd.) was added at 0°C. The temperature was raised to room temperature and 3 hours of stirring was carried out thereafter. The solvent was driven off, following which water was added, extraction was carried out with ethyl acetate, and the organic layer was dried over sodium sulfate. After filtration and concentration, the resulting crude product was purified by silica gel column chromatography (hexane/ethyl acetate), giving 1.09 g of the aryl sulfonate ester compound NSO-2-PGEE (hereinafter, referred to as NSO-2-PGEE-1) as a white solid (yield: 44% (2-step yield from NSO-2-Na)). The results of ¹H-NMR and LC/MS measurement are shown below.
¹H-NMR (500 MHz, CDCl₃):
δ 0.92-0.97 (m, 12H), 1.34 and 1.40 (a pair of d, J = 6.5 Hz, 12H), 3.32-3.52 (m, 16H), 4.80-4.87 (m, 4H), 7.37 (s, 2H), 8.22 (d, J = 8.5 Hz, 2H), 8.45 (s, 2H),
8.61 (d, J = 8.5 Hz, 2H), 8.69 (s, 2H).
LC/MS (ESI⁺) m/z; 1264 [M+NH₄]⁺

### [Synthesis Example 1-2] Synthesis of NSO-2-PGEE-2

The sulfonic acid ester compound NSO-2-PGEE was synthesized in accordance with the following scheme.

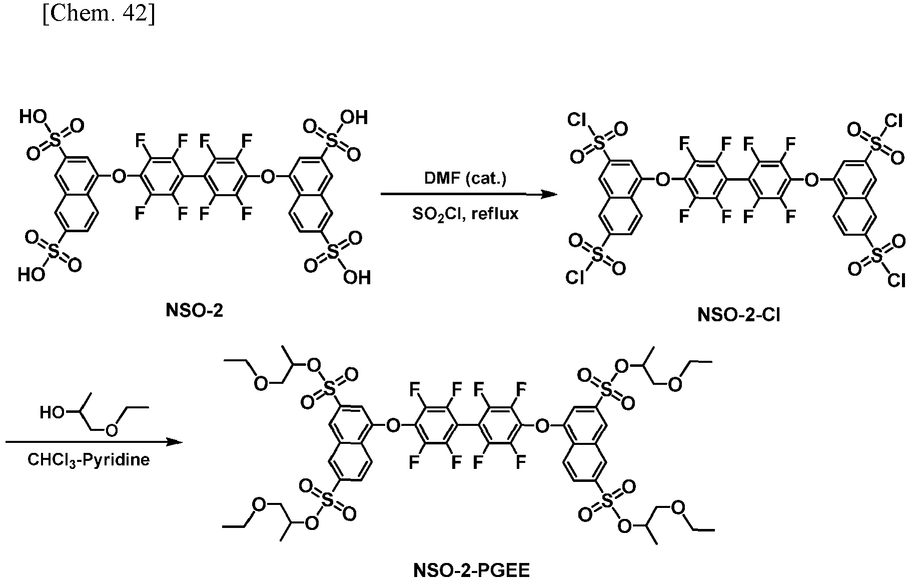

Thionyl chloride (8 mL) and DMF (85 µL) as a catalyst were added to 2 g (2.2 mmol) of NSO-2 synthesized in accordance with the method described in WO 2006/025342, and the system was refluxed under heating for 1 hour, following which the thionyl chloride was driven off, giving a solid containing NSO-2-Cl. This compound was used in the next step without further purification. Chloroform (12 mL) and pyridine (8 mL) were added to the solid, and 2.75 g (26.4 mmol) of propylene glycol monoethyl ether (Junsei Chemical Co., Ltd.) was added at 0°C. The temperature was raised to room temperature and 3 hours of stirring was carried out thereafter. The solvent was driven off, following which water was added, extraction was carried out with ethyl acetate, and the organic layer was dried over sodium sulfate. After filtration and concentration, the resulting crude product was purified by silica gel column chromatography (hexane/ethyl acetate), giving 1.50 g of the sulfonic acid ester compound NSO-2-PGEE (hereinafter, referred to as NSO-2-PGEE-2) as a white solid (yield: 54% (2-step yield from NSO-2)). The results of ¹H-NMR and LC/MS measurement are shown below.
¹H-NMR (500 MHz, CDCl₃):
δ 0.92-0.97 (m, 12H), 1.34 and 1.40 (a pair of d, J = 6.5 Hz, 12H), 3.32-3.52 (m, 16H), 4.80-4.87 (m, 4H), 7.37 (s, 2H), 8.22 (d, J = 8.5 Hz, 2H), 8.45 (s, 2H),
8.61 (d, J = 8.5 Hz, 2H), 8.69 (s, 2H).
LC/MS (ESI⁺) m/z; 1264 [M+NH₄]⁺

### [Synthesis Example 2] Synthesis of 4FNS-4-PGEE

The sulfonic acid ester compound 4FNS-4-PGEE was synthesized in accordance with the following scheme.

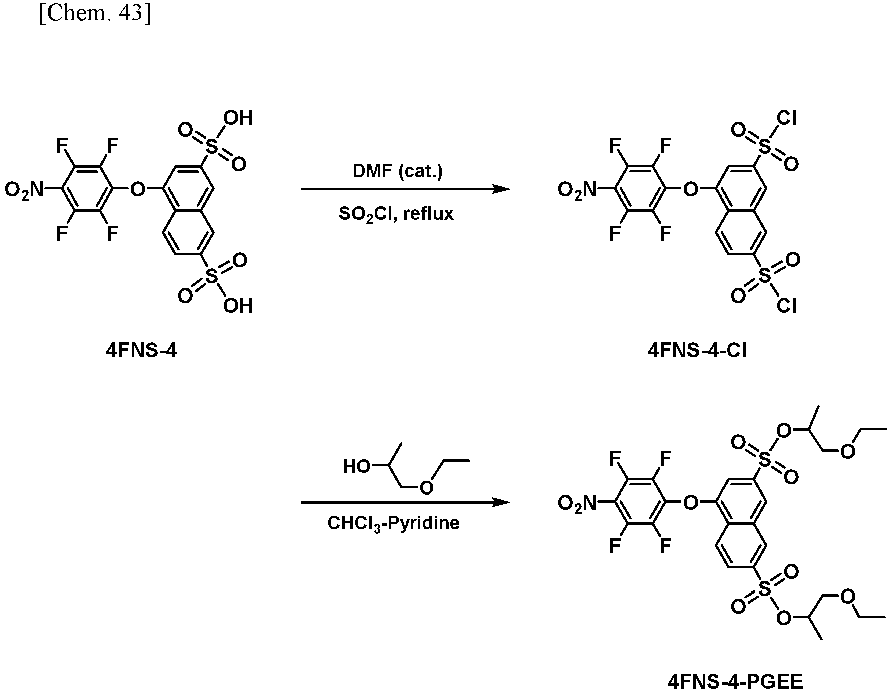

25 g of thionyl chloride and 0.4 mL of DMF as a catalyst were added to 4.97 g (10 mmol) of 4FNS-4 synthesized in accordance with the method described in WO 2015/111654, and the system was refluxed under heating for 1 hour, following which the thionyl chloride was driven off, giving a solid containing 4FNS-4-Cl. This compound was used in the next step without further purification.

Chloroform (30 mL) and pyridine (20 mL) were added to the solid, and 6.24 g (60 mmol) of propylene glycol monoethyl ether was added at 0°C. The temperature was raised to room temperature and 1.5 hours of stirring was carried out thereafter. The solvent was driven off, following which water was added, extraction was carried out with ethyl acetate, and the organic layer was dried over sodium sulfate. After filtration and concentration, the resulting crude product was purified by silica gel column chromatography (hexane/ethyl acetate), giving 1.32 g of the sulfonic acid ester compound 4FNS-4-PGEE as a white solid (yield: 20% (2-step yield from 4FNS-4)). The results of ¹H-NMR and LC/MS measurement are shown below.
¹H-NMR (500 MHz, CDCl₃):
δ 0.89-0.95 (m, 6H), 1.34 and 1.39 (a pair of d, J = 6.5 Hz, 6H), 3.28-3.50 (m, 8H), 4.81-4.87 (m, 2H), 7.26 (s, 1H), 8.22 (d, J = 9.0 Hz, 1H), 8.47 (s, 1H),
8.54 (d, J = 9.0 Hz, 1H), 8.68 (s, 1H).
LC/MS (ESI⁺) m/z; 687 [M+NH₄]⁺

### [2] Preparation of Charge-Transporting Varnish

### [Example 1-1]

Chloroform (5 g) was added to a mixture of NSO-2-PGEE-1 (12.5 mg) and compound H1 (12.5 mg) of the following formula (H1) which had been synthesized in accordance with the method described WO 2005/094133, and the system was dissolved by performing stirring at room temperature, the resulting solution was filtered by a syringe filter having a pore size of 0.2 µm, giving charge-transporting varnish A1.

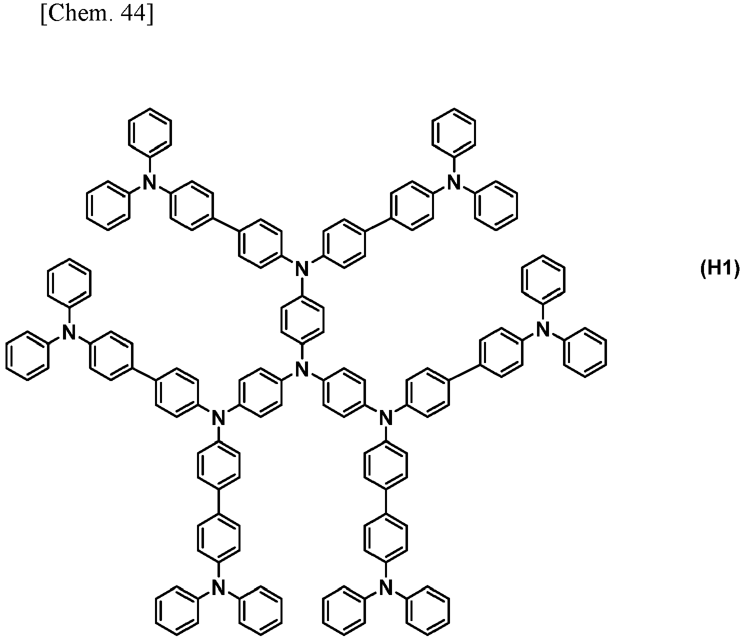

### [Comparative Example 1-1]

Chloroform (5 g) was added to compound H1 (25 mg), the system was dissolved by performing stirring at room temperature, and the resulting solution was filtered by a syringe filter having a pore size of 0.2 µm, giving charge-transporting varnish A2.

### [Example 1-2]

Chloroform (5 g) was added to a mixture of NSO-2-PGEE-1 (12.5 mg) and compound H2 (12.5 mg) of the following formula (H2) which had been synthesized in accordance with the method described WO 2013/098175, and the system was dissolved by performing stirring at room temperature, the resulting solution was filtered by a syringe filter having a pore size of 0.2 µm, giving charge-transporting varnish B1.

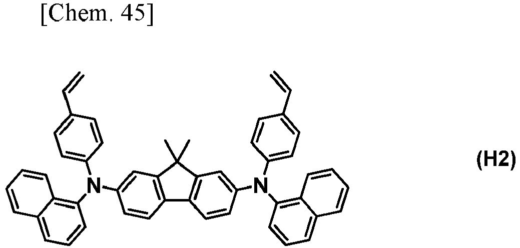

### [Comparative Example 1-2]

Chloroform (5 g) was added to compound H2 (25 mg), the system was dissolved by performing stirring at room temperature, and the resulting solution was filtered by a syringe filter having a pore size of 0.2 µm, giving charge-transporting varnish B2.

### [Example 1-3]

Chloroform (5 g) was added to a mixture of compound H3 (TFB Polymer, from Luminescence Technology Corp., LT-N148) (12.5 mg) of the following formula (H3) and NSO-2-PGEE-1 (12.5 mg), and the system was dissolved by performing stirring at room temperature, the resulting solution was filtered by a syringe filter having a pore size of 0.2 µm, giving charge-transporting varnish C1.

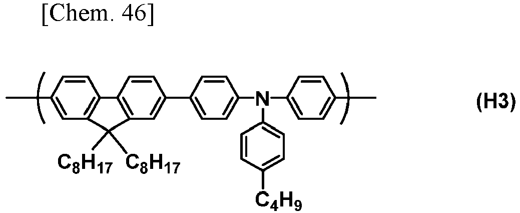

### [Comparative Example 1-3]

Chloroform (5 g) was added to compound H3 (25 mg), the system was dissolved by performing stirring at room temperature, and the resulting solution was filtered by a syringe filter having a pore size of 0.2 µm, giving charge-transporting varnish C2.

### [Comparative Example 1-4]

Chloroform (5g) was added to a mixture of compound H1 (12.5 mg) and NSO-2 (12.5 mg), and the system was stirred at room temperature at 350 rpm for 60 minutes. NSO-2 was not dissolved.

### [Comparative Example 1-5]

Chloroform (5g) was added to a mixture of compound H2 (12.5 mg) and NSO-2 (12.5 mg), and the system was stirred at room temperature at 350 rpm for 60 minutes. NSO-2 was not dissolved.

### [Comparative Example 1-6]

Chloroform (5g) was added to a mixture of compound H3 (12.5 mg) and NSO-2 (12.5 mg), and the system was stirred at room temperature at 350 rpm for 60 minutes. NSO-2 was not dissolved.

### [Example 1-4]

Compound H4 (190 mg) of the following formula (H4) which had been synthesized in accordance with the method described in WO 2015/050253, Synthesis Example 18, and NSO-2-PGEE-2 (337 mg) were added to a mixed solvent of 3-phenoxytoluene (5 g) and tetralin (5 g), and the system was stirred for 5 minutes under heating at 50°C and 400 rpm. As a result, the NSO-2-PGEE-2 was dissolved completely in the solvent. The resulting solution was filtered using a PTFE filter having a pore size of 0.2 µm, giving charge-transporting varnish D.

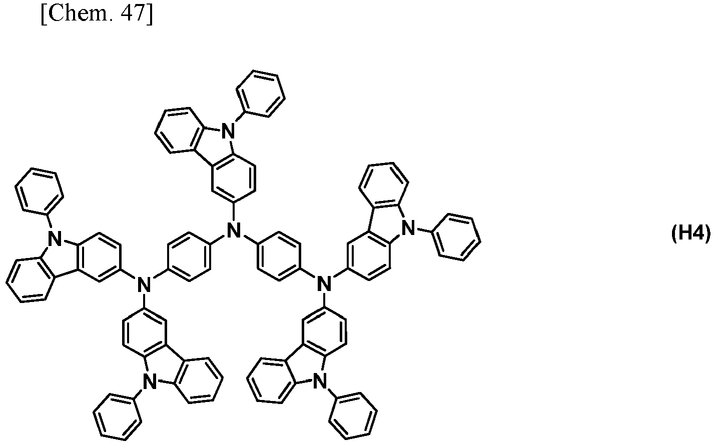

### [Example 1-5]

Compound H4 (48 mg), compound H5 (149 mg) of the following formula (H5) which had been synthesized in accordance with the method described in WO 2015/050253, Production Example 24-2, and NSO-2-PGEE-2 (329 mg) were added to a mixed solvent of 3-phenoxytoluene (5 g) and tetralin (5 g), and the system was stirred for 5 minutes under heating at 50°C and 400 rpm. As a result, the NSO-2-PGEE-2 was dissolved completely in the solvent. The resulting solution was filtered using a PTFE filter having a pore size of 0.2 µm, giving charge-transporting varnish E.

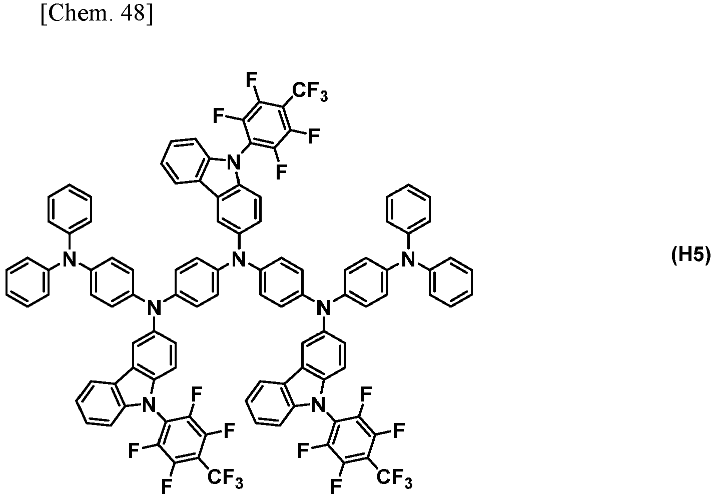

### [Example 1-6]

Compound H4 (190 mg) and NSO-2-PGEE-2 (337 mg) were added to a mixed solvent of triethylene glycol butyl methyl ether (7 g) and butyl benzoate (3 g), and the system was stirred for 5 minutes under heating at 50°C and 400 rpm. As a result, the NSO-2-PGEE-2 was dissolved completely in the solvent. The resulting solution was filtered using a PTFE filter having a pore size of 0.2 µm, giving charge-transporting varnish F.

### [Example 1-7]

Compound H4 (48 mg), compound H5 (149 mg) and NSO-2-PGEE-2 (329 mg) were added to a mixed solvent of triethylene glycol butyl methyl ether (7 g) and butyl benzoate (3 g), and the system was stirred for 5 minutes under heating at 50°C and 400 rpm. As a result, the NSO-2-PGEE-2 was dissolved completely in the solvent. The resulting solution was filtered using a PTFE filter having a pore size of 0.2 µm, giving charge-transporting varnish G.

### [Example 1-8]

Compound H4 (190 mg) and NSO-2-PGEE-2 (337 mg) were added to a mixed solvent of 4-methoxytoluene (7 g) and cyclohexylbenzene (3 g), and the system was stirred for 5 minutes under heating at 50°C and 400 rpm. As a result, the NSO-2-PGEE-2 was dissolved completely in the solvent. The resulting solution was filtered using a PTFE filter having a pore size of 0.2 µm, giving charge-transporting varnish H.

### [Example 1-9]

Compound H4 (48 mg), compound H5 (149 mg) and NSO-2-PGEE-2 (329 mg) were added to a mixed solvent of 4-methoxytoluene (7 g) and cyclohexylbenzene (3 g), and the system was stirred for 5 minutes under heating at 5°C and 400 rpm. As a result, the NSO-2-PGEE-2 was dissolved completely in the solvent. The resulting solution was filtered using a PTFE filter having a pore size of 0.2 µm, giving charge-transporting varnish I.

### [Example 1-10]

Compound H4 (190 mg) and NSO-2-PGEE-2 (337 mg) were added to a mixed solvent of ethyl benzoate (7 g) and dibenzyl ether (3 g), and the system was stirred for 5 minutes under heating at 50°C and 400 rpm. As a result, the NSO-2-PGEE-2 was dissolved completely in the solvent. The resulting solution was filtered using a PTFE filter having a pore size of 0.2 µm, giving charge-transporting varnish J.

### [Example 1-11]

Compound H4 (48 mg), compound H5 (149 mg) and NSO-2-PGEE-2 (329 mg) were added to a mixed solvent of ethyl benzoate (7 g) and dibenzyl ether (3 g), and the system was stirred for 5 minutes under heating at 50°C and 400 rpm. As a result, the NSO-2-PGEE-2 was dissolved completely in the solvent. The resulting solution was filtered using a PTFE filter having a pore size of 0.2 µm, giving charge-transporting varnish K.

### [Example 1-12]

Compound H4 (270 mg) and 4FNS-4-PGEE (257 mg) were added to a mixed solvent of 3-phenoxytoluene (3 g) and butyl benzoate (7 g), and the system was stirred for 10 minutes under heating at 50°C and 350 rpm. As a result, the 4FNS-4-PGEE was dissolved completely in the solvent. The resulting solution was filtered using a PTFE filter having a pore size of 0.2 µm, giving charge-transporting varnish L.

### [Example 1-13]

Compound H4 (105 mg) and 4FNS-4-PGEE (100 mg) were added to a mixed solvent of diethylene glycol (4 g; dielectric constant: 25.2) and triethylene glycol dimethyl ether (6 g; dielectric constant: 5.1), and the system was stirred for 10 minutes under heating at 50°C and 350 rpm. As a result, the 4FNS-4-PGEE was dissolved completely in the solvent. The resulting solution was filtered using a PTFE filter having a pore size of 0.2 µm, giving charge-transporting varnish M.

### [Comparative Example 1-7]

Compound H4 (120 mg) and 4FNS-4 (85 mg) were added to a mixed solvent of diethylene glycol (4 g) and triethylene glycol dimethyl ether (6 g), and the system was stirred for 10 minutes under heating at 50°C and 350 rpm. As a result, the 4FNS-4 was dissolved completely in the solvent. The resulting solution was filtered using a PTFE filter having a pore size of 0.2 µm, giving charge-transporting varnish N.

### [Example 1-14]

Chloroform (5 g) was added to a mixture of compound H3 (25 mg) and methyl p-toluenesulfonate (Tokyo Chemical Industry Co., Ltd.) (38 mg), and the system was dissolved by performing stirring at room temperature, the resulting solution was filtered by a syringe filter having a pore size of 0.2 µm, giving charge-transporting varnish O.

### [Example 1-15]

Chloroform (5 g) was added to a mixture of compound H3 (25 mg) and ethyl benzenesulfonate (Tokyo Chemical Industry Co., Ltd.) (38 mg), and the system was dissolved by performing stirring at room temperature, the resulting solution was filtered by a syringe filter having a pore size of 0.2 µm, giving charge-transporting varnish P.

### [3] Single-Layer Device and Evaluation of Device Characteristics

In the following Working Examples and Comparative Examples, a glass substrate with dimensions of 25 mm × 25 mm × 0.7 t and having ITO patterned on the surface to a film thickness of 150 nm was used as the ITO substrate. Prior to use, impurities on the surface were removed with an O₂ plasma cleaning system (150 W, 30 seconds).

### [Example 2-1]

Charge-transporting varnish A1 was applied onto the ITO substrate using a spin coater, and then subjected to firing at 230°C for 15 minutes, thereby forming a 60-nm thin film on the ITO substrate.

Using a vapor deposition system (degree of vacuum, 2.0×10⁻⁵ Pa), thin films of aluminum were deposited thereon, giving a single-layer device. Vapor deposition was carried out at a deposition rate of 0.2 nm/s. The thicknesses of the aluminum thin film was set to 80 nm.

To prevent the device characteristics from deteriorating due to the influence of oxygen, moisture and the like in air, the single-layer device was sealed with sealing substrates, following which the characteristics were evaluated. Sealing was carried out by the following procedure.

The single-layer device was placed between sealing substrates in a nitrogen atmosphere having an oxygen concentration of 2 ppm or less and a dew point of not more than -85°C, and the sealing substrates were laminated together using an adhesive (MORESCO Moisture Cut WB90US(P), from Moresco Corporation). At this time, a desiccant (HD-071010W-40, from Dynic Corporation) was placed, together with the single-layer device, within the sealing substrates. The laminated sealing substrates were irradiated with an ultraviolet ray (wavelength, 365 nm; dosage, 6,000 mJ/cm²) and then annealed at 80°C for 1 hour to cure the adhesive.

### [Comparative Example 2-1]

Except that charge-transporting varnish A2 was used instead of charge-transporting varnish A1, the same procedure as in Example 2-1 was carried out to fabricate a single-layer device.

### [Example 2-2]

Except that charge-transporting varnish B1 was used instead of charge-transporting varnish A1, the same procedure as in Example 2-1 was carried out to fabricate a single-layer device.

### [Comparative Example 2-2]

Except that charge-transporting varnish B2 was used instead of charge-transporting varnish A1, the same procedure as in Example 2-1 was carried out to fabricate a single-layer device.

### [Example 2-3]

Except that charge-transporting varnish C1 was used instead of charge-transporting varnish A1, the same procedure as in Example 2-1 was carried out to fabricate a single-layer device.

### [Comparative Example 2-3]

Except that charge-transporting varnish C2 was used instead of charge-transporting varnish A1, the same procedure as in Example 2-1 was carried out to fabricate a single-layer device.

The current densities at a driving voltage of 5 V were measured for the single-layer devices fabricated in Examples 2-1 to 2-3 and Comparative Examples 2-1 to 2-3. The results are shown in Table 1.

**[Table 1]**

| | Charge-transporting varnish | Current density (mA/cm²) |
|---|---|---|
| Example 2-1 | A1 | 3,250 |
| Comparative Example 2-1 | A2 | 321 |
| Example 2-2 | B1 | 2,706 |
| Comparative Example 2-2 | B2 | 293 |
| Example 2-3 | C1 | 408 |
| Comparative Example 2-3 | C2 | 14 |

### [4] Fabrication of Hole-Only Devices (HOD) and Evaluation of Device Characteristics-1

The ITO substrate used in the following Examples and Comparative Examples was the same as that described above.

### [Example 3-1]

Charge-transporting varnish A1 was applied onto the ITO substrate using a spin coater, and then subjected to firing at 230°C for 15 minutes, thereby forming a 60-nm thin film on the ITO substrate.

Using a vapor deposition system (degree of vacuum, 2.0 × 10⁻⁵ Pa), thin films of α-NPD and aluminum were successively deposited thereon, giving a hole-only device. Vapor deposition was carried out at a deposition rate of 0.2 nm/s. The thicknesses of the α-NPD thin film and the aluminum thin film were set to respectively 30 nm and 80 nm.

To prevent the device characteristics from deteriorating due to the influence of oxygen, moisture and the like in air, the hole-only device was sealed with sealing substrates, following which the characteristics were evaluated. Sealing was carried out by the following procedure.

The hole-only device was placed between sealing substrates in a nitrogen atmosphere having an oxygen concentration of 2 ppm or less and a dew point of not more than -85°C, and the sealing substrates were laminated together using an adhesive (MORESCO Moisture Cut WB90US(P), from Moresco Corporation). At this time, a desiccant (HD-071010W-40, from Dynic Corporation) was placed, together with the hole-only device, within the sealing substrates. The laminated sealing substrates were irradiated with an ultraviolet ray (wavelength, 365 nm; dosage, 6,000 mJ/cm²) and then annealed at 80°C for 1 hour to cure the adhesive.

### [Comparative Example 3-1]

Except that charge-transporting varnish A2 was used instead of charge-transporting varnish A1, the same procedure as in Example 3-1 was carried out to fabricate a hole-only device.

### [Example 3-2]

Except that charge-transporting varnish B1 was used instead of charge-transporting varnish A1, the same procedure as in Example 3-1 was carried out to fabricate a hole-only device.

### [Comparative Example 3-2]

Except that charge-transporting varnish B2 was used instead of charge-transporting varnish A1, the same procedure as in Example 3-1 was carried out to fabricate a hole-only device.

### [Example 3-3]

Except that charge-transporting varnish C1 was used instead of charge-transporting varnish A1, the same procedure as in Example 3-1 was carried out to fabricate a hole-only device.

### [Comparative Example 3-3]

Except that charge-transporting varnish C2 was used instead of charge-transporting varnish A1, the same procedure as in Example 3-1 was carried out to fabricate a hole-only device.

The current densities at a driving voltage of 5 V were measured for the hole-only devices fabricated in Examples 3-1 to 3-3 and Comparative Examples 3-1 to 3-3. The results are shown in Table 2.

**[Table 2]**

| | Charge-transporting varnish | Current density (mA/cm²) |
|---|---|---|
| Example 3-1 | A1 | 459 |
| Comparative Example 3-1 | A2 | 9 |
| Example 3-2 | B1 | 4 |
| Comparative Example 3-2 | B2 | 0.06 |
| Example 3-3 | C1 | 39 |
| Comparative Example 3-3 | C2 | 2 |

As shown in Tables 1 and 2, the charge-transporting varnish of the invention which contains an aryl sulfonate ester compound had higher electrical conductivity and higher hole-transportability as compared to a charge-transporting varnish which does not contain an aryl sulfonate ester compound.

### [5] Fabrication of HOD and Evaluation of Device Characteristics-2

The ITO substrate used in the following Examples and Comparative Examples was the same as that described above.

### [Example 4-1]

Charge-transporting varnish D was applied onto the ITO substrate using a spin coater and was subsequently pre-fired at 120°C for 1 minute in open air and then subjected to main firing at 230°C for 15 minutes, thereby forming a 30-nm hole-injecting layer thin film on the ITO substrate.

Next, in a glove box under a nitrogen atmosphere, a 0.6 wt% xylene solution of TFB polymer (LT-N148, from Luminescence Technology) was spin-coated onto the hole-injecting layer to form a film, and heating and baking at 130°C was carried out for 10 minutes, thereby forming a 40-nm hole-transporting layer thin-film.

Using a vapor deposition system (degree of vacuum, 2.0×10⁻⁵ Pa), thin films of aluminum were deposited thereon, giving a hole-only device. Vapor deposition was carried out at a deposition rate of 0.2 nm/s. The thicknesses of the aluminum thin film was set to 80 nm.

To prevent the device characteristics from deteriorating due to the influence of oxygen, moisture and the like in air, the hole-only device was sealed with sealing substrates, following which the characteristics were evaluated. Sealing was carried out by the following procedure.

The hole-only device was placed between sealing substrates in a nitrogen atmosphere having an oxygen concentration of 2 ppm or less and a dew point of not more than -85°C, and the sealing substrates were laminated together using an adhesive (MORESCO Moisture Cut WB90US(P), from Moresco Corporation). At this time, a desiccant (HD-071010W-40, from Dynic Corporation) was placed, together with the hole-only device, within the sealing substrates. The laminated sealing substrates were irradiated with an ultraviolet ray (wavelength, 365 nm; dosage, 6,000 mJ/cm²) and then annealed at 80°C for 1 hour to cure the adhesive.

### [Example 4-2]

Except that charge-transporting varnish E was used instead of charge-transporting varnish D, the same procedure as in Example 4-1 was carried out to fabricate a hole-only device.

### [Example 4-3]

Except that charge-transporting varnish F was used instead of charge-transporting varnish D, the same procedure as in Example 4-1 was carried out to fabricate a hole-only device.

### [Example 4-4]

Except that charge-transporting varnish G was used instead of charge-transporting varnish D, the same procedure as in Example 4-1 was carried out to fabricate a hole-only device.

### [Example 4-5]

Except that charge-transporting varnish H was used instead of charge-transporting varnish D, the same procedure as in Example 4-1 was carried out to fabricate a hole-only device.

### [Example 4-6]

Except that charge-transporting varnish I was used instead of charge-transporting varnish D, the same procedure as in Example 4-1 was carried out to fabricate a hole-only device.

### [Example 4-7]

Except that charge-transporting varnish J was used instead of charge-transporting varnish D, the same procedure as in Example 4-1 was carried out to fabricate a hole-only device.

### [Example 4-8]

Except that charge-transporting varnish K was used instead of charge-transporting varnish D, the same procedure as in Example 4-1 was carried out to fabricate a hole-only device.

### [Comparative Example 4-1]

Except that a hole-injecting layer was not formed, the same procedure as in Example 4-1 was carried out to fabricate a hole-only device.

The current densities at a driving voltage of 5 V were measured for the hole-only devices fabricated in Examples 4-1 to 4-8 and Comparative Example 4-1. The results are shown in Table 3.

**[Table 3]**

| | Charge-transporting varnish | Current density (mA/cm²) |
|---|---|---|
| Example 4-1 | D | 1,840 |
| Example 4-2 | E | 2,025 |
| Example 4-3 | F | 2,188 |
| Example 4-4 | G | 1,725 |
| Example 4-5 | H | 2,167 |
| Example 4-6 | I | 2,115 |
| Example 4-7 | J | 2,240 |
| Example 4-8 | K | 1,628 |
| Comparative Example 4-1 | - | 49 |

As shown in Table 3, a charge-transporting varnish containing the sulfonic acid ester compound of the invention had high hole-transportability.

### [6] Fabrication of HOD and Evaluation of Device Characteristics-3

The ITO substrate used in the following Examples and Comparative Examples was the same as that described above.

### [Example 5-1]

Charge-transporting varnish L was applied onto the ITO substrate using a spin coater and was subsequently pre-fired at 120°C for 1 minute in open air and then subjected to main firing at 230°C for 15 minutes, thereby forming a 40-nm thin film on the ITO substrate.

Using a vapor deposition system (degree of vacuum, 2.0×10⁻⁵ Pa), thin films of α-NPD and aluminum were successively deposited thereon, giving a hole-only device. Vapor deposition was carried out at a deposition rate of 0.2 nm/s. The thicknesses of the α-NPD thin film and the aluminum thin film were set to respectively 30 nm and 80 nm.

To prevent the device characteristics from deteriorating due to the influence of oxygen, moisture and the like in air, the hole-only device was sealed with sealing substrates, following which the characteristics were evaluated. Sealing was carried out by the following procedure.

The hole-only device was placed between sealing substrates in a nitrogen atmosphere having an oxygen concentration of 2 ppm or less and a dew point of not more than -85°C, and the sealing substrates were laminated together using an adhesive (MORESCO Moisture Cut WB90US(P), from Moresco Corporation). At this time, a desiccant (HD-071010W-40, from Dynic Corporation) was placed, together with the hole-only device, within the sealing substrates. The laminated sealing substrates were irradiated with an ultraviolet ray (wavelength, 365 nm; dosage, 6,000 mJ/cm²) and then annealed at 80°C for 1 hour to cure the adhesive.

### [Example 5-2]

Except that charge-transporting varnish M was used instead of charge-transporting varnish L, the same procedure as in Example 5-1 was carried out to fabricate a hole-only device.

### [Comparative Example 5-1]

Except that charge-transporting varnish N was used instead of charge-transporting varnish L, the same procedure as in Example 5-1 was carried out to fabricate a hole-only device.

The current densities at a driving voltage of 4 V were measured for the hole-only devices fabricated in Examples 5-1 to 5-2 and Comparative Example 5-1. The results are shown in Table 4.

**[Table 4]**

| | Charge-transporting varnish | Current density (mA/cm²) |
|---|---|---|
| Example 5-1 | L | 2,370 |
| Example 5-2 | M | 2,086 |
| Comparative Example 5-1 | N | 97 |

As shown in Table 4, a charge-transporting varnish containing the sulfonic acid ester compound of the invention had higher hole-transportability as compared to a charge-transporting varnish containing a conventional sulfonic acid ester compound.

### [7] Fabrication of Organic EL Devices and Evaluation of Device Characteristics

### [Example 6-1]

Charge-transporting varnish A1 was applied onto the ITO substrate using a spin coater, and then subjected to firing at 230°C for 15 minutes, thereby forming a 60-nm thin film on the ITO substrate.

Using a vapor deposition system (degree of vacuum: 2.0×10⁻⁵ Pa), α-NPD of 30 nm and Alq₃ of 40 nm were successively deposited thereon. At this time, the vapor deposition was carried out at a deposition rate of 0.2 nm/sec. Subsequently, thin films of lithium fluoride and aluminum were successively deposited, giving an organic EL device. At this time, the vapor deposition was carried out at a rate of 0.02 nm/sec for lithium fluoride and, at a rate of 0.2 nm/sec for aluminum. The thicknesses of the lithium fluoride thin film and the aluminum thin film were set to respectively 0.5 nm and 80 nm.

To prevent the device characteristics from deteriorating due to the influence of oxygen, moisture and the like in air, the organic EL device was sealed with sealing substrates, following which the characteristics were evaluated. Sealing was carried out by the following procedure.

The organic EL device was placed between sealing substrates in a nitrogen atmosphere having an oxygen concentration of 2 ppm or less and a dew point of not more than -85°C, and the sealing substrates were laminated together using an adhesive (MORESCO Moisture Cut WB90US(P), from Moresco Corporation). At this time, a desiccant (HD-071010W-40, from Dynic Corporation) was placed, together with the organic EL device, within the sealing substrates. The laminated sealing substrates were irradiated with an ultraviolet ray (wavelength, 365 nm; dosage, 6,000 mJ/cm²) and then annealed at 80°C for 1 hour to cure the adhesive.

### [Comparative Example 6-1]

Except that charge-transporting varnish A2 was used instead of charge-transporting varnish A1, the same procedure as in Example 6-1 was carried out to fabricate an organic EL device.

### [Example 6-2]

Except that charge-transporting varnish B1 was used instead of charge-transporting varnish A1, the same procedure as in Example 6-1 was carried out to fabricate an organic EL device.

### [Comparative Example 6-2]

Except that charge-transporting varnish B2 was used instead of charge-transporting varnish A1, the same procedure as in Example 6-1 was carried out to fabricate an organic EL device.

### [Example 6-3]

Except that charge-transporting varnish C1 was used instead of charge-transporting varnish A1, the same procedure as in Example 6-1 was carried out to fabricate an organic EL device.

### [Example 6-4]

Except that charge-transporting varnish O was used instead of charge-transporting varnish A1, the same procedure as in Example 6-1 was carried out to fabricate an organic EL device.

### [Example 6-5]

Except that charge-transporting varnish P was used instead of charge-transporting varnish A1, the same procedure as in Example 6-1 was carried out to fabricate an organic EL device.

### [Comparative Example 6-3]

Except that charge-transporting varnish C2 was used instead of charge-transporting varnish A1, the same procedure as in Example 6-1 was carried out to fabricate an organic EL device.

The current densities and the luminances at a predetermined driving voltage were measured for the organic EL devices fabricated in Examples 6-1 to 6-5 and Comparative Examples 6-1 to 6-3. The results are shown in Table 5.

**[Table 5]**

| | Charge-transporting varnish | Driving voltage (V) | Current density (mA/cm²) | Luminance (cd/m²) |
|---|---|---|---|---|
| Example 6-1 | A1 | 6 | 18.1 | 794 |
| Comparative Example 6-1 | A2 | 6 | 1.1 | 20 |
| Example 6-2 | B1 | 7 | 153 | 346 |
| Comparative Example 6-2 | B2 | 7 | 1.1×10⁻² | 0.28 |
| Example 6-3 | C1 | 10 | 31.3 | 148 |
| Example 6-4 | O | 10 | 3.3 | 85 |
| Example 6-5 | P | 10 | 1.0 | 19 |
| Comparative Example 6-3 | C2 | 10 | 4.7×10⁻² | 6.8×10⁻² |

As shown in Table 5, the charge-transporting varnish of the invention which contains an aryl sulfonate ester compound had higher organic EL characteristics as compared to a charge-transporting varnish which does not contain an aryl sulfonate ester compound.

## Claims

1. A charge-transporting varnish comprising: (A) an aryl sulfonate ester compound; (B) a tertiary aryl amine compound having at least one nitrogen atom with all the nitrogen atoms forming a tertiary aryl amine structure; and (C) an organic solvent.

2. The charge-transporting varnish according to claim 1, wherein the aryl sulfonate ester compound is a fluorine atom-containing aryl sulfonate ester compound.

3. The charge-transporting varnish according to claim 1, wherein the aryl sulfonate ester compound is a compound of the following formula (1) or (1'): 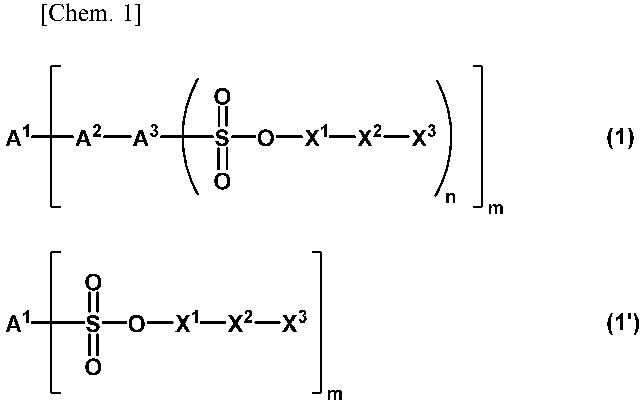 wherein A¹ is an m-valent hydrocarbon group of 6 to 20 carbon atoms which optionally has a substituent and which contains one or more aromatic rings, or an m-valent group derived from the following formula (2) or (3): 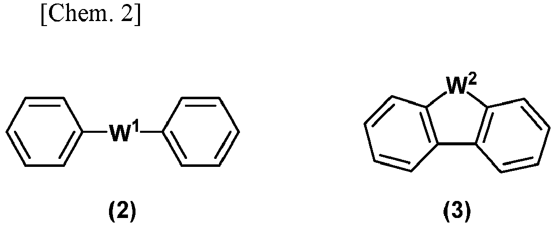
wherein W¹ and W² are each independently -O-, -S-, -S (O)- or -S(O₂)-, or -N-, Si-, -P- or -P(O)- which optionally has a substituent;
A² is -O-, -S- or -NH-;
A³ is an (n+1)-valent aromatic group of 6 to 20 carbon atoms;
X¹ is an alkylene group of 2 to 5 carbon atoms, the alkylene group optionally having -O-, -S- or a carbonyl group interposed between carbon atoms, the alkylene group being optionally substituted with alkyl groups of 1 to 20 carbon atoms at some or all of hydrogen atoms;
X² is a single bond, -O-, -S- or -NR-, where R is a hydrogen atom or a monovalent hydrocarbon group of 1 to 10 carbon atoms;
X³ is a monovalent hydrocarbon group of 1 to 20 carbon atoms which optionally has a substituent;
m is an integer that satisfies the condition 1 ≤ m ≤ 4; and
n is an integer that satisfies the condition 1 ≤ n ≤ 4.

4. The charge-transporting varnish according to claim 3, wherein A¹ is an m-valent hydrocarbon group of 6 to 20 carbon atoms which is substituted with a fluorine atom, and contains one or more aromatic rings, or an m-valent group derived from a compound of formula (2) or (3).

5. The charge-transporting varnish according to any one of claims 1 to 4, wherein the aryl sulfonate ester compound is a compound of any one of the following formulas (1-1) to (1-3): 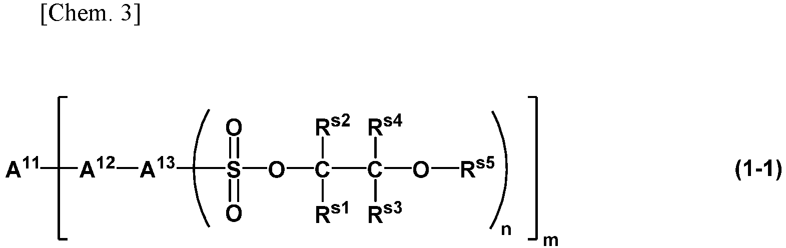
wherein R^{s1} to R^{s4} are each independently a hydrogen atom or a linear or branched alkyl group of 1 to 6 carbon atoms, and R^{s5} is a monovalent hydrocarbon group of 2 to 20 carbon atoms which optionally has a substituent;
A¹¹ is an m-valent group derived from perfluorobiphenyl, A¹² is -O- or -S-, and A¹³ is an (n+1)-valent group derived from naphthalene or anthracene; and
m and n are the same as described above; 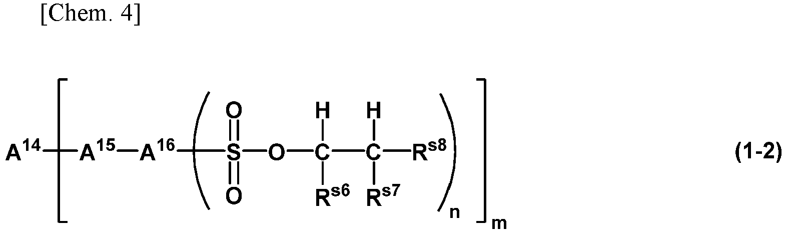
wherein R^{s6} and R^{s7} are each independently a hydrogen atom, or a linear or branched monovalent aliphatic hydrocarbon group, and R^{s8} is a linear or branched monovalent aliphatic hydrocarbon group, provided that the total number of carbon atoms of R^{s6}, R^{s7} and R^{s8} is 6 or more;
A¹⁴ is an m-valent hydrocarbon group which optionally has a substituent and which contains one or more aromatic rings, A¹⁵ is -O- or -S-, and A¹⁶ is an (n+1)-valent aromatic group; and
m and n are the same as described above; and 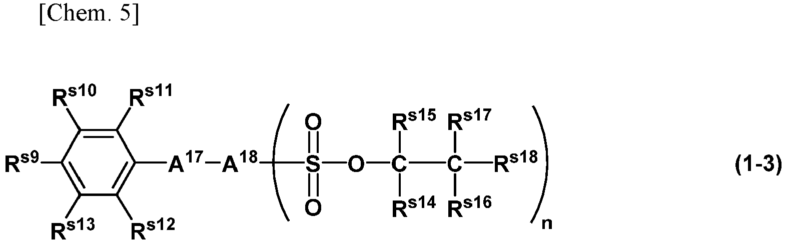
wherein R^{s9} to R^{s13} are each independently a hydrogen atom, a nitro group, a cyano group, a halogen atom, an alkyl group of 1 to 10 carbon atoms, a halogenated alkyl group of 1 to 10 carbon atoms, or a halogenated alkenyl group of 2 to 10 carbon atoms;
R^{s14} to R^{s17} are each independently a hydrogen atom, or a linear or branched monovalent aliphatic hydrocarbon group of 1 to 20 carbon atoms;
R^{s18} is a linear or branched monovalent aliphatic hydrocarbon group of 1 to 20 carbon atoms, or -OR^{s19}, where R^{s19} is a monovalent hydrocarbon group of 2 to 20 carbon atoms which optionally has a substituent;
A¹⁷ is -O-, -S- or -NH-;
A¹⁸ is an (n+1)-valent aromatic group; and
n is the same as described above.

6. The charge-transporting varnish according to any one of claims 1 to 5, wherein the tertiary aryl amine compound has at least two nitrogen atoms, with all the nitrogen atoms forming a tertiary aryl amine structure.

7. The charge-transporting varnish according to any one of claims 1 to 6, wherein the organic solvent is a low-polarity organic solvent.

8. A charge-transporting thin film obtained using the charge-transporting varnish according to any one of claims 1 to 7.

9. An organic electroluminescence device comprising the charge-transporting thin film according to claim 8.
